# EUROPEAN PATENT SPECIFICATION

(11) **EP 1 763 511 B1**
(45) Date of publication and mention of the grant of the patent: **28.07.2010**
(21) Application number: 05742988.8
(22) Date of filing: 03.05.2005
(51) Int. Cl.: C07C 323/20, C07C 323/29, C07D 207/333, C07D 213/32, C07D 295/096, C07D 307/38, C07D 333/18, C07D 413/06, A61K 31/192, A61K 31/341, A61K 31/381, A61K 31/40, A61K 31/4402, A61K 31/535, A61P 3/10

(54) **PHENOXYACETIC ACID DERIVATIVES AS PPAR AGONISTS**
PHENOXYESSIGSÄUREDERIVATE ALS PPAR-AGONISTEN
DERIVES D'ACIDE PHENOXYACETIQUE EN TANT QU'AGONISTES DE PPAR

(30) Priority: 05.05.2004 DK 200400717
(43) Date of publication of application: 21.03.2007
(73) Proprietor: High Point Pharmaceuticals, LLC, High Point, NC 27265 (US)
(72) Inventor: POLIVKA, Zdenék, 150 00 Praha 5 (CZ); SINDELAR, Karel, 140 00 Praha 4 (CZ); SAUERBERG, Per, DK-3520 Farum (DK); PETTERSSON, Ingrid, DK-1864 Frederiksberg (DK)
(74) Representative: Russell, Lindsey
(86) International application number: PCT/EP2005/052012
(87) International publication number: WO 2005/105735

(56) References cited:
- WO-A-2004/022533

## Description

### FIELD OF THE INVENTION

The present invention relates to novel compounds, to the use of these compounds as pharmaceutical compositions, to pharmaceutical compositions comprising the compounds and to a method of treatment employing these compounds and compositions. More specifically, the compounds of the invention can be utilised in the treatment and/or prevention of conditions mediated by the Peroxisome Proliferator-Activated Receptors (PPAR), in particular the PPARδ subtype.

### BACKGROUND OF THE INVENTION

Coronary artery disease (CAD) is the major cause of death in Type 2 diabetic and metabolic syndrome patients (i.e. patients that fall within the 'deadly quartet' category of impaired glucose tolerance, insulin resistance, hypertriglyceridaemia and/or obesity).

The hypolipidaemic fibrates and antidiabetic thiazolidinediones separately display moderately effective triglyceride-lowering activities although they are neither potent nor efficacious enough to be a single therapy of choice for the dyslipidaemia often observed in Type 2 diabetic or metabolic syndrome patients. The thiazolidinediones also potently lower circulating glucose levels of Type 2 diabetic animal models and humans. Studies on the molecular actions of these compounds indicate that thiazolidinediones and fibrates exert their action by activating distinct transcription factors of the peroxisome proliferator activated receptor (PPAR) family, resulting in increased and decreased expression of specific enzymes and apolipoproteins respectively, both key-players in regulation of plasma triglyceride content. Fibrates, on the one hand, are PPARα activators, acting primarily in the liver. Thiazolidinediones, on the other hand, are high affinity ligands for PPARγ acting primarily on adipose tissue.

Adipose tissue plays a central role in lipid homeostasis and the maintenance of energy balance in vertebrates. Adipocytes store energy in the form of triglycerides during periods of nutritional affluence and release it in the form of free fatty acids at times of nutritional deprivation. The development of white adipose tissue is the result of a continuous differentiation process throughout life. Much evidence points to the central role of PPARγ activation in initiating and regulating this cell differentiation. Several highly specialised proteins are induced during adipocyte differentiation, most of them being involved in lipid storage and metabolism. The exact link from activation of PPARγ to changes in glucose metabolism, most notably a decrease in insulin resistance in muscle, has not yet been clarified. A possible link is via free fatty acids such that activation of PPARγ induces Lipoprotein Lipase (LPL), Fatty Acid Transport Protein (FATP) and Acyl-CoA Synthetase (ACS) in adipose tissue but not in muscle tissue. This, in turn, reduces the concentration of free fatty acids in plasma dramatically, and due to substrate competition at the cellular level, skeletal muscle and other tissues with high metabolic rates eventually switch from fatty acid oxidation to glucose oxidation with decreased insulin resistance as a consequence.

PPARα is involved in stimulating β-oxidation of fatty acids. In rodents, a PPARα-mediated change in the expression of genes involved in fatty acid metabolism lies at the basis of the phenomenon of peroxisome proliferation, a pleiotropic cellular response, mainly limited to liver and kidney and which can lead to hepatocarcinogenesis in rodents. The phenomenon of peroxisome proliferation is not seen in man. In addition to its role in peroxisome proliferation in rodents, PPARα is also involved in the control of HDL cholesterol levels in rodents and humans. This effect is, at least partially, based on a PPARα-mediated transcriptional regulation of the major HDL apolipoproteins, apo A-I and apo A-II. The hypotriglyceridemic action of fibrates and fatty acids also involves PPARα and can be summarised as follows: (I) an increased lipolysis and clearance of remnant particles, due to changes in lipoprotein lipase and apo C-III levels, (II) a stimulation of cellular fatty acid uptake and their subsequent conversion to acyl-CoA derivatives by the induction of fatty acid binding protein and acyl-CoA synthase, (III) an induction of fatty acid β-oxidation pathways, (IV) a reduction in fatty acid and triglyceride synthesis, and finally (V) a decrease in VLDL production. Hence, both enhanced catabolism of triglyceride-rich particles as well as reduced secretion of VLDL particles constitutes mechanisms that contribute to the hypolipidemic effect of fibrates.

PPARδ activation was initially reported not to be involved in modulation of glucose or triglyceride levels. (Berger et al., j. Biol. Chem., 1999, Vol 274, pp. 6718-6725). Later it has been shown that PPARδ activation leads to increased levels of HDL cholesterol in db/db mice (Leibowitz et al. FEBS letters 2000, 473, 333-336). Further, a PPARδ agonist when dosed to insulin-resistant middle-aged obese rhesus monkeys caused a dramitic dose-dependent rise in serum HDL cholesterol while lowering the levels of small dense LDL, fasting triglycerides and fasting insulin (Oliver et al. PNAS 2001, 98, 5306-5311). The same paper also showed that PPARδ activation increased the reverse cholesterol transporter ATP-binding cassette A1 and induced apolipoprotein A1-specific cholesterol efflux. The involvement of PPARδ in fatty acid oxidation in muscles was further substantiated in PPARα knock-out mice. Muoio et al. (J. Biol. Chem. 2002, 277, 26089-26097) showed that the high levels of PPARδ in skeletal muscle can compensate for deficiency in PPARα.

Recently, two different transgenic mouse models over-expressing PPARδ in either adipose tissue (Cell 2003, 113, 159-170) or in muscle tissue (FASEB J. 2003, 17, 209-226) have both shown up-regulation of genes (LPL, FABP, FAT, CD36, CPT1 b, and ACS) and proteins (UCP-2) responsible for lipid uptake and metabolism and energy uncoupling. Both types of mice had reduced adipose tissue and were protected against high fat diet induced body weight gain. Further, pharmacological treatment of both high fat diet induced insulin resistant mice and diabetic ob/ob with the potent PPARδ agonist GW501516 showed lowering of plasma glucose and insulin and improved insulin sensitivity (PNAS 2003, 100, 15924-15929). In vivo increased oxygen consumption suggesting fuel-switch from glucose to FFA, as well as FFA oxidation in skeletal muscle was demonstrated both in vivo and in vitro. Supportive for the hypothesis of skeletal muscle being the major target organ were two publications on in vitro treatment of C2C12 muscle cells with GW501516 showing regulation of genes involved with TG hydrolysis and FFA oxidation (LPL↑, ACS4↑, CTP1↑), preferential lipid utilization (PDK4↑), energy expenditure (UCP1↑,-2↑, -3↑) and lipid efflux (ABCA1/G1↑) (BioChem. Biophys. Acta 2003, 1633, 43-50; Mol. Endocrin. 2003, 17, 2477-2493). Direct and an indirect mechanisms recently demonstrated prompted the authors to suggest that "PPARδ and its ligands may serve as therapeutic targets to attenuate inflammation and slow the progression of atherosclerosis" (Science 2003, 302, 453-457).

Taken together these observations suggest that PPARδ activation is useful in the treatment and prevention of cardiovascular diseases and conditions including atherosclerosis, hypertriglyceridemia, and mixed dyslipidaemia as well as type 2 diabetes.

A number of PPARδ compounds have been reported to be useful in the treatment of hyperglycemia, hyperlipidemia and hypercholesterolemia (WO 01/00603, WO 02/59098, WO 03/084916, WO 03/074050, WO 03/074051, WO 03/074052, WO 03/035603, WO 03/97607, WO 04/005253, WO 03/33493, WO 03/16291, WO 02/76957, 02/46154, WO 03/16265, WO 021100812, WO 02/98840, WO 02/80899, WO 02/79162, WO03/072100, WO 01/25181, WO 02/14291, WO 01/79197, WO 99/4815, WO 97/28149, WO 98/27974, WO 97/28115, WO 97/27857, WO 97/28137, WO 97/27847).

Glucose lowering as a single approach does not overcome the macrovascular complications associated with Type 2 diabetes and metabolic syndrome. Novel treatments of Type 2 diabetes and metabolic syndrome must therefore aim at lowering both the overt hypertriglyceridaemia associated with these syndromes as well as alleviation of hyperglycaemia.

This indicate that research for compounds displaying various degree of PPARα, PPARγ and PPARδ activation should lead to the discovery of efficacious triglyceride and/or cholesterol and/or glucose lowering drugs that have great potential in the treatment of diseases such as type 2 diabetes, dyslipidemia, syndrome X (including the metabolic syndrome, i.e. impaired glucose tolerance, insulin resistance, hypertrigyceridaemia and/or obesity), cardiovascular diseases (including atherosclerosis) and hypercholesteremia.

### DEFINITIONS

In the structural formulas given herein and throughout the present specification the following terms have the indicated meaning:

The term "C₁₋₆-alkyl" as used herein, alone or in combination, represent a linear or branched, saturated hydrocarbon chain having the indicated number of carbon atoms. Representative examples include, but are not limited to methyl, ethyl, n-propyl, isopropyl, butyl, isobutyl, *sec*-butyl, *tert*-butyl, pentyl, isopentyl, hexyl, isohexyl and the like.

The term "C₁₋₆-alkylcarbonyl as used herein, represents a "C₁₋₆-alkyl" group as defined above having the indicated number of carbon atoms linked through a carbonyl group. Representative examples include, but are not limited to, methylcarbonyl, ethylcarbonyl, n-propylcarbonyl, isopropylcarbonyl, butylcarbonyl, isobutylcarbonyl, *sec*-butylcarbonyl, *tert-*butylcarbonyl, n-pentylcarbonyl, isopentylcarbonyl, neopentylcarbonyl, *tert*-pentylcarbonyl, n-hexylcarbonyl, isohexylcarbonyl and the like.

The term "C₁₋₆-alkylsulfonyl" as used herein refers to a monovalent substituent comprising a "C₁₋₆-alkyl" group as defined above linked through a sulfonyl group. Representative examples include, but are not limited to, methylsulfonyl, ethylsulfonyl, n-propylsulfonyl, isopropylsulfonyl, n-butylsulfonyl, isobutylsulfonyl, *sec*-butylsulfonyl, *tert*-butylsulfonyl, n-pentylsulfonyl, isopentylsulfonyl, neopentylsulfonyl, *tert*-pentylsulfonyl, n-hexylsulfonyl, isohexylsulfonyl and the like.

The term "C₁₋₆-alkylamido" as used herein, refers to an acyl group linked through an amino group; Representative examples include, but are not limited to acetylamino, propionylamino, butyrylamino, isobutyrylamino, pivaloylamino, valerylamino and the like.

The term "C₃₋₆-cycloalkyl" as used herein, alone or in combination, represent a saturated monocyclic hydrocarbon group having the indicated number of carbon atoms. Representative examples include, but are not limited to cyclopropyl, cyclobutyl, cyclopentyl, cyclohexyl and the like.

The term "C₂₋₆-alkenyl" as used herein, represent an olefinically unsaturated branched or straight hydrocarbon group having from 2 to the specified number of carbon atoms and at least one double bond. Representative examples include, but are not limited to, vinyl, 1-propenyl, 2-propenyl, allyl, iso-propenyl, 1,3-butadienyl, 1-butenyl, hexenyl, pentenyl and the like.

The term "C₂₋₆-alkynyl" as used herein, represent an unsaturated branched or straight hydrocarbon group having from 2 to the specified number of carbon atoms and at least one triple bond. Representative examples include, but are not limited to, 1-propynyl, 2-propynyl, 1-butynyl, 2-butynyl, 1-pentynyl, 2-pentynyl and the like.

The term "C₄₋₆-alkenynyl" as used herein, represent an unsaturated branched or straight hydrocarbon group having from 4 to the specified number of carbon atoms and both at least one double bond and at least one triple bond. Representative examples include, but are not limited to, 1-penten-4-ynyl, 3-penten-1-ynyl, 1,3-hexadiene-5-ynyl and the like.

The term "C₁₋₆-alkoxy" as used herein, alone or in combination, refers to a straight or branched configuration linked through an ether oxygen having its free valence bond from the ether oxygen. Examples of linear alkoxy groups are methoxy, ethoxy, propoxy, butoxy, pentoxy, hexoxy and the like. Examples of branched alkoxy are isopropoxy, sec-butoxy, tert-butoxy, isopentyloxy, isohexyloxy and the like.

The term "C₃₋₆-cycloalkoxy" as used herein, alone or in combination, represent a saturated monocyclic hydrocarbon group having the indicated number of carbon atoms linked through an ether oxygen having its free valence bond from the ether oxygen. Examples of cycloalkoxy groups are cyclopropyloxy, cyclobutyloxy, cyclopentyloxy, cyclohexyloxy and the like.

The term "C₁₋₆-alkylthio" as used herein, alone or in combination, refers to a straight or branched monovalent substituent comprising a "C₁₋₆-alkyl" group as defined above linked through a divalent sulfur atom having its free valence bond from the sulfur atom and having 1 to 6 carbon atoms. Representative examples include, but are not limited to, methylthio, ethylthio, propylthio, butylthio, pentylthio and the like.

The term "C₃₋₆-cycloalkylthio" as used herein, alone or in combination, represent a saturated monocyclic hydrocarbon group having the indicated number of carbon atoms linked through a divalent sulfur atom having its free valence bond from the sulfur atom. Examples of cycloalkoxy groups are cyclopropylthio, cyclobutylthio, cyclopentylthio, cyclohexylthio and the like.

The term "C₁₋₆-alkylamino" as used herein, alone or in combination, refers to a straight or branched monovalent substituent comprising a "C₁₋₆-alkyl" group as defined above linked through amino having a free valence bond from the nitrogen atom. Representative examples include, but are not limited to, methylamino, ethylamino, propylamino, butylamino, pentylamino and the like.

The term "C₁₋₆-alkylaminocarbonyl" as used herein refers to a monovalent substituent comprising a C₁₋₆-monoalkylamino group linked through a carbonyl group such as e.g. methylaminocarbonyl, ethylaminocarbonyl, n-propylaminocarbonyl, isopropylaminocarbonyl, n-butylaminocarbonyl, sec-butylaminocarbonyl, isobutylaminocarbonyl, tert-butylaminocarbonyl, n-pentylaminocarbonyl, 2-methylbutylaminocarbonyl, 3-methylbutylaminocarbonyl, n-hexylaminocarbonyl, 4-methylpentylaminocarbonyl, neopentylaminocarbonyl, n-hexylaminocarbonyl and 2-2-dimethylpropylaminocarbonyl and the like.

The term "C₃₋₆-cycloalkylamino" as used herein, alone or in combination, represent a saturated monocyclic hydrocarbon group having the indicated number of carbon atoms linked through amino having a free valence bond from the nitrogen atom. Representative examples include, but are not limited to, cyclopropylamino, cyclobutylamino, cyclopentylamino, cyclohexylamino and the like.

The term "C₁₋₆-alkoxyC₁₋₆alkyl" as used herein, alone or in combination, refers to a "C₁₋₆-alkyl" group as defined above whereto is attached a "C₁₋₆-alkoxy" group as defined above. Representative examples include, but are not limited to, methoxymethyl, ethoxymethyl, methoxyethyl, ethoxyethyl and the like.

The term "aryl" as used herein refers to an aromatic monocyclic or an aromatic fused bi- or tricyclic hydrocarbon group. Representative examples include, but are not limited to, phenyl, naphthyl, anthracenyl, phenanthrenyl, azulenyl, fluorenyl, indenyl, pentalenyl and the like.

The term "arylene" as used herein refers to divalent aromatic monocyclic or a divalent aromatic fused bi- or tricyclic hydrocarbon group. Representative examples include, but are not limited to, phenylene, naphthylene and the like.

The term "arylcarbonyl" as used herein represents an "aryl" group as defined above linked through a carbonyl group. Representative examples include, but are not limited to, phenylcarbonyl, naphthylcarbonyl, anthracenylcarbonyl, phenanthrenylcarbonyl, azulenylcarbonyl and the like

The term "arylsulfonyl" as used herein refers to an "aryl" group as defined above linked through a sulfonyl group. Representative examples include, but are not limited to, phenylsulfonyl, naphthylsulfonyl, anthracenylsulfonyl, phenanthrenylsulfonyl, azulenylsulfonyl, and the like.

The term "arylamido" as used herein refers to an arylcarbonyl group linked through an amino group. Representative examples include, but are not limited to phenylcarbonylamino, naphthylcarbonylamino, anthracenylcarbonylamino, phenanthrenylcarbonylamino, azulenylcarbonylamino and the like.

The term "halogen" means fluorine, chlorine, bromine or iodine.

The term "perhalomethyl" means trifluoromethyl, trichloromethyl, tribromomethyl or triiodomethyl.

The term "perhalomethoxy" means trifluoromethoxy, trichloromethoxy, tribromomethoxy or triiodomethoxy.

The term "C₁₋₆-dialkylamino" as used herein refers to an amino group wherein the two hydrogen atoms independently are substituted with a straight or branched, saturated hydrocarbon chain having the indicated number of carbon atoms. Representative examples include, but are not limited to, dimethylamino, N-ethyl-N-methylamino, diethylamino, dipropylamino, N-(n-butyl)-N-methylamino, di(n-pentyl)amino and the like.

The term "acyl" as used herein refers to a monovalent substituent comprising a "C₁₋₆-alkyl" group as defined above linked through a carbonyl group. Representative examples include, but are not limited to, acetyl, propionyl, butyryl, isobutyryl, pivaloyl, valeryl and the like.

The term "heteroaryl" as used herein, alone or in combination, refers to a monovalent substituent comprising a 5-7 membered monocyclic aromatic system or a 8-10 membered bicyclic aromatic system containing one or more heteroatoms selected from nitrogen, oxygen and sulfur, e.g. furyl, thienyl, pyrrolyl, imidazolyl, pyrazolyl, triazolyl, pyridyl, pyrazinyl, pyrimidinyl, pyridazinyl, isothiazolyl, isoxazolyl, oxazolyl, oxadiazolyl, thiadiazolyl, quinolyl, isoquinolyl, quinazolinyl, quinoxalinnyl, isoindolyl, indolyl, benzimidazolyl, benzoxazolyl, benzothiazolyl, benzofuranyl, tetrazolyl, carbazolyl, benzothienyl, pteridinyl and purinyl and the like.

The term "heteroarylene" as used herein, alone or in combination, refers to divalent 5-7 membered monocyclic aromatic system or a 8-10 membered bicyclic aromatic system containing one or more heteroatoms selected from nitrogen, oxygen and sulfur, e.g. furylene, thienylene, pyrrolylene, imidazolylene, pyrazolylene, triazolylene, pyridylene, pyrazinylene, pyrimidinylene, pyridazinylene, isothiazolylene, isoxazolylene, oxazolylene, oxadiazolylene, thiadiazolylene, quinolylene, isoquinolylene, quinazolinylene, quinoxalinnylene, indolylene, benzimidazolylene, benzofuranylene, benzothienylene, pteridinylene and purinylene and the like.

The term "heteroaryloxy" as used herein, alone or in combination, refers to a heteroaryl as defined herein linked to an oxygen atom having its free valence bond from the oxygen atom e.g. pyrrolyloxy, imidazolyloxy, pyrazolyloxy, triazolyloxy, pyrazinyloxy, pyrimidinyloxy, pyridazinyloxy, isothiazolyloxy, isoxazolyloxy, oxazolyloxy, oxadiazolyloxy, thiadiazolyloxy, quinolinyloxy, isoquinolinyloxy, quinazolinyloxy, quinoxalinyloxy, indoltloxy, benzimidazolyloxy, benzofuranyloxy, pteridinyloxy and purinyloxy and the like.

The term "aralkyl" as used herein refers to a straight or branched saturated carbon chain containing from 1 to 6 carbons substituted with an aromatic carbohydride. Representative examples include, but are not limited to, benzyl, phenethyl, 3-phenylpropyl, 1-naphthylmethyl, 2-(1-naphthyl)ethyl and the like.

The term "aryloxy" as used herein refers to phenoxy, 1-naphthyloxy, 2-naphthyloxy and the like.

The term "aralkoxy" as used herein refers to a C₁₋₆-alkoxy group substituted with an aromatic carbohydride, such as benzyloxy, phenethoxy, 3-phenylpropoxy, 1-naphthylmethoxy, 2-(1-naphtyl)ethoxy and the like.

The term "heteroaralkyl" as used herein refers to a straight or branched saturated carbon chain containing from 1 to 6 carbons substituted with a heteroaryl group; such as (2-furyl)methyl, (3-furyl)methyl, (2-thienyl)methyl, (3-thienyl)methyl, (2-pyridyl)methyl, 1-methyl-1-(2-pyrimidyl)ethyl and the like.

The term "heteroaralkoxy" as used herein refers to a heteroarylalkyl as defined herein linked to an oxygen atom having its free valence bond from the oxygen atom. Representative examples include, but are not limited to, (2-furyl)methyl, (3-furyl)methyl, (2-thienyl)methyl, (3-thienyl)methyl, (2-pyridyl)methyl, 1-methyl-1-(2-pyrimidyl)ethyl linked to oxygen, and the like.

The term "arylthio" as used herein, alone or in combination, refers to an aryl group linked through a divalent sulfur atom having its free valence bond from the sulfur atom, the aryl group optionally being mono- or polysubstituted with C₁₋₆-alkyl, halogen, hydroxy or C₁₋₆-alkoxy. Representative examples include, but are not limited to, phenylthio, (4-methylphenyl)-thio, (2-chlorophenyl)thio and the like.

The term "heterocyclyl" as used herein represents a saturated 3 to 12 membered monocyclic ring containing one or more heteroatoms selected from nitrogen, oxygen, sulfur, S(=O) and S(=O)₂. Representative examples are aziridinyl, azetidinyl, oxetanyl, pyrrolidinyl, imidazolidinyl, oxazolidinyl, thiazolidinyl, isothiazolidinyl, piperidinyl, homopiperidinyl, piperazinyl, morpholinyl, thiomorpholinyl, 1-oxo-thiomorpholinyl, 1,1 -dioxo-thiomorpholinyl, tetrahydrofuranyl, tetrahydrothienyl, tetrahydro-1,1-dioxothienyl, tetrahydropyranyl, tetrahydrothiopyranyl, 1,4-dioxanyl, 1,3-dioxanyl, and the like. Heterocyclyl is also intended to represent a saturated bicyclic ring containing one or more heteroatoms selected from nitrogen, oxygen, sulfur, S(=O) and S(=O)₂. Representative examples are octahydroindolyl, decahydroquinoxalinyl, and the like. Heterocyclyl is also intended to represent a saturated heterocyclic ring containing one or more heteroatoms selected from nitrogen, oxygen, sulfur, S(=O) and S(=O)₂ and having one or two bridges. Representative examples are 3-azabicydo[3.2.2]nonyl, 2-azabicyclo[2.2.1]heptyl, 3-azabicyclo[3.1.0]hexyl, 2,5-diazabicyclo[2.2.1]heptyl, atropinyl, tropinyl, quinuclidinyl, 1,4-diazabicyclo[2.2.2]octanyl, and the like. Heterocyclyl is also intended to represent a saturated heterocyclic ring containing one or more heteroatoms selected from nitrogen, oxygen, sulfur, S(=O) and S(=O)₂ and containing one or more spiro atoms. Representative examples are 1,4-dioxaspiro[4.5]decanyl, 8-azaspiro[4.5]decanyl, 2,8-diazaspiro[4.5]decanyl, and the like.

The term "five to eight member ring" as used herein refers to a saturated or unsaturated, substituted or unsubstituted hydrocarbon chain or hydrocarbon-heteroatom chain having from 3 to 6 atoms wherein the carbon atom in Ar, to which they are attached, and the adjacent carbon atom form a five to eight member ring.

Certain of the above defined terms may occur more than once in the structural formulae, and upon such occurrence each term shall be defined independently of the other.

The term "optionally substituted" as used herein means that the groups in question are either unsubstituted or substituted with one or more of the substituents specified. When the groups in question are substituted with more than one substituent the substituents may be the same or different.

The term "treatment" is defined as the management and care of a patient for the purpose of combating or alleviating the disease, condition or disorder, and the term includes the administration of the active compound to prevent the onset of the symptoms or complications, or alleviating the symptoms or complications, or eliminating the disease, condition, or disorder.

The term "pharmaceutically acceptable" is defined as being suitable for administration to humans without adverse events.

### DESCRIPTION OF THE INVENTION

The present invention relates to compounds of the general formula (I): 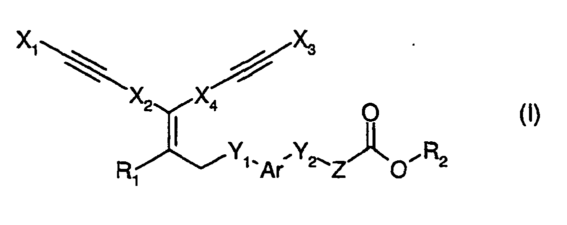 wherein X₁ is aryl or heteroaryl each of which is optionally substituted with one or more substituents selected from
- halogen, hydroxy, cyano, amino or carboxy; or
- C₁₋₆-alkyl, C₃₋₆-cycloalkyl, C₂₋₆-alkenyl, C₂₋₆-alkynyl, aryl, aralkyl, heteroaryl, heteroaralkyl, C₁₋₆-alkoxy, C₃₋₆-cycloalkoxy, aryloxy, aralkoxy, heteroaralkoxy, C₁₋₆-alkylthio, arylthio, C₃₋₆-cycloalkylthio, C₁₋₆-alkylcarbonyl, arylcarbonyl, C₁₋₆-alkylsulfonyl, arylsulfonyl, C₁₋₆-alkylamido, arylamido, C₁₋₆-alkylaminocarbonyl, C₁₋₆-alkylamino, C₁₋₆-dialkylamino or C₃₋₆-cycloalkylamino each of which is optionally substituted with one or more halogens; or
X₁ is C₁₋₆-alkyl, C₃₋₆-cycloalkyl, C₂₋₆-alkenyl, carbamoyl or C₃₋₆-cycloalkyl-C₁₋₆-alkyl each of which is optionally substituted with one or more substituents selected from
- halogen, hydroxy, cyano, amino or carboxy; or
- C₁₋₆-alkyl, C₃₋₆-cycloalkyl, C₂₋₆-alkenyl, C₂₋₆-alkynyl, C₃₋₆-cycloalkyl-C₁₋₆-alkyl, aryl, aralkyl, heteroaryl, heteroaralkyl, heterocyclyl, C₁₋₆-alkoxy, C₃₋₆-cycloalkoxy, C₃₋₆-cycloalkyl-C₁₋₆-alkoxy, aryloxy, heteroaryloxy, aralkoxy, heteroaralkoxy, C₁₋₆-alkylthio, C₃₋₆-cycloalkylthio, C₃₋₆-cycloalkyl-C₁₋₆-alkylthio,arylthio, heteroarylthio, aryl-C₁₋₆-alkylthio, heteroaryl-C₁₋₆-alkylthio, C₁₋₆-alkylcarbonyl, C₃₋₆-cycloalkylcarbonyl, C₃₋₆-cycloalkyl- C₁₋₆-alkyl-carbonyl, arylcarbonyl, heteroarylcarbonyl, C₁₋₆-alkylsulfonyl, C₃₋₆-cycloalkylsulfonyl, C₃₋₆-cycloalkyl-C₁₋₆-alkylsulfonyl, arylsulfonyl, heteroarylsulfonyl, C₁₋₆-alkylsulfamoyl, di-(C₁₋₆-alkyl)sulfamoyl, C₁₋₆-alkoxycarbonyl, C₃₋₆-cycloalkoxycarbonyl, C₃₋₆-cycloalkyl-C₁₋₆-alkoxycarbonyl, amino-C₁₋₆-alkyl, C₁₋₆-alkylamino-C₁₋₆-alkyl, di-(C₁₋₆-alkyl)amino-C₁₋₆-alkyl, C₁₋₆-alkylamido, C₃₋₆-cycloalkylamido, C₃₋₆-cycloalkyl-C₁₋₆-alkylamido, arylamido, C₁₋₆-alkylaminocarbonyl, C₃₋₆-cycloalkylaminocarbonyl, C₃₋₆-cycloalkyl-C₁₋₆-alkylaminocarbonyl, di-(C₁₋₆-alkyl)aminocarbonyl, di-(C₃₋₆-cycloalkyl-C₁₋₆-alkyl)aminocarbonyl, C₁₋₆-alkylamino , C₃₋₆-cycloalkylamino, C₃₋₆-cycloalkyl-C₁₋₆-alkylamino, di-(C₁₋₆-alkyl)amino, di-(C₃₋₆-cycloalkyl)amino or di-(C₃₋₆-cycloalkyl-C₁₋₆-alkyl)amino each of which is optionally substituted with one or more of halogen, cyano, hydroxy, acetyl or oxo
X₂ is arylene or heteroarylene each of which is optionally substituted with one or more substituents selected from
- halogen, hydroxy, cyano, amino or carboxy; or
- C₁₋₆-alkyl, C₃₋₆-cycloalkyl, C₂₋₆-alkenyl, C₂₋₆-alkynyl, C₁₋₆-alkoxy, C₃₋₆-cycloalkoxy, C₁₋₆-alkylthio, C₃₋₆-cycloalkylthio, C₁₋₆-alkylamino, C₁₋₆-dialkylamino or C₃₋₆-cycloalkylamino each of which is optionally substituted with one or more halogens; and
X₃ is aryl or heteroaryl each of which is optionally substituted with one or more substituents selected from
- halogen, hydroxy, cyano, amino or carboxy; or
- C₁₋₆-alkyl, C₃₋₆-cycloalkyl, C₂₋₆-alkenyl, C₂₋₆-alkynyl, aryl, aralkyl, heteroaryl, heteroaralkyl, C₁₋₆-alkoxy, C₃₋₆-cycloalkoxy, aryloxy, aralkoxy, heteroaralkoxy, C₁₋₆-alkylthio, arylthio, C₃₋₆-cycloalkylthio, C₁₋₆-alkylcarbonyl, arylcarbonyl, C₁₋₆-alkylsulfonyl, arylsulfonyl, C₁₋₆-alkylamido, arylamido, C₁₋₆-alkylaminocarbonyl, C₁₋₆-alkylamino, C₁₋₆-dialkylamino or C₃₋₆-cycloalkylamino each of which is optionally substituted with one or more halogens; or
X₃ is C₁₋₆-alkyl, C₃₋₆-cycloalkyl, C₂₋₆-alkenyl, carbamoyl or C₃₋₆-cycloalkyl-C₁₋₆-alkyl each of which is optionally substituted with one or more substituents selected from
- halogen, hydroxy, cyano, amino or carboxy; or
- C₁₋₆-alkyl, C₃₋₆-cycloalkyl, C₂₋₆-alkenyl, C₂₋₆-alkynyl, C₃₋₆-cycloalkyl-C₁₋₆-alkyl, aryl, aralkyl, heteroaryl, heteroaralkyl, heterocyclyl, C₁₋₆-alkoxy, C₃₋₆-cycloalkoxy, C₃₋₆-cycloalkyl-C₁₋₆-alkoxy, aryloxy, heteroaryloxy, aralkoxy, heteroaralkoxy, C₁₋₆-alkylthio, C₃₋₆-cycloalkylthio, C₃₋₆-cycloalkyl-C₁₋₆-alkylthio,arylthio, heteroarylthio, aryl-C₁₋₆-alkylthio, heteroaryl-C₁₋₆-alkylthio, C₁₋₆-alkylcarbonyl, C₃₋₆-cycloalkylcarbonyl, C₃₋₆-cycloalkyl- C₁₋₆-alkyl-carbonyl, arylcarbonyl, heteroarylcarbonyl, C₁₋₆-alkylsulfonyl, C₃₋₆-cycloalkylsulfonyl, C₃₋₆-cycloalkyl-C₁₋₆-alkylsulfonyl, arylsulfonyl, heteroarylsulfonyl, C₁₋₆-alkylsulfamoyl, di-(C₁₋₆-alkyl)sulfamoyl, C₁₋₆-alkoxycarbonyl, C₃₋₆-cycloalkoxycarbonyl, C₃₋₆-cycloalkyl-C₁₋₆-alkoxycarbonyl, amino-C₁₋₆-alkyl, C₁₋₆-alkylamino-C₁₋₆-alkyl, di-(C₁₋₆-alkyl)amino-C₁₋₆-alkyl, C₁₋₆-alkylamido, C₃₋₆-cycloalkylamido, C₃₋₆-cycloalkyl-C₁₋₆-alkylamido, arylamido, C₁₋₆-alkylaminocarbonyl, C₃₋₆-cycloalkylaminocarbonyl, C₃₋₆-cycloalkyl-C₁₋₆-alkylaminocarbonyl, di-(C₁₋₆-alkyl)aminocarbonyl, di-(C₃₋₆-cycloalkyl-C₁₋₆-alkyl)aminocarbony), C₁₋₆-alkylamino, C₃₋₆-cycloalkylamino, C₃₋₆-cycloalkyl-C₁₋₆-alkylamino, di-(C₁₋₆-alkyl)amino, di-(C₃₋₆-cycloalkyl)amino or di-(C₃₋₆-cycloalkyl-C₁₋₆-alkyl)amino each of which is optionally substituted with one or more of halogen, cyano, hydroxy, acetyl or oxo
X₄ is arylene or heteroarylene each of which is optionally substituted with one or more substituents selected from
- halogen, hydroxy, cyano, amino or carboxy; or
- C₁₋₆-alkyl, C₃₋₆-cycloalkyl, C₂₋₆-alkenyl, C₂₋₆-alkynyl, C₁₋₆-alkoxy, C₃₋₆-cycloalkoxy, C₁₋₆-alkylthio, C₃₋₆-cycloalkylthio, C₁₋₆-alkylamino, C₁₋₆-dialkylamino or C₃₋₆-cydoalkylamino each of which is optionally substituted with one or more halogens; and
Ar is arylene which is optionally substituted with one or more substituents selected from
- halogen, hydroxy or cyano; or
- C₁₋₆-alkyl, C₃₋₆-cycloalkyl, C₂₋₆-alkenyl, C₂₋₆-alkynyl, aryl, heteroaryl, aralkyl, heteroaralkyl, C₁₋₆-alkoxy, C₃₋₆-cycloalkoxy, aryloxy, aralkoxy, heteroaralkoxy, C₁₋₆-alkylthio, arylthio or C₃₋₆-cycloalkylthio each of which is optionally substituted with one or more halogens; or
- two of the substituents when placed in adjacent positions together with the atoms to which they are attached may form a five to eight member ring; and
Y₁ is O or S; and
Y₂ is O or S; and
Z is -(CH₂)ₙ- wherein n is 1, 2 or 3; and
R₁ is hydrogen, halogen or a substituent selected from
- C₁₋₆-alkyl, C₃₋₆-cycloalkyl, C₂₋₆-alkenyl, C₂₋₆-alkynyl, aralkyl, heteroaralkyl, C₁₋₆-alkoxy, C₃₋₆-cycloalkoxy, aryloxy, aralkoxy, heteroaralkoxy, C₁₋₆-alkylthio, arylthio or C₃₋₆-cycloalkylthio each of which is optionally substituted with one or more halogens; and
R₂ is hydrogen, C₁₋₆-alkyl, C₃₋₆-cycloalkyl, C₂₋₆-alkenyl, C₂₋₆-alkynyl, C₄₋₆-alkenynyl or aryl; or
a pharmaceutically acceptable salt thereof, or a pharmaceutically acceptable solvate thereof, or any tautomeric forms, stereoisomers, mixture of stereoisomers including a racemic mixture, or polymorphs.

In one embodiment, the present invention is concerned with compounds of formula (I) wherein X₁ is aryl optionally substituted with one or more substituents selected from
- halogen; or
- C₁₋₆-alkyl, aryl, C₁₋₆-alkoxy or C₁₋₆-alkylsulfonyl each of which is optionally substituted with one or more halogens.
   In another embodiment, the present invention is concerned with compounds of formula (I) wherein X₁ is aryl optionally substituted with one or more substituents selected from
- halogen; or
- C₁₋₆-alkyl optionally substituted with one or more halogens.

In another embodiment, the present invention is concerned with compounds of formula (I) wherein X₁ is aryl optionally substituted with halogen.

In another embodiment, the present invention is concerned with compounds of formula (I) wherein X₁ is phenyl optionally substituted with one or more substituents selected from
- halogen; or
- C₁₋₆-alkyl, aryl, C₁₋₆-alkoxy or C₁₋₆-alkylsulfonyl each of which is optionally substituted with one or more halogens.
   In another embodiment, the present invention is concerned with compounds of formula (I) wherein X₁ is phenyl optionally substituted with one or more substituents selected from
- halogen; or
- C₁₋₆-alkyl optionally substituted with one or more halogens.

In another embodiment, the present invention is concerned with compounds of formula (I) wherein X₁ is phenyl optionally substituted with one or more halogens.

In another embodiment, the present invention is concerned with compounds of formula (I) wherein X₁ is phenyl optionally substituted with one or more of methyl or ethyl.

In another embodiment, the present invention is concerned with compounds of formula (I) wherein X₁ is phenyl optionally substituted with one or more of perhalomethyl.

In another embodiment, the present invention is concerned with compounds of formula (I) wherein X₁ is phenyl.

In another embodiment, the present invention is concerned with compounds of formula (I) wherein X₁ is heteroaryl optionally substituted with one or more substituents selected from
- halogen; or
- C₁₋₆-alkyl, aryl, C₁₋₆-alkoxy or C₁₋₆-alkylsulfonyl each of which is optionally substituted with one or more halogens.

In another embodiment, the present invention is concerned with compounds of formula (I) wherein X₁ is heteroaryl optionally substituted with one or more substituents selected from
- halogen; or
- C₁₋₆-alkyl optionally substituted with one or more halogens.

In another embodiment, the present invention is concerned with compounds of formula (I) wherein X₁ is pyrrolyl, pyridyl, furyl or thienyl, each of which is optionally substituted with one or more of halogens or C₁₋₆-alkyl, which is optionally substituted with one or more halogens.

In another embodiment, the present invention is concerned with compounds of formula (I) wherein X₁ is pyrrolyl or pyridyl optionally substituted with one or more C₁₋₆-alkyl optionally substituted with one or more halogens.

In another embodiment, the present invention is concerned with compounds of formula (I) wherein X₁ is furyl or thienyl optionally substituted with one or more halogens.

In another embodiment, the present invention is concerned with compounds of formula (I) wherein X₁ is pyrrolyl optionally substituted with one or more C₁₋₆-alkyl.

In another embodiment, the present invention is concerned with compounds of formula (I) wherein X₁ is pyridyl optionally substituted with one or more C₁₋₆-alkyl.

In another embodiment, the present invention is concerned with compounds of formula (I) wherein X₁ is furyl optionally substituted with one or more halogens.

In another embodiment, the present invention is concerned with compounds of formula (I) wherein X₁ is thienyl optionally substituted with one or more halogens.

In another embodiment, the present invention is concerned with compounds of formula (I) wherein X₁ is benzothienyl or benzofuryl optionally substituted with one or more of C₁₋₆-alkyl.

In another embodiment, the present invention is concerned with compounds of formula (I) wherein X₁ is C₁₋₆-alkyl or carbamoyl optionally substituted with one or more substituents selected from
- halogen, hydroxy, cyano, amino or carboxy; or
- C₁₋₆-alkyl, C₃₋₆-cycloalkyl, C₂₋₆-alkenyl, C₂₋₆-alkynyl, C₃₋₆-cycloalkyl-C₁₋₆-alkyl, aryl, aralkyl, heteroaryl, heteroaralkyl, heterocyclyl, C₁₋₆-alkoxy, C₃₋₆-cycloalkoxy, C₃₋₆-cycloalkyl-C₁₋₆-alkoxy, aryloxy, heteroaryloxy, aralkoxy, heteroaralkoxy, C₁₋₆-alkylthio, C₃₋₆-cycloalkylthio, C₃₋₆-cycloalkyl-C₁₋₆-alkylthio,arylthio, heteroarylthio, aryl-C₁₋₆-alkylthio, heteroaryl-C₁₋₆-alkylthio, C₁₋₆-alkylcarbonyl, C₃₋₆-cycloalkylcarbonyl, C₃₋₆-cycloalkyl- C₁₋₆-alkyl-carbonyl, arylcarbonyl, heteroarylcarbonyl, C₁₋₆-alkylsulfonyl, C₃₋₆-cycloalkylsulfonyl, C₃₋₆-cycloalkyl-C₁₋₆-alkylsulfonyl, arylsulfonyl, heteroarylsulfonyl, C₁₋₆-alkylsulfamoyl, di-(C₁₋₆-alkyl)sulfamoyl, C₁₋₆-alkoxycarbonyl, C₃₋₆-cycloalkoxycarbonyl, C₃₋₆-cycloalkyl-C₁₋₆-alkoxycarbonyl, amino-C₁₋₆-alkyl, C₁₋₆-alkylamino-C₁₋₆-alkyl, di-(C₁₋₆-alkyl)amino-C₁₋₆-alkyl, C₁₋₆-alkylamido, C₃₋₆-cycloalkylamido, C₃₋₆-cycloalkyl-C₁₋₆-alkylamido, arylamido, C₁₋₆-alkylaminocarbonyl, C₃₋₆-cycloalkylaminocarbonyl, C₃₋₆-cycloalkyl-C₁₋₆-alkylaminocarbonyl, di-(C₁₋₆-alkyl)aminocarbonyl, di-(C₃₋₆cycloalkyl-C₁₋₆-alkyl)aminocarbonyl, C₁₋₆-alkylamino, C₃₋₆-cycloalkylamino, C₃₋₆-cycloalkyl-C₁₋₆-alkylamino, di-(C₁₋₆-alkyl)amino, di-(C₃₋₆-cycloalkyl)amino or di-(C₃₋₆-cycloalkyl-C₁₋₆-alkyl)amino, each of which is optionally substituted with one or more of halogen, cyano, hydroxy, acetyl or oxo.

In another embodiment, the present invention is concerned with compounds of formula (I) wherein X₁ is C₁₋₆-alkyl or carbamoyl optionally substituted with one or more substituents selected from
- halogen or hydroxy; or
- C₁₋₆-alkyl, aryl, heteroaryl, heterocyclyl, C₁₋₆-alkoxy, C₁₋₆-alkylthio, C₃₋₆-cycloalkylthio, C₃₋₆-cycloalkyl-C₁₋₆-alkylthio,arylthio, heteroarylthio, aryl-C₁₋₆-alkylthio, C₁₋₆-alkylcarbonyl, arylcarbonyl, C₁₋₆alkylsuffonyl, arylsulfonyl, amino-C₁₋₆-alkyl, C₁₋₆-alkylamino-C₁₋₆-alkyl, di-(C₁₋₆-alkyl)amino-C₁₋₆-alkyl, C₁₋₆-alkylamido, arylamido, C₁₋₆-alkylaminocarbonyl, di-(C₁₋₆-alkyl)-aminocarbonyl, C₁₋₆-alkylamino, di-(C₁₋₆-alkyl)amino, each of which is optionally substituted with one or more of halogen, cyano, hydroxy, acetyl or oxo.

In another embodiment, the present invention is concerned with compounds of formula (I) wherein X₁ is C₁₋₅-alkyl or carbamoyl optionally substituted with one or more substituents selected from C₁₋₆-alkyl, heteroaryl, heterocyclyl, C₁₋₆-alkylamino, di-(C₁₋₆-alkyl)-amino, each of which is optionally substituted with one or more of acetyl or oxo.

In another embodiment, the present invention is concerned with compounds of formula (I) wherein X₁ is C₁₋₆-alkyl optionally substituted with one or more substituents selected from C₁₋₆-alkyl, heteroaryl, heterocyclyl, C₁₋₆-alkylamino, di-(C₁₋₆-alkyl)amino, each of which is optionally substituted with one or more of acetyl or oxo.

In another embodiment, the present invention is concerned with compounds of formula (I) wherein X₁ is C₁₋₆-alkyl optionally substituted with one or more of C₁₋₆-alkyl.

In another embodiment, the present invention is concerned with compounds of formula (I) wherein X₁ is C₁₋₆-alkyl optionally substituted with heteroaryl.

In another embodiment, the present invention is concerned with compounds of formula (I) wherein X₁ is C₁₋₆-alkyl optionally substituted with one or more of heterocyclyl optionally substituted with one or more of acetyl or oxo.

In another embodiment, the present invention is concerned with compounds of formula (I) wherein X₁ is C₁₋₆-alkyl optionally substituted with one or more of morpholinyl, piperazinyl or pyrrolidinyl, each of which is optionally substituted with one or more of acetyl or oxo.

In another embodiment, the present invention is concerned with compounds of formula (I) wherein X₁ is C₁₋₆-alkyl optionally substituted with C₁₋₆-alkylamino optionally substituted with acetyl.

In another embodiment, the present invention is concerned with compounds of formula (I) wherein X₁ is C₁₋₆-alkyl optionally substituted with di-(C₁₋₆-alkyl)amino.

In another embodiment, the present invention is concerned with compounds of formula (I) wherein X₁ is carbamoyl optionally substituted with acetyl.

In another embodiment, the present invention is concerned with compounds of formula (I) wherein X₂ is arylene optionally substituted with one or more substituents selected from
- halogen or
- C₁₋₆-alkyl optionally substituted with one or more halogens.

In another embodiment, the present invention is concerned with compounds of formula (I) wherein X₂ is arylene optionally substituted with one or more halogens.

In another embodiment, the present invention is concerned with compounds of formula (I) wherein X₂ is phenylene optionally substituted with one or more substituents selected from
- halogen or
- C₁₋₆-alkyl optionally substituted with one or more halogens.

In another embodiment, the present invention is concerned with compounds of formula (I) wherein X₂ is phenylene optionally substituted with one or more halogens.

In another embodiment, the present invention is concerned with compounds of formula (I) wherein X₂ is phenylene optionally substituted with one or more of C₁₋₆-alkyl.

In another embodiment, the present invention is concerned with compounds of formula (I) wherein X₂ is phenylene.

In another embodiment, the present invention is concerned with compounds of formula (I) wherein X₂ is heteroarylene optionally substituted with one or more substituents selected from
- halogen or
- C₁₋₆-alkyl optionally substituted with one or more halogens.

In another embodiment, the present invention is concerned with compounds of formula (I) wherein X₂ is heteroarylene optionally substituted with one or more halogens. In another embodiment, the present invention is concerned with compounds of formula (I) wherein X₂ is heteroarylene.

In another embodiment, the present invention is concerned with compounds of formula (I) wherein X₂ is thienylene.

In another embodiment, the present invention is concerned with compounds of formula (I) wherein X₃ is aryl optionally substituted with one or more substituents selected from
- halogen; or
- C₁₋₆-alkyl, aryl, C₁₋₆-alkoxy or C₁₋₆-alkylsulfonyl each of which is optionally substituted with one or more halogens.

In another embodiment, the present invention is concerned with compounds of formula (I) wherein X₃ is aryl optionally substituted with one or more substituents selected from
- halogen; or
- C₁₋₆-alkyl optionally substituted with one or more halogens.

In another embodiment, the present invention is concerned with compounds of formula (I) wherein X₃ is phenyl optionally substituted with one or more substituents selected from
- halogen; or
- C₁₋₆-alkyl, aryl, C₁₋₆-alkoxy or C₁₋₆-alkylsulfonyl each of which is optionally substituted with one or more halogens.

In another embodiment, the present invention is concerned with compounds of formula (I) wherein X₃ is phenyl optionally substituted with one or more substituents selected from
- halogen; or
- C₁₋₆-alkyl optionally substituted with one or more halogens.

In another embodiment, the present invention is concerned with compounds of formula (I) wherein X₃ is phenyl optionally substituted with one or more halogens.

In another embodiment, the present invention is concerned with compounds of formula (I) wherein X₃ is phenyl optionally substituted with one or more of methyl or ethyl.

In another embodiment, the present invention is concerned with compounds of formula (I) wherein X₃ is phenyl optionally substituted with one or more of perhalomethyl.

In another embodiment, the present invention is concerned with compounds of formula (I) wherein X₃ is phenyl.

In another embodiment, the present invention is concerned with compounds of formula (I) wherein X₃ is heteroaryl optionally substituted with one or more substituents selected from
- halogen; or
- C₁₋₆-alkyl, aryl, C₁₋₆-alkoxy or C₁₋₆-alkylsulfonyl each of which is optionally substituted with one or more halogens.

In another embodiment, the present invention is concerned with compounds of formula (I) wherein X₃ is heteroaryl optionally substituted with one or more substituents selected from
- halogen; or
- C₁₋₆-alkyl optionally substituted with one or more halogens.

In another embodiment, the present invention is concerned with compounds of formula (I) wherein X₃ is pyrrolyl, pyridyl, furyl or thienyl, each of which is optionally substituted with one or more of halogens or C₁₋₆-alkyl, which is optionally substituted with one or more halogens.

In another embodiment, the present invention is concerned with compounds of formula (I) wherein X₃ is pyrrolyl or pyridyl optionally substituted with one or more C₁₋₆-alkyl optionally substituted with one or more halogens.

In another embodiment, the present invention is concerned with compounds of formula (I) wherein X₃ is furyl or thienyl optionally substituted with one or more halogens.

In another embodiment, the present invention is concerned with compounds of formula (I) wherein X₃ is pyrrolyl optionally substituted with one or more C₁₋₆-alkyl.

In another embodiment, the present invention is concerned with compounds of formula (I) wherein X₃ is pyridyl optionally substituted with one or more C₁₋₆-alkyl.

In another embodiment, the present invention is concerned with compounds of formula (I) wherein X₃ is furyl optionally substituted with one or more halogens.

In another embodiment, the present invention is concerned with compounds of formula (I) wherein X₃ is thienyl optionally substituted with one or more halogens.

In another embodiment, the present invention is concerned with compounds of formula (I) wherein X₃ is benzothienyl or benzofuryl optionally substituted with one or more of C₁₋₆-alkyl.

In another embodiment, the present invention is concerned with compounds of formula (I) wherein X₃ is C₁₋₆-alkyl or carbamoyl optionally substituted with one or more substituents selected from
- halogen, hydroxy, cyano, amino or carboxy; or
- C₁₋₆-alkyl, C₃₋₆-cycloalkyl, C₂₋₆-alkenyl, C₂₋₆-alkynyl, C₃₋₆-cycloalkyl-C₁₋₆-alkyl, aryl, aralkyl, heteroaryl, heteroaralkyl, heterocyclyl, C₁₋₆-alkoxy, C₃₋₆-cycloalkoxy, C₃₋₆-cycloalkyl-C₁₋₆-alkoxy, aryloxy, heteroaryloxy, aralkoxy, heteroaralkoxy, C₁₋₆-alkylthio, C₃₋₆-cycloalkylthio, C₃₋₆-cycloalkyl-C₁₋₆-alkylthio,arylthio, heteroarylthio, aryl-C₁₋₆-alkylthio, heteroaryl-C₁₋₆-alkylthio, C₁₋₆-alkylcarbonyl, C₃₋₆-cycloalkylcarbonyl, C₃₋₆-cycloalkyl- C₁₋₆-alkyl-carbonyl, arylcarbonyl, heteroarylcarbonyl, C₁₋₆-alkylsulfonyl, C₃₋₆-cycloalkylsulfonyl, C₃₋₆-cycloalkyl-C₁₋₆-alkylsulfonyl, arylsulfonyl, heteroarylsulfonyl, C₁₋₆-alkylsulfamoyl, di-(C₁₋₆-alkyl)sulfamoyl, C₁₋₆₋alkoxycarbonyl, C₃₋₆-cycloalkoxycarbonyl, C₃₋₆-cycloalkyl-C₁₋₅-alkoxycarbonyl, amino-C₁₋₆-alkyl, C₁₋₆-alkylamino-C₁₋₆-alkyl, di-(C₁₋₆-alkyl)amino-C₁₋₆-alkyl, C₁₋₆-alkylamido, C₃₋₆-cyloalkylamido, C₃₋₆-cycloalkyl-C₁₋₆-alkylamido, arylamido, C₁₋₆-alkylaminocarbonyl, C₃₋₆-cycloalkylaminocarbonyl, C₃₋₆-cycloalkyl-C₁₋₆-alkylaminocarbonyl, di-(C₁₋₆-alkyl)aminocarbonyl, di-(C₃₋₆-cycloalkyl-C₁₋₆-alkyl)aminocarbonyl, C₁₋₆-alkylamino, C₃₋₆-cycloalkylamino, C₃₋₆-cycloalkyl-C₁₋₆-alkylamino, di-(C₁₋₆-alkyl)amino, di-(C₃₋₆-cycloalkyl)amino or di-(C₃₋₆-cycloalkyl-C₁₋₆-alkyl)amino each of which is optionally substituted with one or more of halogen, cyano, hydroxy, acetyl or oxo.

In another embodiment, the present invention is concerned with compounds of formula (I) wherein X₃ is C₁₋₆-alkyl or carbamoyl optionally substituted with one or more substituents selected from
- halogen or hydroxy; or
- C₁₋₆-alkyl, aryl, heteroaryl, heterocyclyl, C₁₋₆-alkoxy, C₁₋₆-alkylthio, C₃₋₆-cycloalkylthio, C₃₋₆-cycloalkyl-C₁₋₆-alkylthio,arylthio, heteroarylthio, aryl-C₁₋₆-alkylthio, C₁₋₆-alkylcarbonyl, arylcarbonyl, C₁₋₆-alkylsulfonyl, arylsulfonyl, amino-C₁₋₆-alkyl, C₁₋₆-alkylamino-C₁₋₆-alkyl, di-(C₁₋₆-alkyl)amino-C₁₋₆-alkyl, C₁₋₆-alkylamido, arylamido, C₁₋₆-alkylaminocarbonyl, di-(C₁₋₆-alkyl)-aminocarbonyl, C₁₋₆-alkylamino, di-(C₁₋₆-alkyl)amino, each of which is optionally substituted with one or more of halogen, cyano, hydroxy, acetyl or oxo.

In another embodiment, the present invention is concerned with compounds of formula (I) wherein X₃ is C₁₋₆-alkyl or carbamoyl optionally substituted with one or more substituents selected from C₁₋₆-alkyl, heteroaryl, heterocyclyl, C₁₋₆-alkylamino, di-(C₁₋₆-alkyl)-amino, each of which is optionally substituted with one or more of acetyl or oxo.

In another embodiment, the present invention is concerned with compounds of formula (I) wherein X₃ is C₁₋₆-alkyl optionally substituted with one or more substituents selected from C₁₋₆-alkyl, heteroaryl, heterocyclyl, C₁₋₆-alkylamino, di-(C₁₋₆-alkyl)amino, each of which is optionally substituted with one or more of acetyl or oxo.

In another embodiment, the present invention is concerned with compounds of formula (I) wherein X₃ is C₁₋₆-alkyl optionally substituted with one or more of C₁₋₆-alkyl.

In another embodiment, the present invention is concerned with compounds of formula (I) wherein X₃ is C₁₋₆-alkyl optionally substituted with heteroaryl.

In another embodiment, the present invention is concerned with compounds of formula (I) wherein X₃ is C₁₋₆-alkyl optionally substituted with one or more of heterocyclyl optionally substituted with one or more of acetyl or oxo.

In another embodiment, the present invention is concerned with compounds of formula (I) wherein X₃ is C₁₋₆-alkyl optionally substituted with one or more of morpholinyl, piperazinyl or pyrrolidinyl, each of which is optionally substituted with one or more of acetyl or oxo.

In another embodiment, the present invention is concerned with compounds of formula (I) wherein X₃ is C₁₋₆-alkyl optionally substituted with C₁₋₆-alkylamino optionally substituted with acetyl.

In another embodiment, the present invention is concerned with compounds of formula (I) wherein X₃ is C₁₋₆-alkyl optionally substituted with di-(C₁₋₆-alkyl)amino.

In another embodiment, the present invention is concerned with compounds of formula (I) wherein X₃ is carbamoyl optionally substituted with acetyl.

In another embodiment, the present invention is concerned with compounds of formula (I) wherein X₄ is arylene optionally substituted with one or more substituents selected from
- halogen or
- C₁₋₆-alkyl optionally substituted with one or more halogens.

In another embodiment, the present invention is concerned with compounds of formula (I) wherein X₄ is arylene optionally substituted with one or more halogens.

In another embodiment, the present invention is concerned with compounds of formula (I) wherein X₄ is phenylene optionally substituted with one or more substituents selected from
- halogen or
- C₁₋₆-alkyl optionally substituted with one or more halogens.

In another embodiment, the present invention is concerned with compounds of formula (I) wherein X₄ is phenylene optionally substituted with one or more halogens.

In another embodiment, the present invention is concerned with compounds of formula (I) wherein X₄ is phenylene optionally substituted with one or more of C₁₋₆-alkyl.

In another embodiment, the present invention is concerned with compounds of formula (I) wherein X₄ is phenylene.

In another embodiment, the present invention is concerned with compounds of formula (I) wherein X₄ is heteroarylene optionally substituted with one or more substituents selected from
- halogen or
- C₁₋₆-alkyl optionally substituted with one or more halogens.

In another embodiment, the present invention is concerned with compounds of formula (I) wherein X₄ is heteroarylene optionally substituted with one or more halogens.

In another embodiment, the present invention is concerned with compounds of formula (I) wherein X₄ is heteroarylene.

In another embodiment, the present invention is concerned with compounds of formula (I) wherein X₄ is thienylene.

In another embodiment, the present invention is concerned with compounds of formula (I) wherein Ar is phenylene which is optionally substituted with one or more substituents selected from
- halogen, hydroxy or cyano; or
- C₁₋₆-alkyl, C₃₋₆-cycloalkyl, C₂₋₆-alkenyl, C₂₋₆-alkynyl, aryl, heteroaryl, aralkyl, heteroaralkyl, C₁₋₆-alkoxy, C₃₋₆-cycloalkoxy, aryloxy, aralkoxy, heteroaralkoxy, C₁₋₆-alkylthio, arylthio or C₃₋₆-cycloalkylthio each of which is optionally substituted with one or more halogens; or.
- two of the substituents when placed in adjacent positions, together with the atoms to which they are attached, may form a five to eight member ring.

In another embodiment, the present invention is concerned with compounds of formula (I) wherein Ar is phenylene which is optionally substituted with one or more substituents selected from
- halogen; or
- C₁₋₆-alkyl, C₁₋₆-alkoxy, aryloxy or aralkoxy each of which is optionally substituted with one
   or more halogens; or
- two of the substituents when placed in adjacent positions together with the atoms to which they are attached form a five membered carbon cycle.

In another embodiment, the present invention is concerned with compounds of formula (I) wherein Ar is phenylene which is optionally substituted with halogen.

In another embodiment, the present invention is concerned with compounds of formula (I) wherein Ar is phenylene which is optionally substituted with one or more of C₁₋₆-alkyl optionally substituted with one or more halogens.

In another embodiment, the present invention is concerned with compounds of formula (I) wherein Ar is phenylene which is optionally substituted with one or more of C₁₋₆-alkoxy optionally substituted with one or more halogens.

In another embodiment, the present invention is concerned with compounds of formula (I) wherein Ar is phenylene which is optionally substituted with one or more of aryloxy optionally substituted with one or more halogens.

In another embodiment, the present invention is concerned with compounds of formula (I) wherein Ar is phenylene which is optionally substituted with one or more of aralkoxy optionally substituted with one or more halogens.

In another embodiment, the present invention is concerned with compounds of formula (I) wherein Ar is phenylene which is optionally substituted with methyl.

In another embodiment, the present invention is concerned with compounds of formula (I) wherein Ar is phenylene.

In another embodiment, the present invention is concerned with compounds of formula (I) wherein Y₁ is S.

In another embodiment, the present invention is concerned with compounds of formula (I) wherein Y₁ is O.

In another embodiment, the present invention is concerned with compounds of formula (I) wherein Y₂ is O.

In another embodiment, the present invention is concerned with compounds of formula (I) wherein Y₂ is S.

In another embodiment, the present invention is concerned with compounds of formula (I) wherein n is 1.

In another embodiment, the present invention is concerned with compounds of formula (I) wherein R₁ is hydrogen or a substituent selected from
- C₁₋₆-alkyl, aralkyl, C₁₋₆-alkoxy, aryloxy, aralkoxy each of which is optionally substituted with one or more halogens.

In another embodiment, the present invention is concerned with compounds of formula (I) wherein R₁ is hydrogen or a substituent selected from
- C₁₋₆-alkyl, C₁₋₆-alkoxy each of which is optionally substituted with one or more halogens.

In another embodiment, the present invention is concerned with compounds of formula (I) wherein R₁ is hydrogen.

In another embodiment, the present invention is concerned with compounds of formula (I) wherein R₁ is methyl or ethyl.

In another embodiment, the present invention is concerned with compounds of formula (I) wherein R₁ is methoxy or ethoxy.

In another embodiment, the present invention is concerned with compounds of formula (I) wherein R₂ is hydrogen or C₁₋₆-alkyl.

In another embodiment, the present invention is concerned with compounds of formula (I) wherein R₂ is hydrogen.

In another embodiment, the present invention is concerned with compounds of formula (I) wherein R₂ is methyl or ethyl.

In another embodiment, the present invention is concerned with compounds of formula I wherein alkyl is methyl or ethyl.

In another embodiment, the present invention is concerned with compounds of formula I wherein alkenyl is vinyl or 1-propenyl.

In another embodiment, the present invention is concerned with compounds of formula I wherein alkynyl is 1-propynyl.

In another embodiment, the present invention is concerned with compounds of formula I wherein alkenynyl is 1-pentene-4-yne.

In another embodiment, the present invention is concerned with compounds of formula I wherein alkoxy is methoxy, ethoxy, isopropoxy or cyclopropoxy.

In another embodiment, the present invention is concerned with compounds of formula I wherein aryl is phenyl.

In another embodiment, the present invention is concerned with compounds of formula I wherein arylene is phenylene.

In another embodiment, the present invention is concerned with compounds of formula I wherein halogen is bromine, fluorine or chlorine.

In another embodiment, the present invention is concerned with compounds of formula I wherein perhalomethyl is trifluoromethyl.

In another embodiment, the present invention is concerned with compounds of formula I wherein perhalomethoxy is trifluoromethoxy,

In another embodiment, the present invention is concerned with compounds of formula I wherein heteroaryl is furyl or thienyl.

In another embodiment, the present invention is concerned with compounds of formula I wherein heteroaryl is pyrrolyl or pyridyl.

In another embodiment, the present invention is concerned with compounds of formula I wherein heteroaryl is benzofuryl or benzothienyl.

In another embodiment, the present invention is concerned with compounds of formula I wherein arylene is phenylene.

In another embodiment, the present invention is concerned with compounds of formula I wherein heteroarylene is thienylene.

In another embodiment, the present invention is concerned with compounds of formula I wherein aralkyl is benzyl.

In another embodiment, the present invention is concerned with compounds of formula I wherein aryloxy is phenoxy.

In another embodiment, the present invention is concerned with compounds of formula I wherein aralkoxy is benzyloxy.

In another embodiment, the present invention is concerned with compounds of formula I wherein the substituents R₁ and X₄ are arranged in a trans-configuration.

In another embodiment, the present invention is concerned with compounds of formula I wherein the substituents R₁ and X₄ are arranged in a cis-configuration.

In another embodiment, the present invention is concerned with compounds of formula I which are PPARδ agonists.

In another embodiment, the present invention is concerned with compounds of formula I which are selective PPARδ agonists.

Examples of compounds of the invention are:
{4-[3,3-Bis-(4-phenylethynyl-phenyl)-allylsulfanyl]-2-methyl-phenoxy}-acetic acid;
{4-[3,3-Bis-(4-phenylethynyl-phenyl)-allylsulfanyl]-2-bromo-phenoxy}-acetic acid; or
a salt thereof with a pharmaceutically acceptable acid or base, or any optical isomer or mixture of optical isomers, including a racemic mixture, or any tautomeric forms.

Other examples of compounds of the invention are:
[4-[3,3-Bis[4-[(thiofen-2-yl)ethylnyl]phenyl]allylsulfanyl]-2-methylphenoxy]acetic acid;
{4-[3,3-Bis-(4-thiophen-3-ylethynylphenyl)allylsulfanyl]-2-methylphenoxy}acetic acid;
[4-[3,3-Bis[4-[(pyridine-2-yl)ethylnyl]phenyl]allylsulfanyl]-2-methylphenoxy]acetic acid;
{4-[3,3-Bis-(4-pyridin-2-ylethynylphenyl)allyloxy]-2-methylphenoxy}acetic acid;
{4-[3,3-Bis-(4-furan-2-ylethynylphenyl)allylsulfanyl]-2-methylphenoxy}acetic acid;
(4-{3,3-Bis-[4-(1-methyl-1H-pyrrol-2-ylethynyl)phenyl]allylsulfanyl}-2-methylphenoxy)acetic acid;
[4-[3,3-Bis[4-[3-(morpholine-4-yl)propyn-1-yl]phenyl]allylsulfanyl]-2-methylphenoxy]acetic acid;
[4-[3,3-Bis[4-[3-(N,N-dimethylamino)propyn-1-yl]phenyl]allylsulfanyl]-2-methylphenoxy]acetic acid;
[4-[3,3-Bis[4-[3-(morpholine-4-yl)propynyl]phenyl]allyloxy]-2-methylphenoxy]acetic acid;
[4-(3,3-Bis-{4-[3-(4-acetyl-piperazin-1-yl)-prop-1-ynyl]-phenyl}allylsulfanyl)-2-methyl-phenoxy]acetic acid;
[4-[3,3-Bis[4-(3-pyrrolidin-1-yl-prop-1-ynyl)phenyl]allylsulfanyl]-2-methylphenoxy]acetic acid;
(4-{3,3-Bis-[4-(3-pyrrolidin-1-yl-prop-1-ynyl)phenyl]allyloxy}-2-methylphenoxy)acetic acid;
[4-[3,3-Bis[5-[3-(morpholine-4-yl)propynyl]thiophene-2-yl]allylsulfanyl]-2-methylphenoxy]-acetic acid;
[4-[3,3-Bis[5-[3-(N-acetyl-N-methylamino)propynyl]thiophene-2-yl]allylsulfanyl]-2-methylphenoxy]acetic acid;
[4-[3,3-Bis[4-[3,3,3-trimethylpropynyl]phenyl]allylsulfanyl]-2-methylphenoxy]acetic acid;
[4-[3,3-Bis[4-[3-(imidazol-1-yl)propynyl]phenyl]allylsulfanyl]-2-methylphenoxy]acetic acid;
[4-[3,3-Bis[4-[3-(2-oxopyrrolidin-1-yl)propynyl]phenyl]allylsulfanyl]-2-methylphenoxy]acetic acid;
[4-[3,3-Bis(4-methylcarbamoylethynylphenyl)allylsulfanyl]-2-methylphenoxy]acetic acid; or a salt thereof with a pharmaceutically acceptable acid or base, or any optical isomer or mixture of optical isomers, including a racemic mixture, or any tautomeric forms.

The present invention also encompasses pharmaceutically acceptable salts of the present compounds. Such salts include pharmaceutically acceptable acid addition salts, pharmaceutically acceptable base addition salts, pharmaceutically acceptable metal salts, ammonium and alkylated ammonium salts. Acid addition salts include salts of inorganic acids as well as organic acids. Representative examples of suitable inorganic acids include hydrochloric, hydrobromic, hydroiodic, phosphoric, sulfuric, nitric acids and the like. Representative examples of suitable organic acids include formic, acetic, trichloroacetic, trifluoroacetic, propionic, benzoic, cinnamic, citric, fumaric, glycolic, lactic, maleic, malic, malonic, mandelic, oxalic, picric, pyruvic, salicylic, succinic, methanesulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids, sulphates, nitrates, phosphates, perchlorates, borates, acetates, benzoates, hydroxynaphthoates, glycerophosphates, ketoglutarates and the like. Further examples of pharmaceutically acceptable inorganic or organic acid addition salts include the pharmaceutically acceptable salts listed in J. Pharm. Sci. 1977, 66, 2, which is incorporated herein by reference. Examples of metal salts include lithium, sodium, potassium, magnesium, zinc, calcium salts and the like. Examples of amines and organic amines include ammonium, methylamine, dimethylamine, trimethylamine, ethylamine, diethylamine, propylamine, butylamine, tetramethylamine, ethanolamine, diethanolamine, triethanolamine, meglumine, ethylenediamine, choline, N,N'-dibenzylethylenediamine, N-benzylphenylethylamine, N-methyl-D-glucamine, guanidine and the like. Examples of cationic amino acids include lysine, arginine, histidine and the like.

The pharmaceutically acceptable salts are prepared by reacting the compound of formula I with 1 to 4 equivalents of a base such as sodium hydroxide, sodium methoxide, sodium hydride, potassium t-butoxide, calcium hydroxide, magnesium hydroxide and the like, in solvents like ether, THF, methanol, t-butanol, dioxane, isopropanol, ethanol etc. Mixture of solvents may be used. Organic bases like lysine, arginine, diethanolamine, choline, guandine and their derivatives etc. may also be used. Alternatively, acid addition salts wherever applicable are prepared by treatment with acids such as hydrochloric acid, hydrobromic acid, nitric acid, sulfuric acid, phosphoric acid, p-toluenesulphonic acid, methanesulfonic acid, acetic acid, citric acid, maleic acid salicylic acid, hydroxynaphthoic acid, ascorbic acid, palmitic acid, succinic acid, benzoic acid, benzenesulfonic acid, tartaric acid and the like in solvents like ethyl acetate, ether, alcohols, acetone, THF, dioxane etc. Mixture of solvents may also be used.

The stereoisomers of the compounds forming part of this invention may be prepared by using reactants in their single enantiomeric form in the process wherever possible or by conducting the reaction in the presence of reagents or catalysts in their single enantiomer form or by resolving the mixture of stereoisomers by conventional methods. Some of the preferred methods include use of microbial resolution, enzymatic resolution, resolving the diastereomeric salts formed with chiral acids such as mandelic acid, camphorsulfonic acid, tartaric acid, lactic acid, and the like wherever applicable or chiral bases such as brucine, (R)- or (S)-phenylethylamine, cinchona alkaloids and their derivatives and the like. Commonly used methods are compiled by Jaques et al in "Enantiomers, Racemates and Resolution" (Wiley Interscience, 1981). More specifically the compound of formula I may be converted to a 1:1 mixture of diastereomeric amides by treating with chiral amines, aminoacids, aminoalcohols derived from aminoacids; conventional reaction conditions may be employed to convert acid into an amide; the dia-stereomers may be separated either by fractional crystallization or chromatography and the stereoisomers of compound of formula I may be prepared by hydrolysing the pure diastereomeric amide.

Various polymorphs of compound of general formula I forming part of this invention may be prepared by crystallization of compound of formula I under different conditions. For example, using different solvents commonly used or their mixtures for recrystallization; crystallizations at different temperatures; various modes of cooling, ranging from very fast to very slow cooling during crystallizations. Polymorphs may also be obtained by heating or melting the compound followed by gradual or fast cooling. The presence of polymorphs may be determined by solid probe nmr spectroscopy, ir spectroscopy, differential scanning calorimetry, powder X-ray diffraction or such other techniques.

The invention also encompasses prodrugs of the present compounds, which on administration undergo chemical conversion by metabolic processes before becoming active pharmacological substances. In general, such prodrugs will be functional derivatives of the present compounds, which are readily convertible in vivo into the required compound of the formula (I). Conventional procedures for the selection and preparation of suitable prodrug derivatives are described, for example, in "Design of Prodrugs", ed. H. Bundgaard, Elsevier, 1985.

The invention also encompasses active metabolites of the present compounds.

The invention also relates to pharmaceutical compositions comprising, as an active ingredient, at least one compound of the formula I or any optical or geometric isomer or tautomeric form thereof including mixtures of these or a pharmaceutically acceptable salt thereof together with one or more pharmaceutically acceptable carriers or diluents.

Furthermore, the invention relates to the use of compounds of the general formula I or their tautomeric forms, their stereoisomers, their polymorphs, their pharmaceutically acceptable salts or pharmaceutically acceptable solvates thereof for the preparation of a pharmaceutical composition for the treatment and/or prevention of conditions mediated by nuclear receptors, in particular the Peroxisome Proliferator-Activated Receptors (PPAR) such as the conditions mentioned above.

In another aspect, the present invention relates to a method of treating and/or preventing Type I or Type II diabetes.

In a still further aspect, the present invention relates to the use of one or more compounds of the general formula I or pharmaceutically acceptable salts thereof for the preparation of a pharmaceutical composition for the treatment and/or prevention of Type I or Type II diabetes.

In a still further aspect, the present compounds are useful for the treatment and/or prevention of IGT.

In a still further aspect, the present compounds are useful for the treatment and/or prevention of Type 2 diabetes.

In a still further aspect, the present compounds are useful for the delaying or prevention of the progression from IGT to Type 2 diabetes.

In a still further aspect, the present compounds are useful for the delaying or prevention of the progression from non-insulin requiring Type 2 diabetes to insulin requiring Type 2 diabetes.

In another aspect, the present compounds reduce blood glucose and triglyceride levels and are accordingly useful for the treatment and/or prevention of ailments and disorders such as diabetes and/or obesity.

In still another aspect, the present compounds are useful for the treatment and/or prophylaxis of insulin resistance (Type 2 diabetes), impaired glucose tolerance, dyslipidemia, disorders related to Syndrome X such as hypertension, obesity, insulin resistance, hyperglycaemia, atherosclerosis, artherosclerosis, hyperlipidemia, coronary artery disease, myocardial ischemia and other cardiovascular disorders.

In still another aspect, the present compounds are useful for the treatment and/or prophylaxis of diseases or complications related to atherosclerosis such as coronary artery diseases, coronary heart diseases, heart attack, myocardial infarct, coronary infarct, transient ischemic attack (TIA) or stroke.

In still another aspect, the present compounds are effective in decreasing apoptosis in mammalian cells such as beta cells of Islets of Langerhans.

In still another aspect, the present compounds are useful for the treatment of certain renal diseases including glomerulonephritis, glomerulosclerosis, nephrotic syndrome, hypertensive nephrosclerosis.

In still another aspect, the present compounds may also be useful for improving cognitive functions in dementia, treating diabetic complications, psoriasis, polycystic ovarian syndrome (PCOS) and prevention and treatment of bone loss, e.g. osteoporosis.

In yet another aspect, the invention also relates to the use of the present compounds, which after administration lower the bio-markers of atherosclerosis like, but not limited to, c-reactive protein (CRP), TNFα and IL-6.

The present compounds may also be administered in combination with one or more further pharmacologically active substances eg., selected from antiobesity agents, antidiabetics, antihypertensive agents, agents for the treatment and/or prevention of complications resulting from or associated with diabetes and agents for the treatment and/or prevention of complications and disorders resulting from or associated with obesity.

Thus, in a further aspect of the invention the present compounds may be administered in combination with one or more antiobesity agents or appetite regulating agents.

Such agents may be selected from the group consisting of CART (cocaine amphetamine regulated transcript) agonists, NPY (neuropeptide Y) antagonists, MC4 (melanocortin 4) agonists, orexin antagonists, TNF (tumor necrosis factor) agonists, CRF (corticotropin releasing factor) agonists, CRF BP (corticotropin releasing factor binding protein) antagonists, urocortin agonists, β3 agonists, MSH (melanocyte-stimulating hormone) agonists, MCH (melanocyte-concentrating hormone) antagonists, CCK (cholecystokinin) agonists, serotonin re-uptake inhibitors, serotonin and noradrenaline re-uptake inhibitors, mixed serotonin and noradrenergic compounds, 5HT (serotonin) agonists, bombesin agonists, galanin antagonists, growth hormone, growth hormone releasing compounds, TRH (thyreotropin releasing hormone) agonists, UCP 2 or 3 (uncoupling protein 2 or 3) modulators, leptin agonists, DA agonists (bromocriptin, doprexin), lipase/amylase inhibitors, RXR (retinoid X receptor) modulators or TR β agonists.

In one embodiment of the invention the antiobesity agent is leptin.

In another embodiment the antiobesity agent is dexamphetamine or amphetamine.

In another embodiment the antiobesity agent is fenfluramine or dexfenfluramine.

In still another embodiment the antiobesity agent is sibutramine.

In a further embodiment the antiobesity agent is orlistat.

In another embodiment the antiobesity agent is mazindol or phentermine.

Suitable antidiabetics comprise insulin, GLP-1 (glucagon like peptide-1) derivatives such as those disclosed in WO 98/08871 to Novo Nordisk A/S, which is incorporated herein by reference as well as orally active hypoglycaemic agents.

The orally active hypoglycaemic agents preferably comprise sulphonylureas, biguanides, meglitinides, glucosidase inhibitors, glucagon antagonists such as those disclosed in WO 99/01423 to Novo Nordisk A/S and Agouron Pharmaceuticals, Inc., GLP-1 agonists, potassium channel openers such as those disclosed in WO 97/26265 and WO 99/03861 to Novo Nordisk A/S which are incorporated herein by reference, DPP-IV (dipeptidyl peptidase-IV) inhibitors, inhibitors of hepatic enzymes involved in stimulation of gluconeogenesis and/or glycogenolysis, glucose uptake modulators, compounds modifying the lipid metabolism such as antihyperlipidemic agents and antilipidemic agents as HMG CoA inhibitors (statins), compounds lowering food intake, RXR agonists and agents acting on the ATP-dependent potassium channel of the β-cells.

In one embodiment of the invention the present compounds are administered in combination with insulin.

In a further embodiment the present compounds are administered in combination with a sulphonylurea eg. tolbutamide, glibenclamide, glipizide or glicazide.

In another embodiment the present compounds are administered in combination with a biguanide eg. metformin.

In yet another embodiment the present compounds are administered in combination with a meglitinide eg. repaglinide or senaglinide.

In a further embodiment the present compounds are administered in combination with an α-glucosidase inhibitor eg. miglitol or acarbose.

In another embodiment the present compounds are administered in combination with an agent acting on the ATP-dependent potassium channel of the β-cells eg. tolbutamide, glibenclamide, glipizide, glicazide or repaglinide.

Furthermore, the present compounds may be administered in combination with nateglinide.

In still another embodiment the present compounds are administered in combination with an antihyperlipidemic agent or antilipidemic agent eg. cholestyramine, colestipol, clofibrate, gemfibrozil, fenofibrate, bezafibrate, tesaglitazar, EML-4156, LY-518674, LY-519818, MK-767, atorvastatin, fluvastatin, lovastatin, pravastatin, simvastatin, cerivastin, acipimox, ezetimibe probucol, dextrothyroxine or nicotinic acid.

In yet another embodiment the present compounds are administered in combination with a thiazolidinedione e.g. troglitazone, ciglitazone, pioglitazone or rosiglitazone.

In a further embodiment the present compounds are administered in combination with more than one of the above-mentioned compounds eg. in combination with a sulphonylurea and metformin, a sulphonylurea and acarbose, repaglinide and metformin, insulin and a sulphonylurea, insulin and metformin, insulin, insulin and lovastatin, etc.

Furthermore, the present compounds may be administered in combination with one or more antihypertensive agents. Examples of antihypertensive agents are β-blockers such as alprenolol, atenolol, timolol, pindolol, propranolol and metoprolol, ACE (angiotensin converting enzyme) inhibitors such as benazepril, captopril, enalapril, fosinopril, lisinopril, quinapril and ramipril, calcium channel blockers such as nifedipine, felodipine, nicardipine, isradipine, nimodipine, diltiazem and verapamil, and α-blockers such as doxazosin, urapidil, prazosin and terazosin. Further reference can be made to Remington: The Science and Practice of Pharmacy, 19th Edition, Gennaro, Ed., Mack Publishing Co., Easton, PA, 1995.

It should be understood that any suitable combination of the compounds according to the invention with one or more of the above-mentioned compounds and optionally one or more further pharmacologically active substances are considered to be within the scope of the present invention.

The present invention also relates to a process for the preparation of the above said novel compounds, their derivatives, their analogs, their tautomeric forms, their stereoisomers, their polymorphs, their pharmaceutically acceptable salts or pharmaceutically acceptable solvates.

### PHARMACEUTICAL COMPOSITIONS

The compounds of the invention may be administered alone or in combination with pharmaceutically acceptable carriers or excipients, in either single or multiple doses. The pharmaceutical compositions according to the invention may be formulated with pharmaceutically acceptable carriers or diluents as well as any other known adjuvants and excipients in accordance with conventional techniques such as those disclosed in Remington: The Science and Practice of Pharmacy, 19th Edition, Gennaro, Ed., Mack Publishing Co., Easton, PA, 1995. The compositions may appear in conventional forms, for example capsules, tablets, aerosols, solutions, suspensions or topical applications.

Typical compositions include a compound of formula I or a pharmaceutically acceptable acid addition salt thereof, associated with a pharmaceutically acceptable excipient which may be a carrier or a diluent or be diluted by a carrier, or enclosed within a carrier which can be in the form of a capsule, sachet, paper or other container. In making the compositions, conventional techniques for the preparation of pharmaceutical compositions may be used. For example, the active compound will usually be mixed with a carrier, or diluted by a carrier, or enclosed within a carrier which may be in the form of a ampoule, capsule, sachet, paper, or other container. When the carrier serves as a diluent, it may be solid, semi-solid, or liquid material which acts as a vehicle, excipient, or medium for the active compound. The active compound can be adsorbed on a granular solid container for example in a sachet. Some examples of suitable carriers are water, salt solutions, alcohols, polyethylene glycols, polyhydroxyethoxylated castor oil, peanut oil, olive oil, gelatine, lactose, terra alba, sucrose, cyclodextrin, amylose, magnesium stearate, talc, gelatin, agar, pectin, acacia, stearic acid or lower alkyl ethers of cellulose, silicic acid, fatty acids, fatty acid amines, fatty acid monoglycerides and diglycerides, pentaerythritol fatty acid esters, polyoxyethylene, hydroxymethylcellulose and polyvinylpyrrolidone. Similarly, the carrier or diluent may include any sustained release material known in the art, such as glyceryl monostearate or glyceryl distearate, alone or mixed with a wax. The formulations may also include wetting agents, emulsifying and suspending agents, preserving agents, sweetening agents or flavouring agents. The formulations of the invention may be formulated so as to provide quick, sustained, or delayed release of the active ingredient after administration to the patient by employing procedures well known in the art.

The pharmaceutical compositions can be sterilized and mixed, if desired, with auxiliary agents, emulsifiers, salt for influencing osmotic pressure, buffers and/or colouring substances and the like, which do not deleteriously react with the active compounds.

The route of administration may be any route, which effectively transports the active compound to the appropriate or desired site of action, such as oral, nasal, pulmonary, transdermal or parenteral e.g. rectal, depot, subcutaneous, intravenous, intraurethral, intramuscular, intranasal, ophthalmic solution or an ointment, the oral route being preferred.

If a solid carrier is used for oral administration, the preparation may be tabletted, placed in a hard gelatin capsule in powder or pellet form or it can be in the form of a troche or lozenge. If a liquid carrier is used, the preparation may be in the form of a syrup, emulsion, soft gelatin capsule or sterile injectable liquid such as an aqueous or non-aqueous liquid suspension or solution.

For nasal administration, the preparation may contain a compound of formula I dissolved or suspended in a liquid carrier, in particular an aqueous carrier, for aerosol application. The carrier may contain additives such as solubilizing agents, e.g. propylene glycol, surfactants, absorption enhancers such as lecithin (phosphatidylcholine) or cyclodextrin, or preservatives such as parabenes.

For parenteral application, particularly suitable are injectable solutions or suspensions, preferably aqueous solutions with the active compound dissolved in polyhydroxylated castor oil.

Tablets, dragees, or capsules having talc and/or a carbohydrate carrier or binder or the like are particularly suitable for oral application. Preferable carriers for tablets, dragees, or capsules include lactose, corn starch, and/or potato starch. A syrup or elixir can be used in cases where a sweetened vehicle can be employed.

A typical tablet which may be prepared by conventional tabletting techniques may contain:

| Core: | |
|---|---|
| Active compound (as free compound or salt thereof) | 5 mg |
| Colloidal silicon dioxide (Aerosil) | 1.5 mg |
| Cellulose, microcryst. (Avicel) | 70 mg |
| Modified cellulose gum (Ac-Di-Sol) | 7.5 mg |
| Magnesium stearate | Ad. |

| Coating: | |
|---|---|
| HPMC approx. | 9 mg |
| *Mywacett 9-40 T approx. | 0.9 mg |

| | |
|---|---|
| *Acylated monoglyceride used as plasticizer for film coating. | |

If desired, the pharmaceutical composition of the invention may comprise the compound of formula (I) in combination with further pharmacologically active substances such as those described in the foregoing.

The compounds of the invention may be administered to a mammal, especially a human in need of such treatment, prevention, elimination, alleviation or amelioration of diseases related to the regulation of blood sugar.

Such mammals include also animals, both domestic animals, e.g. household pets, and non-domestic animals such as wildlife.

The compounds of the invention are effective over a wide dosage range. A typical oral dosage is in the range of from about 0.001 to about 100 mg/kg body weight per day, preferably from about 0.01 to about 50 mg/kg body weight per day, and more preferred from about 0.05 to about 10 mg/kg body weight per day administered in one or more dosages such as 1 to 3 dosages. The exact dosage will depend upon the frequency and mode of administration, the sex, age, weight and general condition of the subject treated, the nature and severity of the condition treated and any concomitant diseases to be treated and other factors evident to those skilled in the art.

The formulations may conveniently be presented in unit dosage form by methods known to those skilled in the art. A typical unit dosage form for oral administration one or more times per day such as 1 to 3 times per day may contain of from 0.05 to about 1000 mg, preferably from about 0.1 to about 50.0 mg, and more preferred from about 0.5 mg to about 200 mg.

Any novel feature or combination of features described herein is considered essential to this invention.

### EXAMPLES

The following examples and general procedures refer to intermediate compounds and final products identified in the specification and in the synthesis schemes. The preparation of the compounds of the present invention is described in detail using the following examples. Occasionally, the reaction may not be applicable as described to each compound included within the disclosed scope of the invention. The compounds for which this occurs will be readily recognised by those skilled in the art. In these cases the reactions can be successfully performed by conventional modifications known to those skilled in the art, that is, by appropriate protection of interfering groups, by changing to other conventional reagents, or by routine modification of reaction conditions. Alternatively, other reactions disclosed herein or otherwise conventional will be applicable to the preparation of the corresponding compounds of the invention. In all preparative methods, all starting materials are known or may easily be prepared from known starting materials. The structures of the compounds are confirmed nuclear magnetic resonance (NMR). NMR shifts (δ) are given in parts per million (ppm. Mp is melting point and is given in °C.

The abbreviations as used in the examples have the following meaning:
- THF:: tetrahydrofuran
- DMSO:: dimethylsulfoxide
- CDCl₃:: deutorated chloroform
- DMF:: N,N-dimethylformamide
- min:: minutes
- h:: hours

### General procedure (A)

### Step A:

Reacting a compound of formula II 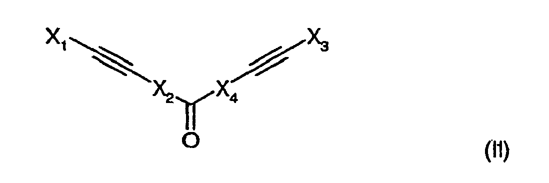 wherein X₁, X₂, X₃ and X₄ are defined as above, through a Horner-Emmons-like process with for example (EtO)₂PO(CHR₁)COOR₆ (wherein R₆ is an alkyl group), in the presence of a base such as sodium hydride, EtONa and the like to give a compound of formula III 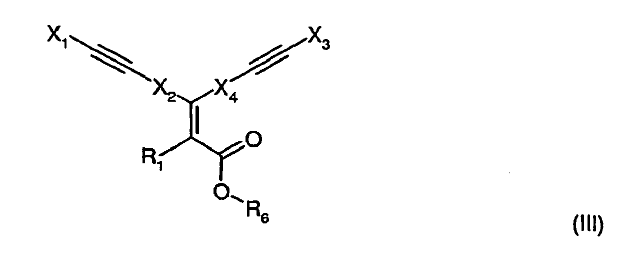 wherein X₁, X₂, X₃, X₄, R₁ and R₆ are defined as above

### Step B:

Reducing the compound of formula III, wherein X₁, X₂, X₃, X₄, R₁ and R₆ are defined as above with a suitable reagent such as diisobutylaluminium hydride, to give a compound of formula IV 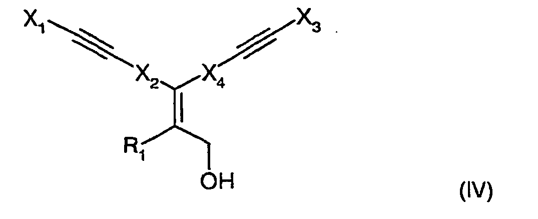 wherein X₁, X₂, X₃, X₄ and R₁ are defined as above, and

### Step C:

Reacting the compound of formula IV, wherein X₁, X₂, X₃, X₄, and R₁ are defined as above, (except that when X₁, X₂, X₃ or X₄, are substituted with hydroxy, this functionality has to be protected) with a compound of formula V 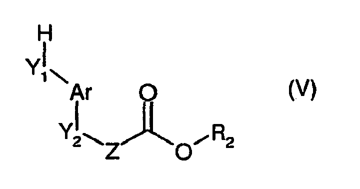 wherein Y₁, Ar, Y₂, Z and R₂ are defined as above, except that R₂ is not hydrogen, under Mitsunobu conditions, using a reagent such as triphenylphosphine/diethylazodicarboxylate and the like, to obtain a compound of formula I, wherein X₁, X₂, X₃, X₄, Y₁, Y₂, Ar, Z, R₁ and R₂ are defined as above, except that R₂ is not hydrogen.

### General procedure (B)

### Step A:

Converting the -OH functionality in the compound of formula IV, wherein X₁, X₂, X₃, X₄, and R₁ are defined as above, to an appropriate leaving group (L) such as p-toluenesulfonate, methanesulfonate, halogen (for example by methods according to: Houben-Weyl, Methoden der organischen Chemie, Alkohole III, 6/1 b, Thieme-Verlag 1984, 4th Ed., pp. 927-939; Comprehensive Organic Transformations. A guide to functional group preparations, VCH Publishers 1989, 1st Ed., pp. 353-363 and J. Org. Chem. ,Vol. 36 (20), 3044-3045,1971), triflate and the like, to give a compound of formula VI 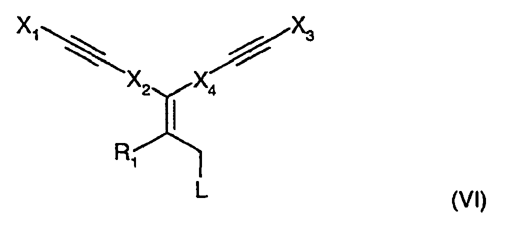 wherein, X₁, X₂, X₃, X₄, R₁ and L are defined as above.

### Step B_{:}

Reacting the compound of formula VI wherein L is a leaving group such as p-toluenesulfonate, methanesulfonate, halogen, triflate and the like and wherein X₁, X₂, X₃, X₄ and R₁ are defined as above with a compound of formula V wherein Y₁, Ar, Y₂, Z and R₂ are defined as above, except that R₂ is not hydrogen, to give a compound of formula I wherein X₁, X₂, X₃, X₄, Y₁, Y₂, Ar, Z, R₁ and R₂ are defined as above, except that R₂ is not hydrogen.

### General procedure (C)

### Step A:

Reacting an compound of formula VII 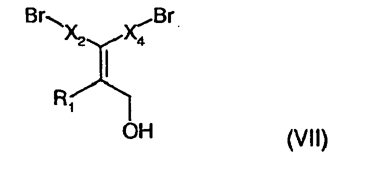 wherein X₂, X₄ and R₁ are defined as above, with an acetylene derivative of X₁ or X₃, wherein X₁ and X₃ are as defined above, under appropriate coupling conditions as Pd₂(dba)₃/Pd(P(t-Bu)₃)₂/Cul/iPr₂NH/THF, to give a compound of formula IV, wherein X₁, X₂, X₃, X₄ and R₁ are defined as above, and

### Step B:

Reacting a compound of formula IV as described under procedure A step C, to obtain a compound of formula I, wherein X₁, X₂, X₃, X₄, Y₁, Y₂, Ar, Z, R₁ and R₂ are defined as above, except that R₂ is not hydrogen.

### General procedure (D)

### Step A:

By chemical or enzymatic saponification of a compound of formula I wherein X₁, X₂, X₃, X₄, Y₁, Y₂, Ar, Z, R₁ and R₂ are defined as above, except that R₂ is not hydrogen, to give a compound of formula I wherein X₁, X₂, X₃, X₄, Y₁, Y₂, Ar, Z, R₁ and R₂ are defined as above, except that R₂ is hydrogen.

### General procedure (E)

### Step A:

Reacting a compound of formula VIII 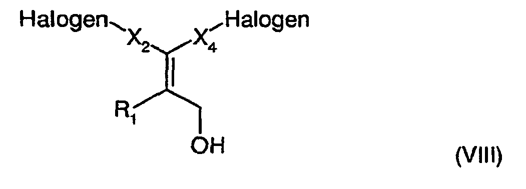 wherein X₂, X₄ and R₁ are defined as above, with a compound of formula V, wherein Y₁, Ar, Y₂, Z and R₂ are defined as above, except that R₂ is not hydrogen, under Mitsunobu conditions, using a reagent such as triphenylphosphine/diethylazodicarboxylate and the like, to obtain a compound of formula IX 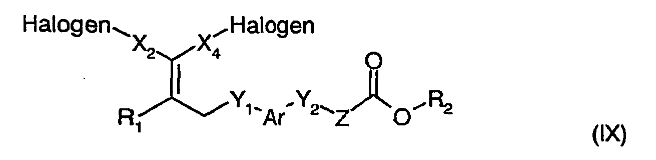 wherein X₂, X₄, Y₁, Y₂, Ar, Z, R₁ and R₂ are defined as above, except that R₂ is not hydrogen.

### Step B:

Reacting a compound of formula IX, wherein X₂, X₄, Y₁, Y₂, Ar, Z, R₁ and R₂ are defined as above, except that R₂ is not hydrogen, with an acetylene derivative of X₁ or X₃ , wherein X₁ and X₃ are as defined above, under appropriate coupling conditions as Pd₂(dba)₃/Pd(P(t-Bu)₃)₂/Cul/iPr₂NH/THF, to give a compound of formula I, wherein X₁, X₂, X₃, X₄, Y₁, Y₂, Ar, Z, R₁ and R₂ are defined as above, except that R₂ is not hydrogen.

### Step C:

Reacting a compound of formula IX, wherein X₂, X₄, Y₁, Y₂, Ar, Z, R₁ and R₂ are defined as above, except that R₂ is not hydrogen, with protected acetylene under appropriate coupling conditions as Pd₂(dba)₃/Pd(P(t-Bu)₃)₂/Cul/iPr₂NH/THF, to give a compound of formula I, wherein X₂, X₄, Y₁, Y₂, Ar, Z, R₁ and R₂ are defined as above, except that R₂ is not hydrogen and X₁ and X₃ are hydrogen.

### Step D:

Reacting a compound of formula I, wherein X₂, X₄, Y₁, Y₂, Ar, Z, R₁ and R₂ are defined as above, except that R₂ is not hydrogen and X₁ and X₃ are hydrogen, with an halogen derivative of X₁ or X₃ , wherein X₁ and X₃ are as defined above, under appropriate coupling conditions as Pd₂(dba)₃/Pd(P(t-Bu)₃)₂/Cul/iPr₂NH/THF, to give a compound of formula I, wherein X₁, X₂, X₃, X₄, Y₁, Y₂, Ar, Z, R₁ and R₂ are defined as above, except that R₂ is not hydrogen.

### General procedure (F).

### Step A:

Converting the -OH functionality in the compound of formula VIII, wherein X₂, X₄ and R₁ are defined as above, to an appropriate leaving group (L) such as p-toluenesulfonate, methanesulfonate, halogen (for example by methods according to: Houben-Weyl, Methoden der organischen Chemie, Alkohole III, 6/1 b, Thieme-Verlag 1984, 4th Ed., pp. 927-939; Comprehensive Organic Transformations. A guide to functional group preparations, VCH Publishers 1989, 1st Ed., pp. 353-363 and J. Org. Chem. ,Vol. 36 (20), 3044-3045, 1971), triflate and the like, to give a compound of formula X: 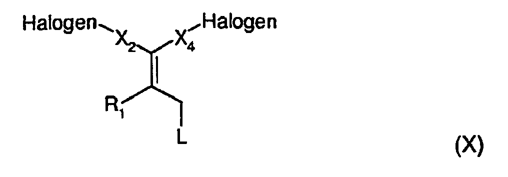 wherein X₂, X₄ and R₁ are defined as above.

### Step B:

Reacting the compound of formula X wherein L is a leaving group such as p-toluenesulfonate, methanesulfonate, halogen, triflate and the like and wherein X₂, X₄ and R₁ are defined as above with a compound of formula V wherein Y₁, Ar, Y₂, Z and R₂ are defined as above, except that R₂ is not hydrogen, to give a compound of formula IX, wherein X₂, X₄, Y₁, Y₂, Ar, Z, R₁ and R₂ are defined as above, except that R₂ is not hydrogen.

### Example 1

### [4-[3,3-Bis[4-(phenylethinyl)phenyl]allylsulfanyl]-2-methylphenoxy]acetic acid

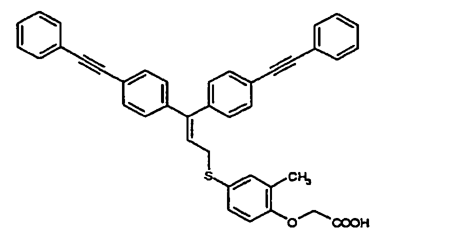

### General procedure (A)

### Step A:

A solution of triethyl phosphonoacetate (7.60 g, 34 mmol) in benzene (30 mL) was added to a suspension of sodium hydride (60 % suspension in oil; 1.40 g, 35 mmol) in benzene (30 mL) under stirring at ambient temperature. After 30 min, a solution of 4,4'-(phenylethinyl)benzophenone (6.50 g, 17.0 mmol; prepared as described in Izv. Akad. Nauk SSSR, Ser.Chim. 1996, 670) in a mixture of benzene and N,N-dimethylformamide (1:1, 60 mL) was added. The resulting mixture was stirred for 20 h, quenched with 5% aqueous hydrochloric acid (50 mL) and washed with water (20 mL). The combined organic solutions were dried with anhydrous magnesium sulfate and evaporated *in vacuo* to give ethyl 3,3-bis[4-(phenylethinyl)phenyl]acrylate.
Yield: 7.50 g (97 %).
M.p. 116-118 °C.
R_{F} (SiO₂, benzene) 0.40.
¹H NMR spectrum (300 MHz, CDCl₃, δ_{H}): 7.51-7.59 (m, 6 H); 7.49 (d, J=8.3 Hz, 2 H); 7.35 (m, 6 H); 7.27 (d, J=8.3 Hz, 2 H); 7.20 (d, J=8.3 Hz, 2 H); 6.40 (s, 1 H); 4.07 (q, J=7.2, 2 H); 1.14 (t, J=7.2 Hz, 3 H).
For C₃₃H₂₄O₂:
Calculated: C, 87.58%; H, 5.35%;
Found: C, 87.09%; H, 5.39%.

### Step B:

In atmosphere of nitrogen, diisobutylaluminium hydride (16% solution in tetrahydrofuran; 20 mL,18.0 mmol) was added dropwise to a solution of the above ester (3.0 g, 6.6 mmol) in tetrahydrofuran (30 mL) at -10 °C and the reaction mixture was stirred for 48 h at ambient temperature. The mixture was quenched with methanol (10 mL), water (30 mL) and concentrated hydrochloric acid (10 mL) and extracted with ethyl acetate (3 x 30 mL). The combined organic layers were dried with anhydrous potassium carbonate and evaporated in *vacuo.* The residue was submitted to column chromatography (silica gel Fluka 60, chloroform) yielding 3,3-bis[4-(phenylethinyl)phenyl]allyl alcohol as white solid beside of unreacted starting ester (1.2 g).
Yield: 1.50 g (92% calculated on converted starting compound).
M.p. 104-104.5°C.
R_{F} (SiO₂, chloroform) 0.30.
¹H NMR spectrum (200 MHz, CDCl₃, δ_{H}): 7.50-7.58 (m, 6 H); 7.46 (dt, 2 H); 7.34 (m, 6 H); 7.23 (dt, 2 H); 7.15 (dt, 2 H); 6.28 (t, J=6.8 Hz, 1 H); 4.23 (d, J=6.8 Hz, 2 H); 1.60 (s, 1H).

### General procedure (B)

### Step A:

Triphenylphosphine (2.62 g, 10.0 mmol) and subsequently tetrabromomethane (3.32 g, 10.0 mmol) were added to a cooled solution of the above allyl alcohol (3.2 g, 7.80 mmol) in dry methylene chloride (60 mL). The reaction mixture was stirred for 2 h at ambient temperature, washed with water (15 mL), dried with anhydrous magnesium sulfate and filtered through a short path of silica gel (Fluka 60) yielding 3,3-bis[4-(phenylethinyl)phenyl]allyl bromide.
Crude yield: 3.70 g (100%).
R_{F} (SiO₂, benzene) 0.70.
¹H NMR spectrum (200 MHz, CDCl₃, δ_{H}): 7.18-7.64 (m, 18 H); 6.38 (t, J=8.5 Hz, 1H); 4.05 (d, J=8.5 Hz, 2 H).

### Step B:

In atmosphere of nitrogen, N,N-diisopropylethylamine (0.70 g, 5.30 mmol) and subsequently a solution of ethyl (4-mercapto-2-methylphenoxy)acetate (1.00 g, 4.40 mmol) in dry tetrahydrofuran (1 mL) were added dropwise to a solution of the above bromide (1.90 g, 4.00 mmol) in dry tetrahydrofuran (15 mL). The resulting mixture was stirred overnight, filtered and the filtrate was evaporated *in vacuo.* The residue was submitted to column chromatography (silica gel Fluka 60, benzene) yielding ethyl [4-[3,3-bis[4-(phenylethinyl)phenyl]-allylsulfanyl]-2-methylphenoxy]acetate.
Yield: 0.55 g (28 %).
M.p. --- (oil).
R_{F} (SiO₂, benzene) 0.40.
¹H NMR spectrum (200 MHz, CDCl₃, δ_{H}): 7.30-7.60 (m, 14 H); 7.13 (m, 4 H); 6.89 (m, 2 H); 6.57 (d, J=9.0 Hz, 1 H); 6.18 (t, J=7.9 Hz, 1 H); 4.61 (s, 2 H); 4.24 (q, J=7.2 Hz, 2 H); 3.53 (d, J=7.9 Hz, 2 H); 2.22 (s, 3 H); 1.25 (t, J=7.2 Hz, 3 H).

### General procedure (D)

### Step A:

The above ester (0.55 g, 0.890 mmol) was dissolved in ethanol (50 mL) and tetrahydrofuran (5 mL). 20% aqueous solution of sodium hydroxide (1 mL, 5 mmol) was added and the mixture was left to stand for 48 h. The solvents were evaporated *in vacuo,* the residue was dissolved in water (10 mL) and the product was precipitated by addition of hydrochloric acid. The solid mass was filtered with suction, washed with water (5 mL) and dried yielding the title compound as amorphous solid.
Yield: 0.45 g (82 %).
R_{F} (SiO₂, chloroform/ethanol 10:1) 0.35.
¹H NMR spectrum (200 MHz, CDCl₃, δ_{H}): 7.30-7.60 (m, 14 H); 7.13 (m, 4 H); 6.91 (d, 2 H); 6.58 (d, J=9.0 Hz, 1 H);6.18 (t, J=7.9 Hz, 1 H); 4.67 (s, 2 H); 3.52 (d, J=7.9 Hz, 2 H); 2.21 (s, 3 H).

A mixture of the title acid (0.45 g, 0.730 mmol) and (L)-lysine (0.11 g, 0.730 mmol) was dissolved in methanol (35 mL), the solvent was evaporated *in vacuo* and the residue was triturated with ether yielding L-lysinate of the title compound as dihydrate.
Yield: 0.50 g (90 %).
M.p. 163-179°C.
For C₄₀H₃₀O₃S, C₆H₁₄N₂0O₂, 2 H₂O:
Calculated: C, 71.48%; H, 6.26%; N, 3.62%; S, 4.15%;
Found: C, 71.00%; H, 6.54%; N, 3.59%; S, 4.28%.

### Example 2

### 4-[3,3-Bis[4-(phenylethynyl)phenyl]allylsulfanyl]-2-bromophenoxy]acetic acid

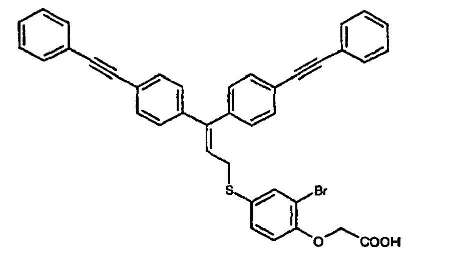

### 4,4'-dithiobis[(2-bromophenoxy)acetic acid diethyl ester]:

Chlorosulfonic acid (168 g, 1.44 mol) was cooled to -5°C and ethyl (2-bromophenoxy)acetate (93.0 g, 0.36 mol; prepared as described in J. Org. Chem. 1962, 27, 3010) was added dropwise under stirring at a such rate that the temperature of the reaction mixture did not exceed 0 °C (about 20 min). The mixture was left to warm up to ambient temperature (1 h) and then poured on crushed ice (1 kg). The product was extracted with dichloromethane (3 x 250 mL); the combined organic solutions were dried with anhydrous magnesium sulfate and evaporated *in vacuo* yielding crude ethyl (2-bromo-4-chlorosulfonylphenoxy)acetate, which was used in the next step without purification.
Yield: 40.0 g (31 %).

A solution of the above ester (40.0 g, 0.112 mol) in acetic acid (80 mL) was added dropwise to a stirred mixture of red phosphorus (10.0 g, 0.322 mol), iodine (1.38 g, 55.0 mmol) and acetic acid (80 mL) at 80°C. The reaction mixture was then refluxed for 3 h, water (20 mL) was added and the whole mixture was refluxed for further 2 h. After cooling, red phosphorus was filtered off and washed with acetic acid (2 x 20 mL). The filtrate was diluted with water (600 mL) and the product was isolated by extraction with chloroform (3 x 300 mL). The combined organic extracts were dried with anhydrous sodium sulphate and evaporated *in vacuo* yielding 4,4'-dithiobis[(2-bromophenoxy)acetic acid].
Yield: 23.0 g (78 %).
M.p. 146-150°C.

A mixture of the above acid (23.0 g, 87.0 mmol), sulfuric acid (25.0 g, 261 mmol) and ethanol (250 mL) was refluxed for 12 h, ethanol was evaporated *in vacuo* and the residue was dissolved in ether (200 mL). The solution was washed with water (2 x 50 mL) and 5 % aqueous solution of sodium hydrogen carbonate (2 x 50 mL) and subsequently was dried with anhydrous magnesium sulfate. The organic solution was evaporated *in vacuo* and the residue was purified by column chromatography (silica gel Fluka 60, chloroform) yielding 4,4'-dithiobis[(2-bromophenoxy)acetic acid diethyl ester].
Yield: 19.0 g (75 %).
¹H NMR spectrum (200 MHz, CDCl₃, δ_{H}): 7.69 (d, J=2.4 Hz, 2 H); 7.35 (dd, J=2.4 and 8.8 Hz, 2 H); 6.69 (d, J=8.8 Hz, 2 H); 4.67 (s, 4 H); 4.26 (q, J=7.2 Hz, 4 H); 1.29 (t, J=7.2, 6 H).

### General procedure (B)

### Step B:

The above diester (2.32 g, 4.00 mmol) was dissolved in N,N-dimethylacetamide (20 mL), sodium borohydride (0.30 g, 7.90 mmol) was added portionwise at 5°C and the mixture was stirred for 30 min. A solution of 3,3-bis[4-(phenylethinyl)phenyl]allyl bromide (1.80 g, 3.80 mmol; prepared as described in example 1) in 2-butanone (30 mL) and potassium carbonate (1.40 g, 10.0 mmol) were added and the resulting mixture was stirred overnight at ambient temperature and then refluxed for 7 h. Water (200 mL) and benzene (150 mL) were added; the organic layer was dried with anhydrous potassium carbonate, filtered and evaporated *in vacuo.* The residue was purified by column chromatography (silica gel Fluka 60, benzene) giving ethyl 4-[3,3-bis[4-(phenylethinyl)phenyl]allylsulfanyl]-2-bromophenoxy]acetate.
Yield: 1.45 g (56 %).
R_{F} (SiO₂, benzene) 0.40.
¹H NMR spectrum (200 MHz, CDCl₃, δ_{H}): 7.48-7.59 (m, 6 H); 7.43 (d, J=8.5 Hz, 2 H); 7.30-7.39 (m, 7 H); 7.18 (dd, J=2.2 and 8.5 Hz, 1 H); 7.12 (d, J=8.5 Hz, 2 H); 6.94 (d, J=8.5 Hz, 2 H); 6.66 (d, J=8.5 Hz, 1 H); 6.16 (t, J=7.9 Hz, 1 H); 4.68 (s, 2 H); 4.25 (q, J=7.2 Hz, 2 H); 3.52 (d, J=7.9 Hz, 2 H);1.25 (t, J=7.2 Hz, 3 H).

### General procedure (D)

### Step A:

The above ester (1.45 g, 2.10 mmol) was dissolved in a mixture of ethanol (30 mL) and tetrahydrofuran (30 mL); 20 % Solution of sodium hydroxide (2 mL, 10.0 mmol) was added and the resulting mixture was left to stand for 48 h. The solvents were evaporated in *vacuo,* the residue was dissolved in water (50 mL) and acidified with hydrochloric acid. The product was extracted with chloroform (3 x 25 mL). The combined organic layer was dried with anhydrous potassium carbonate, filtered and evaporated *in vacuo.* The residue was purified by column chromatography (silica gel Fluka 60, chloroform) yielding the title compound.
Yield: 0.87 g (62 %).
R_{F} (SiO₂, chloroform/ethanol 10:1): 0.55.
¹H NMR spectrum (200 MHz, CDCl₃, δ_{H}): 7.48-7.59 (m, 6 H); 7.43 (d, J=8.5 Hz, 2 H); 7.39-7.30 (m, 7 H); 7.19 (dd, J=2.2 and 8.5 Hz, 1 H); 7.13 (d, J=8.5 Hz, 2 H); 6.95 (d, J=8.5 Hz, 2 H); 6.67 (d, J=8.5 Hz, 1H); 6.15 (t, J=7.9 Hz, 1 H); 4.71 (s, 2 H); 3.53 (d, J=7.9 Hz, 2 H).

A mixture of the title acid (0.87 g, 1.33 mmol) and (L)-lysine (0.194 g, 1.33 mmol) was dissolved in methanol (35 mL), the solvent was evaporated *in vacuo* and the residue was triturated with ether (2x10 mL) yielding L-lysinate of the title compound as trihydrate.
Yield: 0.85 g (80 %).
M.p. 164-169 °C.

For C₄₅H₄₁BrN₂O₅S, 3 H₂O:
Calculated: C, 63.15%; H, 5.53%; Br, 9.34%; N, 3.27%; S, 3.75%;
Found: C, 62.79%; H, 5.06%; Br, 9.99%; N, 4.07%; S, 3.51%.

### Example 3

### [4-[3,3-Bis[4-[(thiofen-2-yl)ethylnyl]phenyl]allylsulfanyl]-2-methylphenoxy]acetic acid

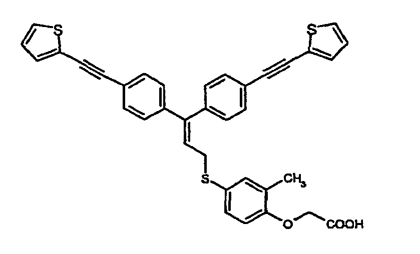

A small part of a solution of vinyl bromide (9.0 g, 84 mmol) in dry tetrahydrofuran (70 mL) was added to magnesium turnings (2.0 g, 82 mmol) and the reaction was initiated with some drops of 1,2-dibromoethane. The rest of the solution of vinyl bromide was added dropwise maintaining the reacting mixture warm and the mixture was stirred for 30 min. A solution of 4,4'-diiodobenzophenone (17.0 g, 39 mmol) in dry tetrahydrofuran (100 mL) was added dropwise under reflux and when the addition was complete, the reaction mixture was stirred for next 30 min. The mixture was cooled, diluted with benzene (100 mL) and then a saturated solution of ammonium chloride (15 mL) was added dropwise. After 30 min, the organic phase was separated, dried (K₂CO₃) and evaporated *in vacuo.* The residue was dissolved in benzene and filtered through a column with SiO₂ (200 g) yielding 14.4 g (79 %) of 3,3-bis(4-iodophenyl) allylalcohol.
M.p. 83-85 °C (light petroleum).
R_{F} (SiO₂, CHCl₃) 0.31.
¹H NMR spectrum (200 MHz, CDCl₃): 7.65 (m, 4 H); 6.92 (m, 4 H); 6.22 (t, J=7.0 Hz, 1 H); 4.18 (d, J=7.0 Hz, 2 H);1.61 (s, 1H).

### General procedure (F)

### Step A:

A solution of trimethylsilyl bromide (5.2 g, 34 mmol) in dichloromethane (50 mL) was added dropwise to a solution of the above alcohol (14.4 g, 31 mmol) in dichloromethane (100 mL) at 0 °C under stirring. The reaction mixture was left to stand overnight, then was washed with 5% solution of sodium hydrogen carbonate (200 mL), dried (MgSO₄) and evaporated *in vacuo* yielding 15.9 g (97 %) of 3,3-bis(4-iodophenyl)allylbromide.
M.p. 105-109 °C (light petroleum).

R_{F} (SiO₂, benzene) 0.72.
¹H NMR spectrum (200 MHz, CDCl₃): 7.76 (m, 2 H); 7.62 (m, 2 H); 6.93 (m, 4 H); 6.32 (t, J=8.6 Hz, 1 H); 3.98 (d, J=8.6 Hz, 2 H).

### Step B:

A solution of ethyl (2-methyl-4-mercaptophenoxy)acetate (3.4 g, 15.0 mmol) in 2-butanone (25 ml) was added to a mixture of the above bromide (7.2 g, 14.0 mmol) and potassium carbonate (2.5 g, 18.0 mmol) in 2-butanone (25 ml). The resulting mixture was refluxed for 10 h, filtered and evaporated *in vacuo.* The residue was purified by chromatography (silica gel Fluka 60, 170 g, benzene) yielding 9.0 g (98 %) of ethyl 4-(3,3-bis(4-iodophenyl)allylsulfanyl)-2-methylphenoxyacetate.
R_{F} (SiO₂, benzene) 0.45.
¹H NMR spectrum (200 MHz, CDCl₃, δ_{H}): 7.59 (m, 4 H); 7.09 (m, 2 H); 6.85 (m, 2 H); 6.59 (m, 2 H); 6.11 (t, J=8.0 Hz, 1H); 4.62 (s, 2 H); 4.23 (q, J=7.2 Hz, 2H); 3.44 (d, J=8.0 Hz); 2.20 (s, 3 H); 1.27 (t, J=7.2 Hz, 3 H).

### General procedure (E)

### Step B:

A 1 M solution of tetrabutylammonium fluoride in tetrahydrofuran (4.70 mL, 4.7 mmol) and tetrakis(triphenylphosphine)palladium (412 mg, 0.357 mmol) were added to a degassed solution of ethyl [4-[3,3-bis(4-iodophenyl)allylsulfanyl]-2-methylphenoxy]acetate (1010 mg, 1.51 mmol), 2-(trimethylsilylethynyl)thiophene (850 mg, 4.71 mmol; prepared as described in J. Org. Chem. 1996, 61, 6909) and ethanol (0.275 mL, 4.71 mmol) in dry tetrahydrofuran (15 mL). In atmosphere of nitrogen, the resulting mixture was heated to 60 °C for 4.5 h and subsequently cooled down. The solution was diluted with dichloromethane (75 mL) and washed with water (2 x 20 mL) and brine (2 x 20 mL). The organic solution was dried with anhydrous magnesium sulfate and subsequently evaporated *in vacuo.* The residue was purified by flash column chromatography (silica gel Fluka 60, hexane/ethyl acetate 15:1 + 0.1 % of triethylamine) yielding ethyl [4-[3,3-bis[4-[(thiofen-2-yl)ethylnyl]phenyl]allylsulfanyl]-2-methylphenoxy]acetate as an yellow oil.
Yield: 260 mg (27 %).
M.p. --- ° C (oil).
R_{F} (SiO₂, hexane/ethyl acetate 90:10) 0.15.
¹H NMR spectrum (250 MHz, CDCl₃, δ_{H}): 7.45 (dm, J=8.4 Hz, 2 H); 7.40 (dm, J=8.5 Hz, 2 H); 7.29 (m, 4 H); 7.12 (m, 4 H); 7.01 (m, 2 H); 6.90 (dm, J=8.3 Hz, 2 H); 6.56 (dm, J=9.1 Hz, 1 H); 6.18 (t, J=8.0 Hz, 1 H); 4.62 (s, 2 H); 4.23 (q, J=7.2 Hz, 2 H); 3.51 (d, J=7.9 Hz, 2 H); 2.22 (s, 3 H); 1.26 (t, J=7.2 Hz, 3 H).

### General procedure (D)

In atmosphere of nitrogen, lithium hydroxide monohydrate (28 mg, 0.667 mmol) was added to an ice-water cooled solution of the above ester (328 mg, 0.520 mmol) in a mixture tetrahydrofuran/methanol/distilled water (5:1:1; 7 mL) and the resulting solution was stirred for 60 min under cooling. Saturated solution of ammonium chloride (10 mL) was added, the mixture was acidified by a few drops of 1 M hydrochloric acid and the resulting mixture was extracted with ether (3 x 20 mL). The organic solution was washed with water (2x10 mL) and brine (2x10 mL), dried with anhydrous magnesium sulfate and evaporated *in vacuo.* The residue was purified by column chromatography (silica gel Fluka 60, chloroform/methanol 95:5) yielding the title acid.
Yield: 222 mg (71 %).
M.p. --- (oil).
R_{F} (SiO₂, chloroform/methanol 85:15) 0.20.
¹H NMR spectrum (250 MHz, CDCl₃, δ_{H}): 7.44 (dm, J=8.4 Hz, 2 H); 7.37 (dm, J=8.5 Hz, 2 H); 7.29-7.24 (m, 4 H); 7.11 (m, 4 H); 7.02-6.97 (m, 2 H); 6.91 (d, J=8.2 Hz, 2 H); 6.56 (d, J=9.1 Hz, 1H); 6.16 (t, J=8.1 Hz, 1 H); 4.57 (s, 2 H); 3.49 (d, J=7.9 Hz, 2 H); 2.17 (s, 3 H).

A solution of L-lysine (34 mg, 0.233 mmol) in distilled water (0.5 mL) was added to a solution of the above acid (145 mg, 0.241 mmol) in dry tetrahydrofuran (5 mL). The resulting solution was stirred for 40 min, evaporated *in vacuo* and the residue was evaporated with absolute ethanol (2x10 mL). The residue was triturated with anhydrous ether (3x10 mL) yielding L-lysinate of the title acid.
Yield: 159 mg (88 %).
M.p. 136 -144 °C (amorphous).
¹H NMR spectrum (250 MHz, DMSO-d₆, δ_{H}): 7.30-7.70 (m, ~ 8 H); 6.85-7.25 (m, 8 H); 6.65 (bd, 1 H); 6.23 (bd, 1 H); 4.25 (s, ~ 2 H); 3.45 (bd, 2 H); 3.24 (bs, 1 H); 2.70 (bm, ~ 2 H); 2.08 (s, 3 H); 1.80-1.15 (m, ~ 6 H).

### Example 4

### {4-[3,3-Bis-(4-thiophen-3-ylethynylphenyl)allylsulfanyl]-2-methylphenoxy}acetic acid

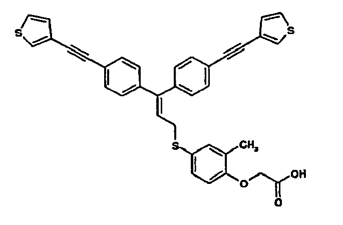

### General procedure (E)

### Step B:

A 1 M solution of tetrabutylammonium fluoride in tetrahydrofuran (3.60 mL, 3.6 mmol) and tetrakis(triphenylphosphine)palladium (345 mg, 0.299 mmol) were added to a degassed solution of ethyl [4-[3,3-bis(4-iodophenyl)allylsulfanyl]-2-methylphenoxy]acetate (1 g, 1.49 mmol; prepared as described in example 3), 3-(trimethylsilylethynyl)thiophene (753 mg, 4.18 mmol; prepared as described in J. Org. Chem. 1996, 61, 6909) and ethanol (0.2 mL, 3.43 mmol) in dry tetrahydrofuran (14 mL). The resulting mixture was heated in atmosphere of nitrogen at 60 °C for 4.5 h and 3-(trimethylsilylethynyl)thiophene (377 mg, 2.09 mmol), tetrakis(triphenylphosphine)palladium (173 mg, 0.150 mmol), 1 M solution of tetrabutylammonium fluoride in tetrahydrofuran (1.80 mL, 1.8 mmol) and ethanol (0.1 mL, 1.71 mmol) were added again. The mixture was heated at 60 °C for 1.5 h and subsequently cooled down. The solution was diluted with dichloromethane (100 mL) and washed with water (2 x 40 mL) and brine (2 x 40 mL). The organic solution was dried with anhydrous magnesium sulfate and subsequently evaporated *in vacuo.* The residue was purified by flash column chromatography (silica gel Fluka 60, hexane/ethyl acetate 15:1) yielding ethyl [4-[3,3-bis[4-[(thiofen-3-yl)ethylnyl]phenyl]allylsulfanyl]-2-methylphenoxy]acetate as an yellow oil.
Yield: 298 mg (32 %).
R_{F} (SiO₂, hexane/ethyl acetate 90:10) 0.20.
¹H NMR spectrum (250 MHz, CDCl₃, δ_{H}): 7.47-6.88 (m, ~17 H); 6.18 (t, 1 H); 4.62 (s, ~2 H); 4.23 (q, J=7.1 Hz, 2 H); 3.51 (d, J=8.0 Hz, 2 H); 2.22 (s, 3 H);1.26 (t, J=7.1 Hz, 3 H).

### General procedure (D)

### Step A:

In atmosphere of nitrogen, lithium hydroxide monohydrate (25 mg, 0.596 mmol) was added to an ice-water cooled solution of the above ester (248 mg, 0.393 mmol) in a mixture tetrahydrofuran/methanol/distilled water (5:1:1; 28 mL) and the resulting solution was stirred under cooling for 90 min. A diluted aqueous solution of tartaric acid (4 mL) was added, the mixture was diluted with water (50 mL) and extracted with diethyl ether (2 x 100 mL). The combined ethereal layers were washed with water (30 mL) and brine (50 mL), dried with anhydrous magnesium sulfate and evaporated. The residue was purified by column chromatography (silica gel Fluka 60, chloroform/methanol 98:2; 95:5) yielding the title acid.
Yield: 130 mg (55 %).
R_{F} (SiO₂, dichloromethane/methanol 9:1) 0.20.
¹H NMR spectrum (250 MHz, DMSO, δ_{H}): 7.91-6.66 (m, ~17 H); 6.23 (t, J=7.9 Hz, 1 H); 4.39 (s, 2 H); 3.47 (d, ~2 H); 2.09 (s, 3 H).

A solution of L-lysine (28 mg, 0.192 mmol) in distilled water (0.5 mL) was added to a solution of the above acid (121 mg, 0.201 mmol) in dry tetrahydrofuran (5 mL). The resulting solution was stirred for 90 min, evaporated *in vacuo* and the residue was evaporated with absolute ethanol (2x10 mL). The residue was triturated with anhydrous ether (2x15 mL) yielding L-lysinate of the title acid.
Yield: 147 mg (98 %).
M.p. 150-157°C (amorphous).
¹H NMR spectrum (200 MHz, DMSO-d₆, δ_{H}): 7.78-6.44 (m, ~17 H); 6.09 (t, J=8.0 Hz, 1 H); 4.06 (s, ~2 H); 3.29 (d, ~2 H (overlapped)); 2.98 (m, ~1H); 2.58-2.53 (m, 2 H): 1.92 (s, 3 H); 1.66-1.09 (m, ~6 H).

### Example 5

### [4-[3,3-Bis[4-[(pyridine-2-yl)ethylnyl]phenyl]allylsulfanyl]-2-methylphenoxy]acetic acid

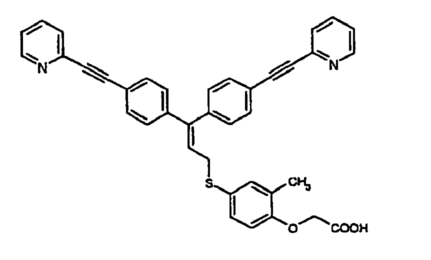

### General procedure (E)

### Step B:

Copper(I) iodide (77 mg, 0.404 mmol), isobutylamine (0.90 mL, 9.06 mmol) and tetrakis(triphenylphosphine)palladium (115 mg, 0.010 mmol) were added to a degassed solution of ethyl [4-[3,3-bis(4-iodophenyl)allylsulfanyl]-2-methylphenoxy]acetate (646 mg, 0.963 mmol; prepared as described in example 3) and 2-ethynylpyridine (0.40 ml, 3.96 mmol) in dry ether (35 mL). In atmosphere of nitrogen, the resulting mixture was stirred at ambient temperature for 2 h and subsequently evaporated *in vacuo.* The residue was purified by flash column chromatography (silica gel Fluka 60, hexane/ethyl acetate 2:1) yielding ethyl [4-[3,3-bis[4-[(pyridine-2-yl)ethylnyl]phenyl]allylsulfanyl]-2-methylphenoxy]acetate as an yellow oil. Yield: 366 mg (61 %).
M.p. -- (oil).
R_{F} (SiO₂, hexane/ethyl acetate 2:1) 0.10.
¹H NMR spectrum (200 MHz, CDCl₃, δ_{H}): 8.63 (m, 2 H); 7.69 (m, 2 H); 7.53 (m, 6 H); 7.24 (m, 2 H); 7.14 (m, 4 H); 6.90 (d, J=8.1 Hz, 2 H); 6.57 (dm, J=9.0 Hz, 1 H); 6.20 (t, J=8.0 Hz, 1 H); 4.63 (s, 2 H); 4.23 (q, J=7.1 Hz, 2 H); 3.50 (d, J=8.0 Hz, 2 H); 2.22 (s, 3 H); 1.26 (t, J=7.1 Hz, 3 H).

### General procedure (D)

### Step A:

In atmosphere of nitrogen, lithium hydroxide monohydrate (30 mg, 0.715 mmol) was added to an ice-water cooled solution of the above ester (346 mg, 0.557 mmol) in a mixture tetrahydrofuran/methanol/distilled water (5:1:1; 7 mL) and the resulting solution was stirred for 60 min under cooling. The reaction mixture was concentrated to half volume, diluted with water (15 mL), neutralized with acetic acid (40 mg, 0.666 mmol) and extracted with ether (3 x 15 mL) and chloroform (3x10 mL). The combined organic layers were washed with water (2 x 10 mL) and brine (2x10 mL), dried with anhydrous magnesium sulfate and evaporated *in vacuo.* The residue was purified by column chromatography (silica gel Fluka 60, dichloromethane/methanol 96:4 - 92:8) yielding the title acid.
Yield: 174 mg (53 %).
M.p. --- (oil).
R_{F} (SiO₂, dichloromethane/methanol 90:10) 0.20.
¹H NMR spectrum (200 MHz, CDCl₃, δ_{H}): 8.63 (m, ~2 H); 7.79-7.14 (m, ~14 H); 6.76 (m, 3 H); 6.18 (t, J=8.2 Hz, 1 H); 4.71 (bs, 2 H); 3.72 (m, 2 H); 2.27 (s, 3 H).

A solution of L-lysine (18.5 mg, 0.127 mmol) in distilled water (0.5 mL) was added to a solution of the above acid (79 mg, 0.133 mmol) in dry tetrahydrofuran (5 mL). The resulting solution was stirred for 90 min, evaporated *in vacuo* and the residue was evaporated with absolute ethanol (2x10 mL). The residue was triturated with anhydrous ether (3x10 mL) yielding L-lysinate of the title acid.
Yield: 52 mg (53 %).
M.p. 144-152°C (amorphous).
¹H NMR spectrum (200 MHz, DMSO-d₆, δ_{H}): 8.61 (bs, 2 H); 7.94-6.75 (m, ~17 H); 6.14 (bt, 1 H); 4.24 (bs, 2 H); 3.62 (bm, ~2 H); 3.13 (bm, ~1H); 2.67 (m, 2 H); 2.15 (bs, 3 H); 1.75-1.19 (m, 6 H).

### Example 6

### {4-[3,3-Bis-(4-pyridin-2-ylethynylphenyl)allyloxy]-2-methylphenoxy}acetic acid

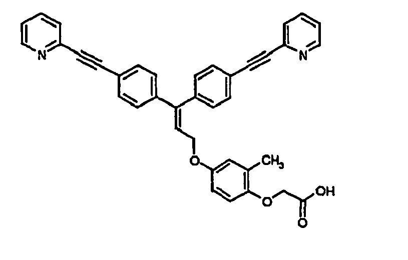

Potassium carbonate (34.4 g, 0.250 mol) and solution of methyl bromoacetate (16.1 ml, 0.175 mol) in butanone (20 ml) were added to a solution of 4-hydroxy-3-methylacetophenone (25 g, 0.166 mol) in butanone (180 ml) and the mixture was refluxed for 1h. After cooling to ambient temperature a white precipitated was filtered off and the filtrate evaporated *in vacuo.* The resulting solid was recrystallized by dissolving it in a mixture of hexanes/diethyl ether/ dichloromethane (120 : 120 : 50 ml) and concentrating *in vacuo. (4-*Acetyl-2-methyl-phenoxy)acetic acid methyl ester was filtered and washed with hexanes (50 mL).
Yield: 35.0 g (95 %).
R_{F} (SiO₂, hexanes/ethyl acetate 1:1) 0.75.
¹H NMR spectrum (200 MHz, CDCl₃, δ_{H}): 7.80-7.76 (m, 2 H); 6.70 (d, J=9.0 Hz, 1H); 4.74 (s, 2 H); 3.82 (s, 3 H); 2.55 (s, 3 H); 2.33 (s, 3 H).

To a solution of the above ester (33.0 g, 0.148 mol) and p-toluenesulfonic acid monohydrate (0.281 g, 0.00148 mol) in dichloromethane (50 ml) a solution of 3-chloroperoxybenzoic acid (53.1 g, 0.237 mol; 77 % in water) in dichloromethane was added (300 ml, dried over magnesium sulfate prior to addition). The mixture was stirred at ambient temperature for 20 h, a solution of sodium sulfite (1 M, 150 ml) was added and the two-phase mixture stirred for 20 min. Then a solution of sodium carbonate (2 M, 150 ml) was added and heterogeneous mixture was vigorously stirred for next 10 min. The organic layer was separated and the aqueous layer was extracted with dichloromethane (50 ml). The combined organic layers were washed with 10 % solution of sodium carbonate (2 x 200 ml) and brine (300 ml). The organic solution was dried with anhydrous magnesium sulfate and its evaporation yielded (4-acetoxy-2-methyl-phenoxy)acetic acid methyl ester as yellowish solid.
Yield: 32.9 g (93 %).
R_{F} (SiO₂, hexanes/ethyl acetate 1:1) 0.80.
¹H NMR spectrum (200 MHz, CDCl₃, δ_{H}): 6.90 (m, 2 H); 6.68 (d, J=8.6, 1H); 4.64 (s, 2 H); 3.80 (s, 3 H); 2.28 (s, 6 H).

A mixture of the above ester (32.9 g, 0.138 mol) and sodium methoxide (0.746 g, 0.0138 mol) in anhydrous methanol (250 ml) was stirred for 24 h. The mixture was evaporated to dryness and a solid residue was dissolved in ethyl acetate (200 ml). The turbid mixture was filtered and the filtrate was washed with saturated aqueous solution of sodium hydrogen carbonate (2 x 150 ml) and brine (200 ml). The organic solution was dried over anhydrous magnesium sulfate and evaporated *in vacuo.* The crude product was recrystallized from ethyl acetate/hexanes yielding (4-Hydroxy-2-methylphenoxy)acetic acid methyl ester as off-white crystals.
Yield: 24.0 g (89 %).
R_{F} (SiO₂, dichloromethane/methanol 99:1) 0.30.
¹H NMR spectrum (200 MHz, CDCl₃, δ_{H}): 6.66-6.58 (m, 3 H); 4.76 (s, 1 H); 4.59 (s, 2 H); 3.80 (s, 3 H); 2.25 (s, 3 H), 2.19 (s, 3 H).

A solution of triethyl phosphonoacetate (17.0 g, 78 mmol) in benzene (30 mL) was added dropwise to the mixture of sodium hydride (60 % suspension in oil; 3.20 g, 80 mmol) in benzene (30 mL) under stirring at ambient temperature. After 30 min, a solution of 4,4'-diiodobenzophenone (17.0 g, 39 mmol) in a mixture of benzene and N,N-dimethylformamide (2:1, 200 mL) was added. The resulting mixture was stirred for 20 h, quenched with 5% aqueous hydrochloric acid (100 mL) and washed with water (100 mL). The combined organic solutions were dried with anhydrous magnesium sulfate and evaporated *in vacuo* to give ethyl 3,3-bis[4-iodophenyl]acrylate.
Yield: 17.40 g (88 %).

M.p. 95-100 °C.
R_{F} (SiO₂, benzene) 0.46.
¹H NMR spectrum (200 MHz, CDCl₃, δ_{H}): 7.69 (q, 4 H); 6.97 (q, 4 H); 6.34 (s, 1 H); 4.07 (q, J=7.15, 2 H); 1.16 (t, J=7.15 Hz, 3 H).

In atmosphere of nitrogen, diisobutylaluminium hydride (16% solution in tetrahydrofuran (100 mL, 90 mmol) was added dropwise to a solution of the above ester (16.4 g, 32 mmol) in tetrahydrofuran (150 mL) at -10 °C. The reaction mixture was stirred for 48 h at ambient temperature and then quenched with water (100 mL) and concentrated hydrochloric acid (30 mL). The mixture was extracted with ethyl acetate (3 x 100 mL), combined organic layers were dried with anhydrous potassium carbonate and evaporated *in vacuo.* The residue was submitted to column chromatography (silica gel Fluka 60, chloroform). Fist fractions contained starting ester (3.2 g), next fractions afforded 3-bis(4-iodophenyl)prop-2-en-1-ol as white solid.
Yield: 10.4 g (86% calculated on converted starting compound). M.p. 140 °C.
R_{F} (SiO₂, chloroform) 0.31.
¹H NMR spectrum (200 MHz, CDCl₃, δ_{H}): 7.65 (q, 4 H); 6.92 (q, 4 H); 6.22 (t, J=6.7 Hz, 1 H); 4.18 (d, J=6.7 Hz, 2 H); 1.61 (s, 1H).

### General procedure (E)

### Step A:

To a solution of 3,3-bis(4-iodophenyl)prop-2-en-1-ol (3.8 g, 8.2 mmol) and ethyl (4-hydroxy-2-methylphenoxy)acetate (1.8 g, 9.0 mmol) in benzene (150 ml) tri-n-butylphosphine (2.43 g, 12.0 mol) was added under nitrogen. The reaction mixture was cooled to 0 °C, and diisopropylazodicarboxylate (3.08 g, 15.0 mmol) was carefully added. Stirring was continued at 0 °C for 2 h and then at room temperature for 16 h. The reaction mixture was evaporated *in vacuo* and the residue was purified on column chromatography using the mixture of hexanes/benzene 4.1 as eluent. This afforded 2.4 g (46 %) of (4-[3,3-bis-(4-iodophenyl)allyloxy]-2-methylphenoxy)acetic acid methyl ester as an oil.
R_{F} (SiO₂, hexane/ethyl acetate 5:2) 0.65.
¹H NMR spectrum (200 MHz, CDCl₃, δ_{H}): 6.93-7.80 (m, 8 H); 6.65 (m, 3 H); 6.33 (t, J=8 Hz, 1 H); 4.61 (s, 2 H); 4.49 (d, J= 8 Hz, 2 H); 3.81 (s, 3 H); 2.28 (s, 3 H).

### Step B:

Copper(I) iodide (70 mg, 0.50 mmol), isobutylamine (1.0 mL, 10 mmol) and tetrakis(triphenylphosphine)palladium (138 mg, 0.012 mmol) were added to a degassed solution of above methyl ester (672 mg, 1.0 mmol) and 2-ethynylpyridine (0.40 ml, 4.0 mmol) in dry ether (45 mL). The resulting mixture was stirred at ambient temperature for 3 h in atmosphere of argon and subsequently evaporated *in vacuo.* The residue was purified by flash column chromatography (silica gel Fluka 60, hexane/ethyl acetate 2:1 -> ethyl acetate) yielding {4-[3,3-bis-(4-pyridin-2-ylethynylphenyl)allylsulfanyl]-2-methylphenoxy}acetic acid methyl ester as an oil.
Yield: 490 mg (79 %).
R_{F} (SiO₂, hexane/ethyl acetate 2:1) 0.20.
¹H NMR spectrum (200 MHz, CDCl₃, δ_{H}): 8.63 (m, 2 H); 7.52-7.74 (m, 8 H); 7.24 (m, 6 H); 6.95 (m, 3 H); 6.38 (t, J=8.0 Hz, 1 H); 4.59 (s, 2 H); 4.54 (d, J=8.0 Hz, 2 H); 3.79 (s, 3 H); 2.26 (s, 3 H).

### General procedure (D)

In atmosphere of argon, lithium hydroxide monohydrate (42 mg, 1.0 mmol) was added to an ice-water cooled solution of the above ester (460 mg, 0.739 mmol) in a mixture tetrahydrofuran/methanol/distilled water (5:1:1; 10 mL) and the resulting solution was stirred for 90 min under cooling. The reaction mixture was neutralized with 10% solution of ammonium chloride and extracted with chloroform (3 x 25 mL). The combined organic layers were washed with brine (2 x 10 mL), dried with anhydrous magnesium sulfate and evaporated in *vacuo.* The residue was purified by column chromatography (silica gel Fluka 60, chloroform/methanol 9:1) yielding the title acid.
Yield: 174 mg (78 %).
M.p. --- (oil).
R_{F} (SiO₂, hexane/ethyl acetate 2:1) 0.15.
¹H NMR spectrum (250 MHz, CD₃COOD, δ_{H}): 8.60 (m, 2 H); 7.35 (m, 12 H); 7.27 (m, 2 H); 6.48-6.65 (m, 3 H); 6.43 (t, J=8.0 Hz, 1 H); 4.45 (d, 2 H); 4.21 (s, 2 H); 2.11 (s, 3 H).

A solution of L-lysine (72 mg, 0.493 mmol) in distilled water (1.0 mL) was added to a solution of the above acid (300 mg, 0.493 mmol) in tetrahydrofuran (20 mL). The resulting solution was stirred for 90 min, filtered with charcoal and evaporated *in vacuo.* The residue was triturated with anhydrous ether (3 x 10 mL) yielding L-lysinate of the title acid.
Yield: 149 mg (40 %).
M.p. 185-193 °C (amorphous).
¹H NMR spectrum (200 MHz, DMSO-d₆, δ_{H}): 8.60 (m, 2 H); 7.79 (m, 2 H); 7.55-7.70 (m, 6 H); 7.41 (m, 2 H); 7.30 (m, 4 H); 6.52-6.70 (m, 3 H); 6.43 (t, J=7.8 Hz, 1 H); 4.45 (d, 2 H); 4.21 (s, 2 H); 3.45 (bm, -2 H); 2.11 (s, 3 H).

### Example 7

### {4-[3,3-Bis-(4-furan-2-ylethynylphenyl)allylsulfanyl]-2-methylphenoxy}acetic acid

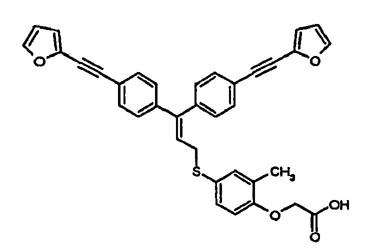

### General procedure (E)

### Step C:

Ethynyltrimethylsilane (1.33 mL, 9.60 mmol) was added to a degassed solution of {4-[3,3-bis-(4-iodophenyl)allylsulfanyl]-2-methylphenoxy}acetic acid ethyl ester (2.20 g, 3.28 mmol; prepared as described in example 3), tetrakis(triphenylphosphine)palladium (114 mg, 0.099 mmol) and copper(I) iodide (44 mg, 0.231 mmol) in triethylamine (71 mL). The resulting mixture was stirred in atmosphere of nitrogen at 80 °C for 30 min and subsequently evaporated *in vacuo.* The residue was triturated with ether (100 mL), the obtained suspension was filtered off and the filtrate was evaporated *in vacuo.* The obtained residue was purified by column chromatography (silica gel Fluka 60, hexane/ethyl acetate 20:1) yielding {4-[3,3-bis-(4-trimethylsilanylethynylphenyl)allylsulfanyl]-2-methylphenoxy}acetic acid ethyl ester.
Yield: 1.42 g (71 %).
R_{F} (SiO₂, hexane/ethyl acetate 20:1) 0.25.
¹H NMR spectrum (250 MHz, CDCl₃, δ_{H}): 7.41-6.54 (m, ∼11 H); 6.14 (t, 1 H); 4.60 (s, 2 H); 4.23 (q, J=7.1 Hz, 2 H); 3.46 (d, J=8.0 Hz, 2 H); 2.20 (s, 3 H);1.26 (t, J=7.1 Hz, ∼3 H); 0.25 (m, ∼18 H).

The above ester (1.11 g, 1.82 mmol) was dissolved in absolute methanol (42 mL) and dry dichloromethane (42 mL), the resulting solution was degassed and cooled with ice-water. In atmosphere of nitrogen, anhydrous potassium carbonate (43 mg, 0.311 mmol) was added and the resulting suspension was stirred for 19 h under cooling. The mixture was diluted with dichloromethane (200 mL) and with brine (250 mL) and layers were separated. The aqueous layer was extracted with dichloromethane (100 mL), the combined organic layers were washed with water (3 x 150 mL), brine (150 mL), dried with anhydrous magnesium sulfate and subsequently *evaporated in vacuo.* The obtained residue was purified by column chromatography (silica gel Fluka 60, hexane/ethyl acetate 20:1) yielding {4-[3,3-bis-(4-ethynylphenyl)allylsulfanyl]-2-methylphenoxy}acetic acid methyl ester as an yellow oil.
Yield: 595 mg (72 %).
R_{F} (SiO₂, hexane/ethyl acetate 9:1) 0.20.
¹H NMR spectrum (200 MHz, CDCl₃, δ_{H}): 7.44-6.53 (m, ∼11 H); 6.16 (t, J=8.0 Hz, 1 H); 4.64 (s, 2 H); 3.77 (s, 3 H); 3.47 (d, J=8.0 Hz, 2 H); 3.11 (s, 1H); 3.09 (s, 1 H); 2.20 (s, 3 H).

### Step D:

Copper(I) iodide (7 mg, 36 mmol) and dichlorobis(triphenylphosphine)palladium (II) (7 mg, 10 mmol) and were added to a degassed mixture of {4-[3,3-bis-(4-ethynylphenyl)allylsulfanyl]-2-methylphenoxy}acetic acid methyl ester (340 mg, 0.7512 mmol), 2-bromofurane (232 mg, 1.58 mmol) and triphenylphosphine (10 mg, 38.1 mmol) in triethylamine (4 mL). In atmosphere of nitrogen, the resulting mixture was heated at 80 °C for 2 h and subsequently cooled down. The solution was diluted with dichloromethane (35 mL), filtered, washed with water (2 x 15 mL) and brine (15 mL). The organic solution was dried with anhydrous magnesium sulfate and subsequently evaporated *in vacuo.* The residue was purified by flash column chromatography (silica gel Fluka 60, hexane/ethyl acetate 9:1) yielding methyl {4-[3,3-bis-(4-furan-2-ylethynylphenyl)allylsulfanyl]-2-methylphenoxy}acetic acid as an oil.
Yield: 360 mg (82 %).
R_{F} (SiO₂, hexane/ethyl acetate 9:1) 0.20.
¹H NMR spectrum (200 MHz, CDCl₃, δ_{H}): 7.42 (m, 6 H); 712 (m, 4 H); 6.87 (m, 2 H); 6.68 (m, 2 H); 6.53 (m, 1 H); 6.44 (m, 2 H) 6.19 (t, 1 H); 4.64 (s, 2 H); 3.76 (s, 3 H); 3.49 (d, 2 H); 2.21 (s, 3 H).

### General procedure (D)

In atmosphere of nitrogen, a solution of lithium hydroxide monohydrate (43 mg, 0.847 mmol) in distilled water (1 mL) was added to an ice-water cooled solution of the above ester (330 mg, 0.564 mmol) in tetrahydrofuran/methanol mixture (1:1; 15 mL) and the resulting solution was stirred for 90 min under cooling. The reaction mixture was diluted with water (15 mL), neutralized with 10% solution of tartaric acid (0.049 mL, 0.857 mmol) and extracted with chloroform (3 x 20 mL). The mixture was washed with water (10 mL), dried with anhydrous magnesium sulfate and evaporated *in vacuo* yielding sufficiently pure crude title acid.
Yield: 295 mg (92 %).

M.p. --- (oil).
R_{F} (SiO₂, chloroform/methanol 9:1) 0.20.

A solution of L-lysine (69 mg, 0.473 mmol) in distilled water (1.0 mL) was added to a solution of the above acid (290 mg, 0.473 mmol) in dry tetrahydrofuran (25 mL). The resulting solution was stirred for 30 min, filtered (with active charcoal Norite) and evaporated *in vacuo.* The residue was triturated with anhydrous ether (3 x 30 mL) yielding L-lysinate of the title acid.
Yield: 126 mg (37 %).
M.p. 145-155 °C (amorphous).
¹H NMR spectrum (300 MHz, CD₃COOD, δ_{H}): 7.42-7.53 (m, 6 H); 7.11-7.19 (m, 4 H); 6.87 (m, 2 H); 6.70 (m, 3 H); 6.48 (m, 2 H); 6.25 (t, J=7.9 Hz, 1 H); 4.75 (s, 2 H); 4.07 (bs, 1 H); 3.50 (d, J=8.0 Hz, 2 H); 3.09 (bs, 2 H); 2.13 (s, 3 H).

### Example 8

### (4-{3,3-Bis-[4-(1-methyl-1H-pyrrol-2-ylethynyl)phenyl]allylsulfanyl}-2-methylphenoxy)acetic acid

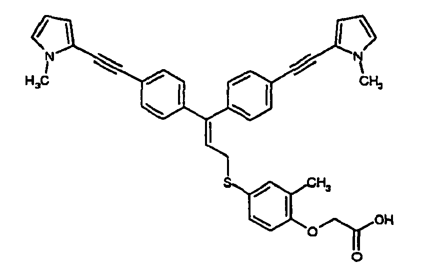

### General procedure (E)

### Step B:

1-Methyl-2-trimethylsilanylethynyl-1H-pyrrole (0.63 g, 3.55 mmol; prepared as described in Synthesis, **1996**, 589) was added to a degassed solution of {4-[3,3-bis-(4-iodophenyl)-allylsulfanyl]-2-methylphenoxy}acetic acid ethyl ester (1.00 g, 1.49 mmol; prepared as described in example 3) in anhydrous tetrahydrofuran (15 mL). Anhydrous ethanol (0.21 mL, 0.35 mmol) and tetrabutylammonium fluoride (1 M solution in THF, 3.6 mL, 3.60 mmol) was added, the solution was stirred at ambient temperature for 15 min and then tetrakis(triphenylphosphine)palladium (0.35 g, 0.30 mmol) was added and the reaction mixture was stirred at 50 °C for 4 h. Next portion of 1-methyl-2-trimethylsilanylethynyl-1H-pyrrole (0.32 g, 1.80 mmol) was added and dark brown mixture was stirred at 50 °C for additional 3 h. The resulting dark brown solution was diluted with dichloromethane (100 mL), organic phase was washed with water (3 x 50 mL) and brine (2 x 25 mL), dried over magnesium sulphate and evaporated *in vacuo.* The residue was purified using column chromatography on silica gel (Fluka 60, hexane/ethyl acetate 95:5) yielding (4-{3,3-bis-[4-(1-methyl-1H-pyrrol-2-ylethynyl)-phenyl]allylsulfanyl}-2-methylphenoxy)acetic acid ethyl ester.
Yield: 0.27 g (29 %).
R_{F} (SiO₂, chloroform/methanol 95:5) 0.40.
¹H NMR spectrum (250 MHz, CDCl₃, δ_{H}): 7.42 (d, J=8.0 Hz, 2 H); 7.36 (d, J=8.2 Hz, 2 H); 712-7.10 (m, 4 H); 6.88 (d, J=8.0 Hz, 2 H); 6.70-6.68 (m, 2 H); 6.58-6.47 (m, 3 H); 6.16 (t, J=7.9 Hz, 1 H); 6,14-6,11 (m, 2 H); 4.61 (s, 2 H); 4.23 (q, J=7.1 Hz, 2 H); 3.76 (s, 3 H); 3.72 (s, 3 H); 3.51 (d, J=7.9 Hz, 2 H); 2.21 (s, 3 H); 1.26 (t, J=7.1 Hz, 3 H).

### General procedure (D)

### Step A:

A solution of lithium hydroxide monohydrate (36 mg, 0.85 mmol) in water (1.5 mL) was added to a solution of the above ethyl ester in ethanol (1.5 mL) and tetrahydrofuran (3 mL) and the mixture was stirred at ambient temperature for 1.5 h. Resulting solution was diluted with water (10 mL), acidified with 2 M hydrochloric acid to pH -2 and extracted with diethyl ether. Organic extracts were washed with water (3 x 15 mL) and brine (2 x 10 mL), dried over magnesium sulphate and evaporated to dryness. A residuum was purified by column chromatography on silica gel (Fluka 60, dichloromethane/methanol 95:5) giving the title acid.
Yield 140 mg (59 %).
R_{F} (SiO₂, chloroform/methanol 90:10) 0.25.
¹H NMR spectrum (250 MHz, CDCl₃+50 µL CD₃COOD, δ_{H}): 7.40 (d, J=8.3 Hz, 2 H); 7.33 (d, J=8.4 Hz, 2 H); 7.12-7.08 (m, 4 H); 6.87 (d, J=8.3 Hz, 2 H); 6.68-6.65 (m, 2 H); 6.54-6.52 (m, 1 H); 6.48-6.43 (m, 2 H); 6.14 (t, J=7.9 Hz, 1 H); 6.11-6.07 (m, 2 H); 4.65 (s, 2 H); 3.73 (s, 3 H); 3.70 (s, 3 H); 3.4+ (d, J=7.9 Hz, 2 H); 2.18 (s, 3 H).

A solution of L-lysinate (30 mg, 0.2 mmol) in water (0.7 mL) was added to a solution of the above acid (120 mg, 0.2 mmol) in tetrahydrofuran (5 mL) and the resulting solution was stirred at ambient temperature for 1.5 h. The solvents were evaporated to dryness, the residue was evaporated twice with anhydrous ethanol (20 mL) and the obtained solid was triturated with anhydrous diethyl ether (3 x 25 mL). This afforded L-lysinate of the title acid.

Yield: 130 mg (88 %).
M.p.: 146-148 °C.
¹H NMR spectrum (250 MHz, DMSO-d₆, δ_{H}): 7.47 (d, J=8.0 Hz, 2 H); 7.41 (d, J=8.2 Hz, 2 H); 7.11 (d, J=8.2 Hz, 2 H); 7.04-7.02 (m, 2 H); 6.91-6.88 (m, 4 H); 6.62 ( d, J=8.9 Hz, 1 H); 6.44-6.41 (m, 2 H); 6.20 (t, J=7.9 Hz, 1 H); 6.05-6.02 (m, 2 H); 4.21 (s, 2 H); 3.69 (s, 3 H); 3.66 (s, 3 H); 3.45 (d, J=7.7 Hz, 2 H); 3.12 (m, ∼ 1 H); 2.71-2.63 (m, ~ 2 H); 2.06 (s, 3 H); 1.66-1.21 (m, - 6 H).

### Example 9

### [4-[3,3-Bis[4-[3-(morpholine-4-yl)propyn-1-yl]phenyl]allylsulfanyl]-2-methylphenoxy]acetic acid

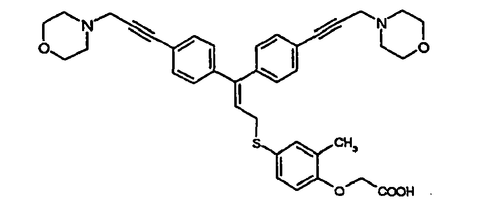

Paraformaldehyde (122 mg, 4.06 mmol), copper(I) iodide (74 mg, 0.389 mmol) and morpholine (0.35 mL, 4.01 mmol) were added to a solution of 3,3-bis(4-ethylnylphenyl)allyl alcohol (478 mg, 1.85 mmol; prepared as described in example 10) in dioxane (20 mL). The resulting mixture was refluxed for 160 min, cooled down and evaporated *in vacuo.* The residue was purified by flash column chromatography (silica gel Fluka 60, chloroform saturated with ammonia/methanol 98.5:1.5) yielding 3,3-bis[4-[3-(morpholine-4-yl)propyn-1-yl]phenyl]allyl alcohol as an oil.
Yield: 696 mg (82 %).
M.p. --- (oil).
R_{F} (SiO₂, chloroform saturated with ammonia/methanol 95:5) 0.40.
¹H NMR spectrum (200 MHz, CDCl₃, δ_{H}): 7.45 (dm, J=8.4 Hz, 2 H); 7.35 (dm, J=8.5 Hz, 2 H); 7.16 (dm, J=8.5 Hz, 2 H); 7.10 (dm, J=8.4 Hz, 2 H); 6.26 (t, J=6.7 Hz, 1 H); 4.22 (d, J=6.7 Hz, 2 H); 3.77 (m, 8 H); 3.53 (s, 2 H); 3.51 (s, 2 H); 2.65 (m, 8 H).

### General procedure (B)

### Step A and B:

An excess of a solution of hydrogen chloride in dry ether was added dropwise to a solution of the above allyl alcohol (406 mg, 0.889 mmol) in dry tetrahydrofuran (10 mL). The formed heterogeneous mixture was stirred for 10 min and subsequently evaporated *in vacuo.* Thionyl chloride (10 mL) was added to the residue; the formed solution was stirred for 90 min and evaporated *in vacuo.* Dry toluene (15 mL) was added to the residue and the mixture was evaporated again. In atmosphere of nitrogen, the residue was dissolved in dry N,N-dimethylformamide (10 mL) and N,N-diisopropylethylamine (1.55 mL, 8.90 mmol) and subsequently a solution of ethyl (4-mercapto-2-methylphenoxy)acetate (339 mg, 1.50 mmol) in dry N,N-dimethylformamide (2 mL) were added. The resulting mixture was stirred overnight and subsequently poured into water (75 mL). The heterogeneous mixture was extracted with ethyl acetate (3 x 30 mL); the organic layers were collected and washed with brine (3 x 20 mL), water (2 x 20 mL) and brine (2 x 20 mL). The organic solution was dried with anhydrous magnesium sulfate and evaporated *in vacuo.* The residue was purified by flash column chromatography (silica gel Fluka 60, chloroform saturated with ammonia/methanol 99:1) yielding ethyl [4-[3,3-bis[4-[3-(morpholine-4-yl)propyn-1-yl]phenyl]allylsulfanyl]-2-methylphenoxy]acetate as an oil.
Yield: 464 mg (79 %).
M.p. --- (oil).
R_{F} (SiO₂, chloroform saturated with ammonia/methanol 95:5) 0.45.
¹H NMR spectrum (200 MHz, CDCl₃, δ_{H}): 7.37 (d, J=8.3 Hz, 2 H); 7.31 (d, J=8.4 Hz, 2 H); 7.08 (m, 4 H); 6.84 (d, J=8.3 Hz, 2 H); 6.56 (d, J=9.2 Hz, 1 H); 6.14 (t, J=7.8 Hz, 1 H); 4.61 (s, 2 H); 4.23 (q, J=7.2 Hz, 2 H); 3.79 (m, 8 H); 3.50 (m, 6 H); 2.65 (m, 8 H); 2.20 (s, 3 H); 1.26 (t, J=7.2 Hz, 3 H).

### General procedure (D)

### Step A:

In atmosphere of nitrogen, lithium hydroxide monohydrate (45 mg, 1.07 mmol) was added to an ice-water cooled solution of the above ester (450 mg, 0.667 mmol) in a mixture tetrahydrofuran/methanol/water (5:1:1; 7 mL) and the resulting solution was stirred for 2 h under cooling. The reaction solution was diluted with water (20 mL) and the whole mixture was washed with toluene (4 x 10 mL). The organic layers were discarded and the aqueous layer was neutralized by addition of a solution of glacial acetic acid (64 mg, 1.07 mmol) in water (3 mL). Solid sodium chloride was added to the turbid solution and the mixture was extracted with ether (4 x 20 mL). The combined organic solution were washed with brine (2 x 10 mL), dried with anhydrous magnesium sulfate and evaporated *in vacuo* yielding the title acid as an oil.
Yield: 294 mg (68 %).
M.p. --- (oil).
R_{F} (SiO₂, chloroform/methanol 85:15) 0.10.
¹H NMR spectrum (200 MHz, CDCl₃, δ_{H}): 11.10 and 10.55 (bs, 1 H); 7.35-6.95 (m, -8 H); 6.73 (d, J=7.9 Hz, 2 H); 6.54 (d, J=8.4 Hz, 1 H); 6.17 (t, J=8.3 Hz, 1 H); 4.58 (s, 2 H); 3.80 (bs, 8 H); 3.61 (s, 2 H); 3.55 (s, 2 H); 3.44 (d, J=8.2 Hz, 2 H); 2.82 (bs, ∼4 H); 2.73 (bs, -4 H); 2.20 (s, 3 H).

A solution of L-lysine (57 mg, 0.390 mmol) in distilled water (1 mL) was added to a solution of the above acid (261 mg, 0.410 mmol) in dry tetrahydrofuran (10 mL). The resulting solution was stirred for 90 min, evaporated *in vacuo* and the residue was evaporated with absolute ethanol (2 x 10 mL). The residue was triturated with anhydrous ether (2 x 10 mL) yielding L-lysinate of the title acid.
Yield: 271 mg (84 %).
M.p. 122-139 °C (amorphous).
¹H NMR spectrum (200 MHz, DMSO-d₆, δ_{H}): 8.07 (bs, -2 H); 7.43-6.83 (m, 10 H); 6.61 (bd, J=8.4 Hz, 1 H); 6.18 (bt, J=7.9 Hz, 1 H); 4.26 (bs, 2 H); 3.59-3.42 (m, 14 H); 3.23 (bs, ∼1H); 2.70 (bs, ∼2 H); -2.50 (bs, ∼8 H); 2.06 (s, 3 H); 1.69-1.30 (m, ~6 H).

### Example 10

### [4-[3,3-Bis[4-[3-(N,N-dimethylamino)propyn-1-yl]phenyl]allylsulfanyl]-2-methylphenoxy]acetic acid

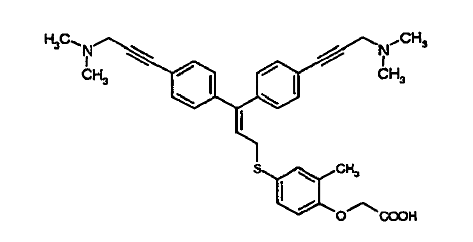

Ethynyltrimethylsilane (1.95 mL, 14.1 mmol) was added to a degassed solution of 3,3-di(4-iodophenyl)allyl alcohol (2.50 g, 5.41 mmol; prepared as described in example 3), tetrakis(triphenylphosphine)palladium (188 mg, 0.163 mmol) and copper(I) iodide (72 mg, 0.378 mmol) in triethylamine (40 mL). In atmosphere of nitrogen, the resulting mixture was stirred for 90 min and subsequently diluted with ether (40 mL). The obtained suspension was filtered and the filtrate was evaporated *in vacuo.* The residue was dissolved in ether (100 mL) and the formed solution was washed with 1 M hydrochloric acid (3 x 20 mL), water (20 mL), 10 % aqueous solution of sodium hydrogen carbonate (20 mL), water (20 mL) and brine (20 mL). The organic solution was dried with anhydrous magnesium sulfate and subsequently evaporated *in vacuo.* The residue was dissolved in absolute methanol (70 mL), the resulting turbid solution was degassed and cooled with ice-water. In atmosphere of nitrogen, anhydrous potassium carbonate (1.55 g,11.2 mmol) was added and the resulting suspension was stirred for 2 h under cooling. The suspension was filtered and the filtrate was evaporated *in vacuo.* The residue was dissolved in dichloromethane (100 mL) and the solution was washed with water (3 x 20 mL) and brine (20 mL). The organic solution was dried with anhydrous magnesium sulfate and subsequently evaporated *in vacuo.* The obtained residue was purified by column chromatography (silica gel Fluka 60, hexane/ethyl acetate 2:1) yielding 3,3-bis(4-ethylnylphenyl)allyl alcohol as a solidifying oil.
Yield: 1.02 g (73 %).
M.p. 94 -97°C (cyclohexane).
R_{F} (SiO₂, dichloromethane) 0.25.
¹H NMR spectrum (250 MHz, CDCl₃, δ_{H}): 7.50 (dm, J=8.4 Hz, 2 H); 7.41 (dm, J=8.5 Hz, 2 H); 7.18 (dm, J=8.7 Hz, 2 H); 7.12 (dm, J=8.5 Hz, 2 H); 6.27 (t, J=6.9 Hz, 1 H); 4.21 (d, J=6.9 Hz, 2 H); 3.12 (s, 1 H); 3.10 (s, 1 H).

Paraformaldehyde (79 mg, 2.63 mmol), copper(I) iodide (49 mg, 0.257 mmol) and 40 % aqueous solution of dimethylamine (0.48 mL, 3.79 mmol) were added to a solution of the above allyl alcohol (298 mg, 1.15 mmol) in dioxane (13 mL). The resulting mixture was refluxed for 1 h, cooled down and evaporated *in vacuo.* The residue was purified by flash column chromatography (silica gel Fluka 60, chloroform saturated with ammonia/methanol 97:3) yielding 3,3-bis[4-[3-(N,N-dimethylamino)propyn-1-yl]phenyl]allyl alcohol as an oil.
Yield: 307 mg (71 %).

M.p. --- (oil).
R_{F} (SiO₂, chloroform saturated with ammonia/methanol 95:5) 0.30.
¹H NMR spectrum (250 MHz, CDCl₃, δ_{H}): 7.43 (dm, J=8.4 Hz, 2 H); 7.34 (dm, J=8.6 Hz, 2 H); 7.17 (dm, J=8.6 Hz, 2 H); 7.09 (dm, J=8.4 Hz, 2 H); 6.25 (t, J=6.9 Hz, 1 H); 4.22 (d, J=6.8 Hz, 2 H); 3.47 (s, 2 H); 3.45 (s, 2 H); 2.37 (s, 6 H); 2.35 (s, 6 H).

### General procedure (B)

### Step A and B:

An excess of a solution of hydrogen chloride in dry ether was added dropwise to a solution of the above allyl alcohol (307 mg, 0.824 mmol) in dry tetrahydrofuran (5 mL). The formed heterogeneous mixture was stirred for 10 min and subsequently evaporated *in vacuo.* Thionyl chloride (10 mL) was added to the residue; the formed solutions was stirred for 60 min and evaporated *in vacuo.* Dry toluene (15 mL) was added to the residue and the mixture was evaporated again. In atmosphere of nitrogen, the residue was dissolved in dry N,N-dimethylformamide (10 mL) and N,N-diisopropylethylamine (1.40 mL, 8.03 mmol) and subsequently a solution of ethyl (4-mercapto-2-methylphenoxy)acetate (308 mg, 1.36 mmol) in dry N,N-dimethylformamide (2 mL) were added. The resulting mixture was stirred overnight and subsequently poured into water (75 mL). The heterogeneous mixture was extracted with ethyl acetate (3 x 30 mL); the organic layers were collected and washed with brine (3 x 20 mL), water (2 x 20 mL) and brine (2 x 20 mL). The organic solution was dried with anhydrous magnesium sulfate and evaporated *in vacuo.* The residue was purified by flash column chromatography (silica gel Fluka 60, chloroform saturated with ammonia/methanol 99:1) yielding ethyl [4-[3,3-bis[4-[3-(N,N-dimethylamino)propyn-1-yl]phenyl]allylsulfanyl]-2-methylphenoxy]acetate.
Yield: 304 mg (63 %).
M.p. --- (oil).
R_{F} (SiO₂, chloroform saturated with ammonia/methanol 95:5) 0.45.
¹H NMR spectrum (200 MHz, CDCl₃, δ_{H}): 7.37 (dm, J=8.3 Hz, 2 H); 7.32 (dm, J=8.4 Hz, 2 H); 7.09 (m, 4 H); 6.83 (dm, J=8.4 Hz, 2 H); 6.55 (d, J=9.0 Hz, 1 H); 6.13 (t, J=7.8 Hz, 1 H); 4.61 (s, 2 H); 4.23 (q, J=7.2 Hz, 2 H); 3.48 (m, 6 H); 2.38 (s, 6 H); 2.35 (s, 6 H); 2.20 (s, 3 H); 1.26 (t, J=7.2 Hz, 3 H).

### General procedure (D)

### Step A:

In atmosphere of nitrogen, a solution of lithium hydroxide monohydrate (12 mg, 0.286 mmol) in distilled water (0.5 mL) was added to an ice-water cooled solution of the above ester (109 mg, 0.188 mmol) in a mixture tetrahydrofuran/methanol (5:1, 3 mL). The resulting solution was stirred for 60 min under cooling, neutralized by addition of a solution of glacial acetic acid (17 mg, 0.283 mmol) in water (0.8 mL) and diluted with dichloromethane (30 mL). The mixture was washed with water (3 x 10 mL) and the collected aqueous phases were re-extracted with dichloromethane (10 mL). The organic phases were combined, dried with anhydrous magnesium sulfate and evaporated *in vacuo.* The residue was triturated with acetonitrile (2 x 4 mL) yielding the title acid as a solid mass.
Yield: 51 mg (49 %).
M.p. 136 -144°C (amorphous).
R_{F} (SiO₂, chloroform saturated with ammonia/methanol 95:5) 0.05.
¹H NMR spectrum (200 MHz, CDCl₃, δ_{H}): 9.49 (bs, ∼1 H); 7.32 (m, -4 H); 7.07 (m, 4 H); 6.71 (d, J=7.9 Hz, 2 H); 6.59 (d, J=9.0 Hz, 1 H); 6.15 (t, J=8.0 Hz, 1 H); 4.55 (s, 2 H); 3.64 (s, 2 H); 3.52 (s, 2 H); 3.42 (d, J=8.0 Hz, 2 H); 2.51 (s, 6 H); 2.41 (s, 6 H); 2.18 (s, 3 H).

### Example 11

### [4-[3,3-Bis[4-[3-(morpholine-4-yl)propynyl]phenyl]allyloxy]-2-methylphenoxy]acetic acid

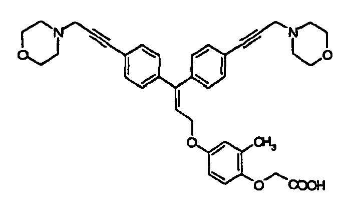

### General procedure (A)

### Step C:

In atmosphere of nitrogen, diisopropylazodicarboxylate (0.16 mL, 0.807 mmol) was added to an ice-water cooled solution of 3,3-bis[4-[3-(morpholine-4-yl)propynyl]phenyl]allyl alcohol (290 mg, 0.635 mmol; prepared as described in example 9), triphenylphosphine (217 mg, 0.827 mmol) and methyl (4-hydroxy-2-methylphenoxy)acetate (137 mg, 0.698 mmol; prepared as described in example 6) in a mixture of anhydrous toluene and tetrahydrofuran (2:1, 12 mL). The mixture was stirred for 20 min under cooling and subsequently overnight at ambient temperature. The reaction solution was concentrated to half volume *in vacuo;* toluene (20 mL) was added and the resulting solution was washed with 10 % aqueous sodium hydroxide (3 x 20 mL), water (20 mL) and brine (2 x 20 mL). The organic solution was dried with anhydrous magnesium sulfate and evaporated *in vacuo.* The residue was purified by column chromatography (silica gel Fluka 60, dichloromethane/methanol 98:2) yielding methyl [4-[3,3-bis[4-[3-(morpholine-4-yl)propynyl]phenyl]allyloxy]-2-methylphenoxy]acetate.
Yield: 297 mg (74 %).
M.p. --- (oil).
R_{F} (SiO₂, dichloromethane/methanol 95:5) 0.30.
¹H NMR spectrum (200 MHz, CDCl₃, δ_{H}): 7.72-6.57 (m, ∼11 H), 6.32 (t, J=6.7 Hz, 1 H), 4.58
(s, 2 H), 4.50 (d, J=6.6 Hz, 2 H), 3.79 (s, ∼3 H), 3.78 (m, ~8 H), 3.54 (s, 2 H), 3.51 (s, 2 H), 2.62 (m, ~8 H), 2.25 (s, 3 H).

### General procedure (D)

### Step A:

Lithium hydroxide monohydrate (38 mg, 0.906 mmol) was added to a solution of the above methyl ester (191 mg, 0.301 mmol) in a mixture tetrahydrofuran/methanol/water (5:1:1, 7 mL). The resulting solution was stirred at 40 °C for 1 h and subsequently a solution of glacial acetic acid (51.5 µL, 0.901 mmol) in distilled water (4 mL) was added and the mixture was diluted with water (50 mL) and ether (30 mL). The aqueous phase was extracted with ether (2 x 30 mL) and the combined ethereal layers were washed with water (2 x 20 mL) and brine (2 x 20 mL). The organic solution was dried with anhydrous sodium sulfate and evaporated *in vacuo* yielding the title acid as an oil.
Yield: 171 mg (92 %).
M.p. --- (oil).
R_{F} (SiO₂, dichloromethane/methanol 90:10) 0.20.
¹H NMR spectrum (200 MHz, CDCl₃, δ_{H}): 7.44-6.58 (m, ∼11 H), 6.33 (t, J=6.8 Hz, 1H), 4.54 (s, ∼2 H), 4.46 (d, J=6.8 Hz, -2 H), 3.81 (m, ~8 H), 3.63 (s, ~2 H), 3.58 (s, ~2 H), 2.76 (m, ~8 H), 2.25 (s, -3 H).

A solution of L-lysine (37 mg, 0.253 mmol) in distilled water (0.5 mL) was added to a solution of the above acid (164 mg, 0.264 mmol) in dry tetrahydrofuran (5 mL). The resulting solution was stirred for 90 min, evaporated *in vacuo* and the residue was evaporated with absolute ethanol (3 x 10 mL). The residue was triturated with anhydrous ether (2 x 15 mL) yielding L-lysinate of the title acid.
Yield: 147 mg (73 %). M.p. 136-145 °C (amorphous).
¹H NMR spectrum (200 MHz, DMSO-d₆, δ_{H}): 7.51-6.58 (m, ∼11 H); 6.36 (t, J=6.8 Hz, 1 H); 4.44 (d, J=6.8 Hz, 2 H); 4.21 (s, 2 H); 3.59 (m, ∼8 H); 3.52 (s, ~2 H); 3.50 (s, ~2 H); 3.18 (m, 1 H); 2.71 (m, ∼2 H); -2.50 (m, ~8 H), 2.09 (s, 3 H), 1.70-1.27 (m, 6 H).

### Example 12

### [4-(3,3-Bis-{4-[3-(4-acetyl-piperazin-1-yl)-prop-1-ynyl]-phenyl}allylsulfanyl)-2-methylphenoxy]acetic acid

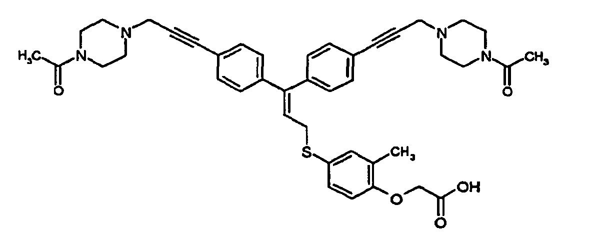

A mixture of 1-piperazin-1-yl-ethanone (15.2 g, 0.119 mol; prepared as described in US 2973362), 3-bromopropyne (17 g, 0.143 mol) and potassium carbonate anhydrous (21.5 g, 0.156 mol) in 2-butanone (150 mL) was refluxed for 6 h. A separated solid was filtered off, washed with 2-butanone (80 mL) and 2-butanone was evaporated *in vacuo.* The residue was purified by vacuum distillation to yield 1-(4-prop-2-ynylpiperazin-1-yl)ethanone, b.p. 115 °C/7 Torr).
Yield: 12.2 g (62 %).
M.p. 63-65 °C.

### General procedure (E)

### Step B:

Copper(I) iodide (24 mg, 0.126 mmol) and tetrakis(triphenylphosphine)palladium (63 mg, 0.055 mmol) were added to a degassed solution of ethyl [4-[3,3-bis(4-iodophenyl)-allylsulfanyl]-2-methylphenoxy]acetate (600 mg, 0.895 mmol; prepared as described in example 3), 1-(4-prop-2-ynylpiperazin-1-yl)ethanone (447 mg, 2.69 mmol) in the mixture of anhydrous triethylamine (25 mL) and tetrahydrofuran (15 mL). In atmosphere of nitrogen, the resulting mixture was stirred at 50 °C for 5 h. The dark suspension was evaporated *in vacuo,* ethyl acetate (50 mL) was added to the residue and the mixture was washed with water (2 x 50 mL) and brine (2 x 40 mL). The organic solution was dried with anhydrous magnesium sulfate and evaporated *in vacuo.* The residue was purified by flash column chromatography (silica gel Fluka 60, dichloromethane/methanol 982, 95:5) yielding ethyl [4-[3,3-bis[4-[3-(4-acetylpiperazin-1-yl)prop-1-ynyl]phenyl]allylsulfanyl]-2-methylphenoxy]acetate as a brown oil.
Yield: 386 mg (58 %).
M.p. --- °C (oil).
R_{F} (SiO₂, dichloromethane/methanol 95:5) 0.20.
¹H NMR spectrum (200 MHz, CDCl₃, δ_{H}): 7.38-6.50 (m, ∼11 H); 6.14 (t, J=7.9 Hz, 1 H); 4.62 (s, 2 H); 4.24 (q, ~2 H); 3.71-3.46 (m, ~8 H); 3.58 (s, ~2 H); 3.55 (s, ~2 H); 3.48 (d, ~2 H); 2.62 (m, ~8 H); 2.20 (s, ~3 H); 2.10 (s, ~3 H); 2.08 (s, ~3 H); 1.27 (t, ∼3 H).

### General procedure (D)

### Step A:

In atmosphere of nitrogen, lithium hydroxide monohydrate (32 mg, 0.763 mmol) was added to an ice-water cooled solution of the above ester (378 mg, 0.506 mmol) in tetrahydrofuran/methanol/distilled water mixture (5:1:1; 7 mL) and the resulting solution was stirred for 2 h under cooling. Glacial acetic acid (0.043 mL, 0.752 mmol) was added dropwise and the solution was stirred for further 10 min. The reaction mixture was diluted chloroform (40 mL), washed with water (2 x 40 mL) and brine (2 x 40 mL). The organic solution was dried with anhydrous sodium sulfate and evaporated *in vacuo* giving sufficiently pure title acid.
Yield: 188 mg (52 %).
M.p. --- (oil).
R_{F} (SiO₂, dichloromethane/methanol 90:10) 0.15.
¹H NMR spectrum (200 MHz, CDCl₃, δ_{H}): 7.74-6.47 (m, ∼11 H); 6.18 (t, J=8.2 Hz, 1 H); 4.61 (s, 2 H); 3.68-3.50 (m, ~8 H); 3.60 (s, ~2 H); 3.55 (s, ~2 H); 3.42 (d, J=8.2 Hz, 2 H); 2.67 (m, ~8 H); 2.17 (s, ∼3 H); 2.14 (s,∼3 H); 2.11 (s, ∼3 H).

A solution of L-lysine (37 mg, 0.253 mmol) in distilled water (0.5 mL) was added to a solution of the above acid (188 mg, 0.262 mmol) in dry tetrahydrofuran (5 mL). The resulting solution was stirred for 2.5 h, evaporated *in vacuo* and the residue was evaporated with absolute ethanol (3 x 10 mL). The residue was triturated with anhydrous ether (2 x 10 mL) yielding L-lysinate of the title acid.
Yield: 144 mg (64 %). M.p. 105 -115 °C (amorphous).
¹H NMR spectrum (250 MHz, DMSO-d₆, δ_{H}): 7.41-6.63 (m, 11 H); 6.18 (t, J=7.7 Hz, 1 H); 4.35 (s, 2 H); 3.58 (s, 2 H); 3.55 (s, 2 H); 3.50 (d, ~2 H); 3.46 (m, 8 H); 3.12 (m, ~1 H); 2.77 (m, ~2 H); 2.50 (m, ~8 H); 2.07 (s, ~3 H); 2.00 (s, ~3 H); 1.98 (s, ~3 H); 1.79-1.19 (m, ∼6 H).

### Example 13

### [4-[3,3-Bis[4-(3-pyrrolidin-1-yl-prop-1-ynyl)phenyl]allylsulfanyl]-2-methylphenoxy]acetic acid

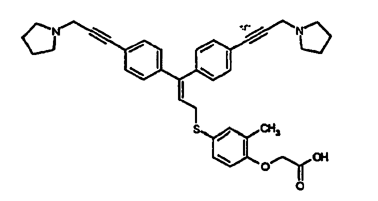

### General procedure (E)

### StepB:

Copper(I) iodide (20 mg, 0.105 mmol) and tetrakis(triphenylphosphine)palladium (52 mg, 0.045 mmol) were added to a degassed solution of ethyl [4-[3,3-bis(4-iodophenyl)-allylsulfanyl]-2-methylphenoxy]acetate (500 mg, 0.746 mmol) and 1-prop-2-ynyl-pyrrolidine (244 mg, 2.24 mmol; prepared as described in J. Med. Chem. 1991, 34, 1073) in anhydrous triethylamine (20 mL) and tetrahydrofuran (10 mL). In atmosphere of nitrogen, the resulting mixture was stirred at 50 °C for 3 h. The dark suspension was evaporated *in vacuo,* ethyl acetate (50 mL) was added to the residue and the mixture was washed with water (2 x 50 mL) and brine (2 x 40 mL). The organic solution was dried with anhydrous magnesium sulfate and evaporated *in vacuo.* The residue was purified by flash column chromatography (silica gel Fluka 60, dichloromethane/methanol 98:2, 95:5) yielding ethyl [4-[3,3-bis[4-(3-pyrrolidin-1-yl-prop-1-ynyl)phenyl]allylsulfanyl]-2-methylphenoxy]acetate as a brown oil.
Yield: 491 mg.
M.p. -- °C (oil).
R_{F} (SiO₂, dichloromethane/methanol 95:5) 0.20.
¹H NMR spectrum (200 MHz, CDCl₃, δ_{H}): 7.74-6.54 (m, ∼11 H); 6.13 (t, J=7.8 Hz, 1 H); 4.62 (s, 2 H); 4.24 (q, ~2 H); 3.71 (s, ~2 H); 3.68 (s, ~2 H); 3.48 (d, J=8.0 Hz, 2 H); 2.79 (m, ~8 H); 2.20 (s, 3 H);1.88 (m, ∼8 H); 1.27 (t, -3 H).

### General procedure (D)

### Step A:

In atmosphere of nitrogen, lithium hydroxide monohydrate (48 mg, 1.14 mmol) was added to an ice-water cooled solution of the above ester (487 mg, 0.770 mmol) in tetrahydrofuran/methanol/distilled water mixture (5:1:1; 7 mL) and the resulting solution was stirred for 90 min under cooling. Glacial acetic acid (0.066 mL, 1.15 mmol) was added dropwise and the solution was stirred for further 10 min. The reaction mixture was diluted with chloroform (40 mL), washed with water (2 x 40 mL) and brine (2 x 40 mL). The organic solution was dried with anhydrous magnesium sulfate and evaporated *in vacuo* giving sufficiently pure title acid.
Yield: 184 mg (40 %). M.p. --- (oil).
¹H NMR spectrum (200 MHz, CDCl₃, δ_{H}): 7.74-6.51 (m, ∼11 H); 6.18 (t, J=8.3 Hz, 1 H); 4.53 (s, 2 H); 3.83 (s, 2 H); 3.70 (s, 2 H); 3.41 (d, J=7.9 Hz, 2 H); 3.01 (m, ∼8 H); 2.18 (s, 3 H); 1.95 (m, ∼8 H).

A solution of L-lysine (40 mg, 0.274 mmol) in distilled water (0.5 mL) was added to a solution of the above acid (174 mg, 0.288 mmol) in dry tetrahydrofuran (5 mL). The resulting solution was stirred for 1.5 h, evaporated *in vacuo* and the residue was evaporated with absolute ethanol (3 x 10 mL). The residue was triturated with anhydrous ether (3 x 10 mL) yielding L-lysinate of the title acid.
Yield: 109 mg (51 %).
M.p. 134 -150 ° C (amorphous).
¹H NMR spectrum (200 MHz, DMSO-d₆, δ_{H}): 7.40-6.57 (m, ∼11 H); 6.17 (t, J=7.9 Hz, 1 H); 4.22 (s, 2 H); 3.58 (s, ∼2 H); 3.61 (s, ~2 H); 3.43 (d, J=7.7 Hz, ~2 H); 3.13 (m, 1 H); 2.72 (m, 2 H); -2.50 (m, ~8 H); 2.05 (s, 3 H); 1.71 (m, ~8 H); 1.59-1.29 (m, ~6 H).

### Example 14

### (4-{3,3-Bis-[4-(3-pyrrolidin-1-yl-prop-1-ynyl)phenyl]allyloxy}-2-methylphenoxy)acetic acid

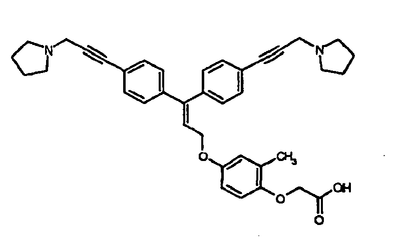

### General procedure (E)

### Step B:

Copper(I) iodide (26 mg, 0.14 mmol), and tetrakis(triphenylphosphine)palladium (69 mg, 0.06 mmol) were added to a degassed solution of 14-[3,3-bis-(4-iodophenyl)allylsulfanyl]-2-methylphenoxy}acetic acid methyl ester (640 mg, 1.0 mmol), N-propargylpyrrolidine (382 mg, 3.5 mmol), and triethylamine (25 mL) in dry tetrahydrofuran (15 ml). The resulting mixture was stirred at 50 °C for 3 h in atmosphere of argon and subsequently evaporated *in vacuo.* The residue was dissolved in ethyl acetate (60 ml) the solution was washed with water (25 mL), brine (25 mL) dried (MgSO₄) and evaporated. The residue was purified by flash column chromatography (silica gel Fluka 60). Fractions eluted with dichloromethane and with a mixture of methanol/dichloromethane (1:25) were discarded. The next fraction which were eluted with the mixture of methanol/chloroform saturated with NH₃/dichloromethane (5:10:85) afforded (4-{3,3-bis-[4-(3-pyrrolidin-1-yl-prop-1-ynyl)phenyl]allyloxy}-2-methylphenoxy)acetic acid methyl ester as an oil.
Yield: 530 mg (88 %).
R_{F} (SiO₂, dichloromethane/methanol 9:1) 0.30.
¹H NMR spectrum (200 MHz, CDCl₃, δ_{H}): 7.11-7.48 (m, 8 H); 6.55-6.67 (m, 3 H); 6.32 (t, J=6.7 Hz, 1H); 4.58 (s, 2 H); 4.49 (d, J=6.6 Hz, 2 H); 3.79 (s, 3 H); 3.70 (m, 4 H); 2.79 (m, 8 H); 2.25 (s, 3 H); 1.88 (m, 8 H).

### General procedure (D)

### Step A:

In atmosphere of argon, lithium hydroxide monohydrate (52 mg, 1.24 mmol) was added to an ice-water cooled solution of the above ester (530 mg, 0.827 mmol) in a mixture tetrahydrofuran/methanol/distilled water (5:1:1; 9 mL) and the resulting solution was stirred for 90 min under cooling. The reaction mixture was diluted with water (10 mL), neutralized with acetic acid and extracted with chloroform (3 x 25 mL). The combined organic layers were washed with water (10 mL), brine (2 x 10 mL), dried with anhydrous magnesium sulfate and evaporated *in vacuo* yielding the title acid.
Yield: 460 mg (95 %).
¹H NMR spectrum (250 MHz, DMSO-d₆ + CD₃COOD, δ_{H}): 7.33-7.51 (m, ~4 H); 7.13-7.19 (m, 4 H); 6.43-6.63 (m, 3 H); 6.35 (t, 1 H); 4.55 (s 2 H); 4.44 (d, 2 H); 4.26 (d, 4 H); 3.69 /(m, 8 H); 2.11 (s 3 H); 2.03 (m, 8 H).

A solution of L-lysine (107 mg, 0.73 mmol) in distilled water (1.0 mL) was added to a solution of the above acid (430 mg, 0.73 mmol) in the mixture of tetrahydrofuran (15 mL) and acetone (25 ml). The resulting solution was stirred for 90 min, filtered and evaporated *in vacuo.* The residue was triturated with anhydrous ether (3 x 30 mL) to give L-lysinate of the title acid.
Yield: 380 mg (71 %).
M.p. 155-165 °C.
¹H NMR spectrum ((250 MHz, DMSO-d₆ + CD₃COOD, δ_{H}): 7.37-7.51 (m, 4 H); 7.15-7.20 (m, 4 H); 6.55-6.65 (m, 3 H); 6.36 (t, J=6.6 Hz, 1 H); 4.44 (s, 2 H); 4.43 (d, J=6.2 Hz, 2 H); 3.90 s and 3.92 s, E 4 H; 3.39 (bt, 1H); 2.94 (m, ∼ 8 H); 2.75 (bt, 2 H); 2.10 (s, 3 H); 1.30-1.85 (m, ~ 12 H).

### Example 15

### [4-[3,3-Bis[5-[3-(morpholine-4-yl)propynyl]thiophene-2-yl]allylsuffanyl]-2-methylphenoxy]-acetic acid

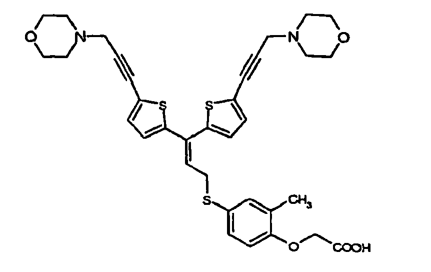

In atmosphere of nitrogen, a solution of triethyl phosphonoacetate (4.30 mL, 21.5 mmol) in dry tetrahydrofuran (5 mL) was added dropwise to a suspension of sodium hydride (80%; 0.680 g, 22.7 mmol) in dry tetrahydrofuran (30 mL). When the gas evolution deceased, the mixture was heated to reflux for 30 min. The formed solution was cooled down, a solution of di(5-bromothiophen-2-yl)methanone (3.03 g, 8.61 mmol; prepared as described in Rec.Trav.Chim.Pays-Bas 1949, 68, 29) in a mixture of anhydrous tetrahydrofuran and toluene (2:1, 15 mL) was added and the resulting solution was refluxed for 2 h. The reaction mixture was cooled down, poured into diluted aqueous solution of tartaric acid (40 mL) and extracted with ethyl acetate (3 x 40 mL). The combined organic layers were washed with water (2 x 30 mL), 10 % aqueous solution of sodium hydrogen carbonate (30 mL), 10 % aqueous solution of sodium metabisulfite (2 x 30 mL) and brine (2 x 30 mL). The organic solution was dried with anhydrous magnesium sulfate and subsequently evaporated *in vacuo.* The residue was purified by crystallization from ethanol yielding ethyl 3,3-di(5-bromothiophen-2-yl)acrylate as sandy crystals.
Yield: 2.55 g (70 %). M.p. 69 - 70° C (ethanol).
R_{F} (SiO₂, hexane/toluene 3:1) 0.15.
¹H NMR spectrum (200 MHz, CDCl₃, δ_{H}): 7.04 (d, J=3.8 Hz, 1 H); 6.99 (d, J=3.9 Hz, 1 H); 6.90 (d, J=3.8 Hz, 1 H); 6.86 (d, J=4.0 Hz, 1H); 6.23 (s, 1 H); 4.12 (q, J=7.2 Hz, 2 H); 1.21 (t, J=7.2 Hz, 3 H).

In atmosphere of nitrogen, cold solution of aluminum chloride (0.666 g, 4.99 mmol) in anhydrous ether (15 mL) was added dropwise to a cooled (-20 °C) solution of lithium aluminum hydride (0.569 g, 15.0 mmol) in dry ether (50 mL) maintaining the reaction temperature between -20 and -18 °C. The resulting suspension was allowed to warm up to -5 °C, stirred at temperature between -5 and 0 °C for 30 min and cooled to -19 °C again. Cooled solution of the above acrylate (2.11 g, 5.00 mmol) in anhydrous ether (15 mL) was added slowly to the reaction mixture maintaining the reaction temperature between -20 and -17 °C. The reaction mixture was stirred for 45 min bellow -17 °C and subsequently water (0.6 mL), 10 % aqueous solution of sodium hydroxide (0.6 mL) and again water (1.8 mL) were added. The resulting suspension was allowed to warm up to ambient temperature; the solid mass was filtered off and washed with ether. The combined filtrates were washed with water (2 x 50 mL) and brine (2 x 50 mL). The organic solution was dried with anhydrous magnesium sulfate and subsequently evaporated *in vacuo* yielding crude 3,3-di(5-bromothiophen-2-yl)allyl alcohol as an slowly solidifying oil.
Yield: 1.72 g (91 %).
R_{F} (SiO₂, hexane/ethyl acetate 2:1) 0.30.
¹H NMR spectrum (200 MHz, CDCl₃, δ_{H}): 7.04 (d, J=3.8 Hz, 1 H); 6.92 (d, J=3.9 Hz, 1 H); 6.80 (d, J=3.8 Hz, 1 H); 6.70 (d, J=3.9 Hz, 1 H); 6.21 (t, J=6.8 Hz, 1 H); 4.28 (d, J=6.8 Hz, 2 H).

### General procedure (E)

### Step A:

In atmosphere of nitrogen, cooled solution of tetrabromomethane (2.25 g, 6.78 mmol) in dry dichloromethane (2 mL) was added to an ice-water cooled solution of the above allyl alcohol (1.72 g, 4.52 mmol) and triphenylphosphine (1.78 g, 6.79 mmol) in dry dichloromethane (50 mL). The mixture was stirred for 30 min under cooling and subsequently water (15 drops) was added. After 15 min, N,N-diisopropylethylamine (1.50 mL, 8.61 mmol) and subsequently a solution of ethyl (4-mercapto-2-methylphenoxy)acetate (2.05 g, 9.06 mmol) in dry dichloromethane (3 mL) were added and the resulting solution was stirred for 3.5 h at ambient temperature under nitrogen and for further 15 min on air. The mixture was diluted with dichloromethane (50 mL) and the solution was washed with water (2 x 25 mL) and brine (2 x 25 mL). The organic solution was dried with anhydrous magnesium sulfate and evaporated *in vacuo.* The residue was purified by column chromatography (silica gel Fluka 60, hexane/ethyl acetate 20:1 + 0.1 % of triethylamine) yielding ethyl [4-[3,3-di(5-bromothiophen-2-yl)allylsulfanyl]-2-methylphenoxy]acetate as a dark red oil.
Yield: 1.64 mg (62 %).
M.p. --- (oil).
R_{F} (SiO₂, hexane/ethyl acetate 20:1) 0.10.
¹H NMR spectrum (200 MHz, CDCl₃ δ_{H}): 7.15 (m, 2 H); 6.94 (d, J=3.8 Hz, 1 H); 6.87 (d, J=3.9 Hz, 1H); 6.58 (d, J=8.4 Hz, 1 H); 6.55 (d, J=3.9 Hz, 1 H); 6.44 (d, J=3.8 Hz, 1 H); 6.10 (t, J=8.0 Hz, 1 H); 4.63 (s, 2 H); 4.25 (q, J=7.2 Hz, 2 H); 3.53 (d, J=8.0 Hz, 2 H); 2.23 (s, 3 H); 1.29 (t, J=7.2 Hz, 3 H).

### Step B:

Copper(I) iodide (22 mg, 0.116 mmol) and tetrakis(triphenylphosphine)palladium (67 mg, 0.058 mmol) were added to a degassed solution of ethyl [4-[3,3-bis(5-bromothiophene-2-yl)allylsulfanyl]-2-methylphenoxy]acetate (424 mg, 0.721 mmol) and N-propargylmorpholine (375 mg, 3.00 mmol) in anhydrous triethylamine (15 mL). In atmosphere of nitrogen, the resulting mixture was stirred at ambient temperature for 2 h and subsequently for 3 h at 65 °C. The dark suspension was evaporated *in vacuo,* dichloromethane (50 mL) was added to the residue and the mixture was washed with water (2 x 10 mL), aqueous solution of sodium bisulphite (2 x 10 mL) and brine (2 x 10 mL). The organic solution was dried with anhydrous magnesium sulfate and evaporated *in vacuo.* The residue was purified by column chromatography (silica gel Fluka 60, dichloromethane/methanol/triethylamine 97:3:0.1) yielding ethyl [4-[3,3-bis[5-[3-(morpholine-4-yl)propynyl]thiophene-2-yl]allylsulfanyl]-2-methylphenoxy]acetate as a brown oil.
Yield: 411 mg (84 %). M.p. --- °C (oil).
R_{F} (SiO₂, dichloromethane/methanol 95:5) 0.35.
¹H NMR spectrum (200 MHz, CDCl₃, δ_{H}): 7.17 (m, 2 H); 7.07 (d, J=3.6 Hz, 1 H); 6.99 (d, J=3.8 Hz, 1 H); 6.64 (d, J=3.8 Hz, 1 H); 6.58 (bd, J=9.0 Hz, 1 H); 6.54 (d, J=3.6 Hz, 1 H); 6.19 (t, J=7.9 Hz, 1 H); 4.63 (s, 2 H); 4.25 (q, J=7.2 Hz, 2 H); 3.77 (m, -8 H); 3.54 (m, 6 H); 2.62 (m, -8 H); 2.23 (s, 3 H); 1.28 (t, J=7.2 Hz, 3 H).

### General procedure (D)

### Step A:

In atmosphere of nitrogen, a solution of lithium hydroxide monohydrate (38 mg, 0.906 mmol) in distilled water (1 mL) was added to an ice-water cooled solution of the above ester (411 mg, 0.607 mmol) in a mixture tetrahydrofuran/methanol (5:1; 6 mL) and the resulting solution was stirred for 45 min under cooling. The reaction mixture was diluted with water (50 mL), acetic acid (54 mg, 0.899 mmol) and solid sodium chloride were added and the solution was extracted with ether (5 x 15 mL). The combined organic layers were washed with water (2 x 10 mL) and brine (2 x 10 mL), dried with anhydrous magnesium sulfate and evaporated *in vacuo* yielding sufficiently pure title acid.
Yield: 291 mg (74 %).
M.p. --- (oil).
R_{F} (SiO₂, dichloromethane/methanol 90:10) 0.20.
¹H NMR spectrum (200 MHz, CDCl₃, δ_{H}): 7.16 (m, ~2 H); 6.98 (m, 2 H); 6.63 (m, 3 H); 6.24 (t, J=8.2 Hz, 1 H); 4.62 (s, 2 H); 3.79 (m, ~8 H); 3.56 (s, 2 H); 3.53 (s, 3 H); 3.43 (d, J=8.2 Hz, 2 H); 2.82 (bs, ∼4 H); 2.67 (m, ∼4 H); 2.19 (s, 3 H).

A solution of L-lysine (50 mg, 0.342 mmol) in distilled water (0.8 mL) was added to a solution of the above acid (235 mg, 0.362 mmol) in dry tetrahydrofuran (8 mL). The resulting solution was stirred for 60 min, evaporated *in vacuo* and the residue was evaporated with absolute ethanol (2 x 10 mL). The residue was triturated with anhydrous ether (3 x 10 mL) yielding L-lysinate of the title acid.
Yield: 243 mg (84 %).
M.p. 113 -128 °C (amorphous).
¹H NMR spectrum (200 MHz, DMSO-d₆, δ_{H}): 7.31-7.07 (m, ~4 H); 6.94-6.61 (m, 3 H); 6.28 (t, J=8.0 Hz, 1 H); 4.25 (bs, 2 H); 3.59 (m, 14 H); 3.20 (bs, ∼1 H); 2.72 (bs, ∼2 H); 2.50 (m, ∼8 H); 2.09 (s, 3 H); 1.73-1.27 (m, ~6 H).

### Example 16

### [4-[3,3-Bis[5-[3-(N-acetyl-N-methylamino)propynyl]thiophene-2-yl]allylsulfanyl]-2-methylphenoxy]acetic acid

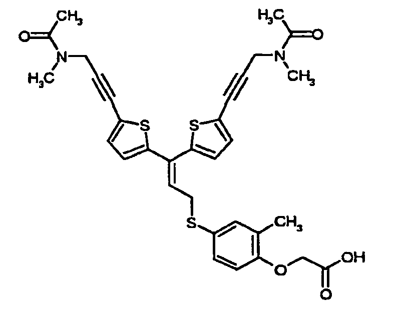

### General procedure (E)

### Step B:

Copper(I) iodide (27 mg, 0.142 mmol) and tetrakis(triphenylphosphine)palladium (80 mg, 0.069 mmol) were added to a degassed solution of ethyl [4-[3,3-bis(5-bromothiophene-2-yl)allylsulfanyl]-2-methylphenoxy]acetate (512 mg, 0.870 mmol; prepared as described example 15) and N-methyl-N-propargylacetamide (338 mg, 3.04 mmol; prepared as described in J. Med. Chem. 1991, 34, 1073) in anhydrous triethylamine (15 mL). In atmosphere of nitrogen, the resulting mixture was stirred at 65 °C for 6 h. The dark suspension was evaporated *in vacuo,* dichloromethane (50 mL) was added to the residue and the mixture was washed with water (2 x 10 mL) and brine (2 x 10 mL). The organic solution was dried with anhydrous magnesium sulfate and evaporated *in vacuo.* The residue was purified by flash column chromatography (silica gel Fluka 60, dichloromethane/methanol 97:3) yielding ethyl [4-[3,3-bis[5-[3-(N-acetyl-N-methylamino)propynyl]thiophene-2-yl]allylsulfanyl]-2-methylphenoxy]acetate as a brown oil.
Yield: 250 mg (44 %).
M.p. ---°C (oil).
R_{F} (SiO₂, dichloromethane/methanol 95:5) 0.20.
¹H NMR spectrum (200 MHz, CDCl₃, δ_{H}): 7.17-6.97 (m, ~4 H); 6.66-6.51 (m, 3 H); 6.21 and 6.19 (t, 1 H); 4.63 (s, 2 H); 4.47 and 4.46 and 4.29 and 4.27 (s, 4 H); 4.25 (q, J=7.2 Hz, 2 H); 3.54 (d, 2 H); 3.13 and 3.11 and 3.04 and 3.03 (s, 6 H); 2.23 (s, 3 H); 2.21 and 2.20 and 2.14 and 2.13 (s, 6 H); 1.28 (t, J=7.2 Hz, 3 H).

### General procedure (D)

### Step A:

In atmosphere of nitrogen, a solution of lithium hydroxide monohydrate (18.9 mg, 0.450 mmol) in distilled water (1 mL) was added to an ice-water cooled solution of the above ester (188 mg, 0.290 mmol) in a mixture tetrahydrofuran/methanol (5:1; 6 mL) and the resulting solution was stirred for 45 min under cooling. Acetic acid (0.025 mL, 0.437 mmol) was added dropwise, the solution was stirred for 10 min and subsequently diluted with dichloromethane (50 mL). The mixture was washed with water (2 x 10 mL) and brine (2 x 10 mL), dried with anhydrous magnesium sulfate and evaporated *in vacuo* yielding sufficiently pure title acid.
Yield: 150 mg (83 %).
M.p. --- (oil).
R_{F} (SiO₂, dichloromethane/methanol 90:10) 0.15.

A solution of L-lysine (34 mg, 0.233 mmol) in distilled water (0.5 mL) was added to a solution of the above acid (150 mg, 0.242 mmol) in dry tetrahydrofuran (5 mL). The resulting solution was stirred for 60 min, evaporated *in vacuo* and the residue was evaporated with absolute ethanol (2 x 10 mL). The residue was triturated with anhydrous ether (3 x 10 mL) yielding L-lysinate of the title acid.
Yield: 152 mg (82 %). M.p. 102 -121°C (amorphous).
¹H NMR spectrum (250 MHz, DMSO-d₆, δ_{H}): 7.28 and 7.25 (d, 1 H); 7.19 and 7.16 (d, 1 H); 7.07 (m, 2 H); 6.84 (m, 1H); 6.71 (m, 1 H); 6.64 (bd, 1 H); 6.29 (bt, 1 H); 4.46 and 4.44 and 4.40 and 4.38 (s, 4 H); 4.25 (bs, 2 H); 3.55 (bd, 2 H); 3.17 (bs, 1 H); 3.05 and 3.03 and 2.88 and 2.86 (s, 6 H); 2.73 (bt, ~2 H); 2.09 (s, 3 H); 2.09 and 2.07 and 2.03 and 2.02 (s, 6 H); 1.70-1.15 (m, ∼6 H).

### Example 17

### [4-[3,3-Bis[4-[3,3,3-trimethylpropynyl]phenyl]allylsulfanyl]-2-methylphenoxy]acetic acid

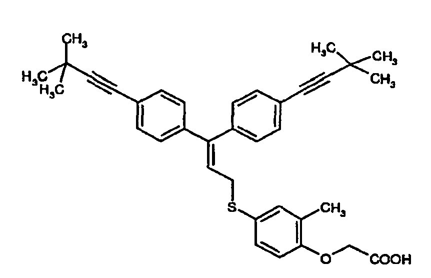

### General procedure (E)

### Step B:

Copper(I) iodide (28 mg, 0.147 mmol) and tetrakis(triphenylphosphine)palladium (72 mg, 0.062 mmol) were added to a degassed solution of ethyl [4-[3,3-bis(4-iodophenyl)allylsulfanyl]-2-methylphenoxy]acetate (700 mg, 1.04 mmol) and 3,3-dimethyl-1-butyne (1.4 mL, 11.5 mmol) in anhydrous triethylamine (25 mL) and tetrahydrofuran (15 mL). The resulting mixture was stirred at 50 °C for 0.5 h in atmosphere of nitrogen. The dark suspension was evaporated *in vacuo,* ethyl acetate (50 mL) was added to the residue and the mixture was washed with water (2 x 50 mL) and brine (2 x 50 mL). The organic solution was dried with anhydrous magnesium sulfate and evaporated *in vacuo.* The residue was purified by flash column chromatography (silica gel Fluka 60, hexane/ethyl acetate 30:1) yielding ethyl [4-[3,3-bis[4-[3,3,3-trimethylpropynyl]phenyl]allylsulfanyl]-2-methylphenoxy]acetate as an yellow crystalline solid.
Yield: 500 mg (83 %).
R_{F} (SiO₂, hexane/ethyl acetate 9:1) 0.40.
¹H NMR spectrum (300 MHz, CDCl₃, δ_{H}): 7.33-6.54 (m, ∼11 H); 6.10 (t, J=7.9 Hz, 1 H); 4.60 (s, 2 H); 4.23 (q, J=7.1 Hz, 2 H); 3.47 (d, J=7.9 Hz, 2 H); 2.20 (s, 3 H); 1.33 (s, ∼9 H); 1.30 (s, ∼9 H); 1.27 (t, J=7.1 Hz, ∼3 H).

### General procedure (D)

### Step A:

In atmosphere of nitrogen, lithium hydroxide monohydrate (42 mg, 1.0 mmol) was added to an ice-water cooled solution of the above ester (388 mg, 0.670 mmol) in a mixture tetrahydrofuran/methanol/water (5:1:1; 7 mL) and the resulting solution was stirred for 40 min under cooling. Acetic acid (0.058 mL, 1.01 mmol) was added dropwise and the solution was stirred for 10 min and subsequently diluted with chloroform (40 mL). The mixture was washed with water (2 x 40 mL) and brine (2 x 40 mL), dried with anhydrous magnesium sulfate and evaporated *in vacuo* yielding sufficiently pure title acid.
Yield: 310 mg (84 %).
R_{F} (SiO₂, ethyl acetate/methanol 7:3) 0.30.
¹H NMR spectrum (300 MHz, CDCl₃, δ_{H}): 7.33-6.57 (m, ∼11 H); 6.10 (t, J=7.9 Hz, 1 H); 4.65 (s, 2 H); 3.49 (d, J=7.9 Hz, 2 H); 2.20 (s, 3 H); 1.33 (s, 9 H); 1.29 (s, 9 H).

A solution of L-lysine (79 mg, 0.540 mmol) in distilled water (0.5 mL) was added to a solution of the above acid (302 mg, 0.548 mmol) in dry tetrahydrofuran (5 mL). The resulting mixture was stirred for 90 min, evaporated *in vacuo* and the residue was evaporated with absolute ethanol (3 x 10 mL). The residue was triturated with anhydrous ether (3 x 10 mL) yielding L-lysinate of the title acid.
Yield: 318 mg (83 %). M.p. 155 -164°C (amorphous).
¹H NMR spectrum (300 MHz, DMSO-d₆, δ_{H}): 7.31-6.58 (m, ∼11 H); 6.13 (t, J=7.9 Hz, 1 H); 4.22 (s, 2 H); ∼3.33 (d, ∼2H (overlapped)); 3.11 (m, ∼1 H); 2.71 (m, 1.7 H); 2.05 (s, 3 H); 1.70-1.42 (m, 4 H); 1.28 (s, ∼9 H); 1.25 (s, ∼9 H).

### Example 18

### [4-[3,3-Bis[4-[3-(imidazol-1-yl)propynyl]phenyl]allylsulfanyl]-2-methylphenoxy]acetic acid

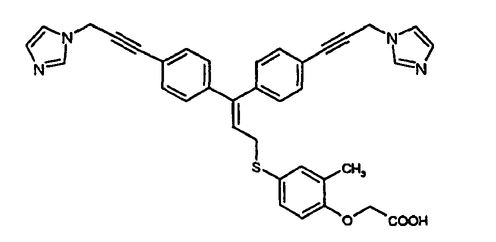

### General procedure (E)

### Step B:

Copper(I) iodide (22 mg, 0.116 mmol) and tetrakis(triphenylphosphine)palladium (65 mg, 0.056 mmol) were added to a degassed solution of ethyl [4-[3,3-bis(4-iodophenyl)allylsulfanyl]-2-methylphenoxy]acetate (624 mg, 0.931 mmol) and N-propargylimidazole (396 mg, 3.73 mmol; prepared as described in Tetrahedron 2001, 57, 607) in dry tetrahydrofuran (7 mL) - dry triethylamine (14 mL) mixture. In atmosphere of nitrogen, the resulting mixture was heated at 65 °C for 6 h, cooled down and subsequently evaporated *in vacuo.* The residue was purified by double flash column chromatography (silica gel Fluka 60, dichloromethane/- methanol 95:5) yielding ethyl [4-[3,3-bis[4-[3-(imidazol-1-yl)propynyl]phenyl]allylsulfanyl]-2-methylphenoxy]acetate as an yellow oil.
Yeld: 412 mg (71 %).
M.p. ---- ° C (oil).
R_{F} (SiO₂ dichloromethane/methanol 95:5, double elution) 0.25.
¹H NMR spectrum (300 MHz, CDCl₃, δ_{H}): 7.66 (s, 1 H); 7.63 (s, 1 H); 7.36 (d, J=8.0 Hz, 2 H); 7.32 (d, J=8.3 Hz, 2 H); 7.08 (m, 8 H); 6.85 (d, J=8.0 Hz, 2 H); 6.56 (bd, J=8.8 Hz, 1 H); 6.15 (t, J=8.0 Hz, 1 H); 4.97 (s, 2 H); 4.94 (s, 2 H); 4.60 (s, 2 H); 4.22 (q, J=7.2 Hz, 2 H); 3.45 (d, J=8.0 Hz, 2 H); 2.18 (s, 3 H); 1.24 (t, J=7.2 Hz, ∼3 H).

### General procedure (D)

### Step A:

In atmosphere of nitrogen, a solution of lithium hydroxide monohydrate (33 mg, 0.786 mmol) in distilled water (1 mL) was added to an ice-water cooled solution of the above ester (392 mg, 0.625 mmol) in tetrahydrofuran/methanol (5:1; 6 mL) mixture and the resulting solution was stirred for 45 min under cooling. The reaction mixture was neutralized with acetic acid (0.045 mL, 0.787 mmol), stirred for 15 min and diluted with dichloromethane (75 mL). The mixture was washed with a diluted solution of sodium chloride (3 x 20 mL) and brine (2 x 15 mL). The organic layer was dried with anhydrous magnesium sulfate and evaporated *in vacuo* yielding sufficiently pure crude title acid.
Yield: 329 mg (88 %). M.p. --- (oil).
R_{F} (SiO₂, dichloromethane/methanol 85:15) 0.05.
¹H NMR spectrum (300 MHz, CDCl₃, δ_{H}): 8.46 (s, 1 H); 7.72 (s, 1 H); 7.41 (d, J=8.0 Hz, 2 H); 7.31 (d, J=8.3 Hz, 2 H); 7.18 (d, J=2.2 Hz, 1 H); 7.10 (m, 6 H); 7.03 (s, 1 H); 6.60 (d, J=8.5 Hz, 1 H); 6.53 (d, J=8.0 Hz, 2 H); 6.21 (t, J=8.3 Hz, 1 H); 4.94 (s, 2 H); 4.93 (s, 2 H); 4.68 (s, 2 H); 3.33 (d, J=8.3 Hz, 2 H); 2.20 (s, 3 H).

A solution of L-lysine (64 mg, 0.438 mmol) in distilled water (0.5 mL) together with a few drops of methanol were added to a solution of the above acid (286 mg, 0.478 mmol) in dry tetrahydrofuran (5 mL). The resulting solution was stirred for 90 min, evaporated *in vacuo* and the residue was evaporated with absolute ethanol (2 x 5 mL). The residue was triturated with acetonitrile/dichloromethane mixture (1:1, 5 mL) and subsequently with anhydrous ether (3 x 5 mL) yielding L-lysinate of the title acid.
Yield: 260 mg (73 %).
M.p.115-121°C(amorphous).
¹H NMR spectrum (300 MHz, DMSO-d₆, δ_{H}): 7.75 (s, 1 H); 7.72 (s, 1 H); 7.43-7.26 (m, 6 H); 7.09-6.80 (m, 8 H); 6.58 (d, J=8.3 Hz, 1 H); 6.18 (t, J=7.4 Hz, 1 H); 5.18 (s, 2 H); 5.15 (s, 2 H); 4.22 (s, 2 H); 3.41 (d, J=7.4 Hz, 2 H); 3.17 (m, 1H); 2.69 (m, 2 H); 2.03 (s, 3 H), 1.75-1.25 (m, ∼6 H).

### Example 19

### [4-[3,3-Bis[4-[3-(2-oxopyrrolidin-1-yl)propynyl]phenyl]allylsulfanyl]-2-methylphenoxy]acetic acid

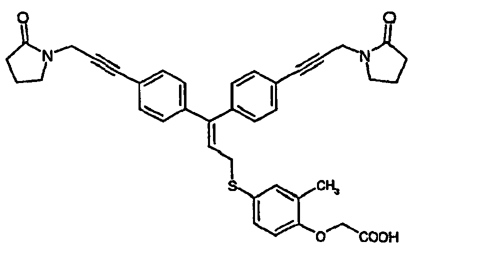

### General procedure (E)

### Step B:

Copper(I) iodide (23 mg, 0.121 mmol) and tetrakis(triphenylphosphine)palladium (67 mg, 0.056 mmol) were added to a degassed solution of ethyl [4-[3,3-bis(4-iodophenyl)-allylsulfanyl]-2-methylphenoxy]acetate (649 mg, 0.968 mmol) and N-propargylpyrrolidinone (476 mg, 3.86 mmol; prepared as described in J. Med. Chem. 1990, 33, 580) in dry tetrahydrofuran (5 mL) - dry triethylamine (15 mL) mixture. In atmosphere of nitrogen, the resulting mixture was heated at 60 °C for 2 h, cooled down and subsequently evaporated in *vacuo.* The residue was purified by flash column chromatography (silica gel Fluka 60, dichloromethane/methanol 98:2) yielding ethyl [4-[3,3-bis[4-[3-(2-oxopyrrolidin-1-yl)propynyl]-phenyl]allylsulfanyl]-2-methylphenoxy]acetate as an yellow oil.
Yield: 504 mg (79 %).
M.p. --- ° C (oil).
R_{F} (SiO₂, dichloromethane/methanol 95:5) 0.35.
¹H NMR spectrum (300 MHz, CDCl₃, δ_{H}): 7.35 (dm, J=8.5 Hz, 2 H); 7.28 (dm, J=8.0 Hz, 2 H); 7.10 (m, 2 H); 7.05 (dm, J=8.5 Hz, 2 H); 6.83 (dm, J=8.3 Hz, 2 H); 6.55 (dm, J=8.8 Hz, 2 H); 6.14 (t, J=8.0 Hz, 1 H); 4.61 (s, 2 H); 4.34 (s, 2 H); 4.31 (s, 2 H); 4.23 (q, J=7.2 Hz, 2 H); 3.56 (m, 4 H); 3.46 (d, J=8.0 Hz, 2 H); 2.43 (m, 4 H); 2.20 (s, 3 H); 2.08 (m, 4 H); 1.26 (t, J=7.2 Hz, 3 H).

### General procedure (D)

### Step A:

In atmosphere of nitrogen, a solution of lithium hydroxide monohydrate (36 mg, 0.858 mmol) in distilled water (1 mL) was added to an ice-water cooled solution of the above ester (417 mg, 0.631 mmol) in tetrahydrofuran/methanol (5:1; 6 mL) mixture and the resulting solution was stirred for 60 min under cooling. The reaction mixture was neutralized with acetic acid (0.049 mL, 0.857 mmol), stirred for 10 min and diluted with dichloromethane (75 mL). The mixture was washed with water (2 x 15 mL) and brine (2 x 15 mL). The organic solution was dried with anhydrous magnesium sulfate and evaporated *in vacuo* yielding sufficiently pure crude title acid.
Yield: 290 mg (73 %).
M.p. --- (oil).
R_{F} (SiO₂, dichloromethane/methanol 85:15) 0.20.
¹H NMR spectrum (300 MHz, CDCl₃, δ_{H}): 7.30 (m, 4 H); 7.09 (m, 3 H); 6.98 (bd, J=7.7 Hz, 1 H); 6.65 (d, J=8.0 Hz, 2 H); 6.52 (d, J=8.5 Hz, 1 H); 6.16 (t, J=8.3 Hz, 1 H); 4.64 (s, 2 H); 4.31 (s, 2 H); 4.30 (s, 2 H); 3.58 (t, J=7.4 Hz, 2 H); 3.54 (t, J=6.9 Hz, 2 H); 3.41 (d, J=8.0 Hz, 2 H); 2.48 (t, J=8.3 Hz, 2 H); 2.43 (t, J=8.3 Hz, 2 H); 2.19 (s, 3 H); 2.09 (m, 4 H).

A solution of L-lysine (58 mg, 0.397 mmol) in distilled water (0.7 mL) together with a few drops of methanol were added to a solution of the above acid (270 mg, 0.427 mmol) in dry tetrahydrofuran (7 mL). The resulting solution was stirred for 90 min, evaporated *in vacuo* and the residue was evaporated with absolute ethanol (2 x 5 mL). The residue was triturated with anhydrous ether (5 x 5 mL) yielding L-lysinate of the title acid.
Yield: 290 mg (87 %).
M.p. 114 -124°C (amorphous).
¹H NMR spectrum (300 MHz, DMSO-d₆, δ_{H}): 7.41 (d, J=8.1 Hz, 2 H); 7.35 (d, J=8.4 Hz, 2 H); 7.05 (m, 4 H); 6.83 (d, J=7.7 Hz, 2 H); 6.60 (d, J=8.8 Hz, 1 H); 6.18 (t, J=7.7 Hz, 1 H); 4.27 (m, 6 H); 3.42 (m, ~6 H); 3.17 (m, 1 H); 2.70 (m, 2 H); 2.25 (m, 4 H); 2.04 (s, 3 H); 1.96 (m, 4 H); 1.74-1.23 (m, 6 H).

### Example 20

### [4-[3,3-Bis(4-methylcarbamoylethynylphenyl)allylsulfanyl]-2-methylphenoxy]acetic acid

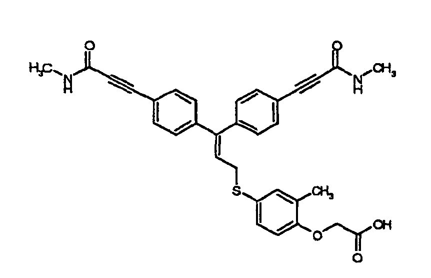

### General procedure (E)

### Step B:

Copper(I) iodide (58 mg, 0.305 mmol) and tetrakis(triphenylphosphine)palladium (151 mg, 0.131 mmol) were added to a degassed solution of ethyl [4-[3,3-bis(4-iodophenyl)allylsulfanyl]-2-methylphenoxy]acetate (1.2 g, 1.79 mmol) and propynoic acid methylamide (542 mg, 6.52 mmol) in anhydrous triethylamine (50 mL) and tetrahydrofuran (28 mL). In atmosphere of nitrogen, the resulting mixture was stirred at 70 °C for 5 h. The dark suspension was evaporated *in vacuo,* dichloromethane (50 mL) was added to the residue and the mixture was washed with water (2 x 50 mL) and brine (2 x 40 mL). The organic solution was dried with anhydrous magnesium sulfate and evaporated *in vacuo.* The residue was purified by flash column chromatography (silica gel Fluka 60, hexane/ethyl acetate 1:6) yielding ethyl [4-[3,3-bis(4-methylcarbamoylethynylphenyl)allylsulfanyl]-2-methylphenoxy]acetate as an yellow oil.
Yield: 301 mg (29 %).
R_{F} (SiO₂, ethyl acetate) 0.45.
¹H NMR spectrum (300 MHz, CDCl₃, δ_{H}): 7.45-6.53 (m, ∼11 H); 6.20 (t, J=8.0 Hz, 1 H); 5.96 (m, 1 H); 5.88 (m, 1 H); 4.62 (s, 2 H); 4.23 (q, J=7.1 Hz, 2 H); 3.45 (d, J=8.0 Hz, 2 H); 2.93 (m, -6 H); 2.19 (s, 3 H); 1.27 (t, ∼3 H).

### General procedure (D)

### Step A:

In atmosphere of nitrogen, lithium hydroxide monohydrate (32 mg, 0.763 mmol) was added to an ice-water cooled solution of the above ester (291 mg, 0.501 mmol) in a mixture tetrahydrofuran/methanol/water (5:1:1; 7 mL) and the resulting solution was stirred for 60 min under cooling. Acetic acid (0.043 mL, 0.752 mmol) was added dropwise, the solution was stirred for 10 min and subsequently diluted with chloroform (40 mL). The mixture was washed with water (2 x 40 mL) and brine (2 x 40 mL), dried with anhydrous magnesium sulfate and evaporated *in vacuo* yielding sufficiently pure title acid.
Yield: 210 mg (76 %).
R_{F} (SiO₂, ethyl acetate/methanol 7:3) 0.15.
¹H NMR spectrum (300 MHz, CDCl₃, δ_{H}): 7.39-6.47 (m, ∼11 H); 6.32 (m, 1 H); 6.21 (t, J=7.9 Hz, 1 H); 6.10 (m, 1 H); 4.65 (s, 2 H); 3.40 (d, J=8.2 Hz, ~2 H); 2.93 (m, ~6 H); 2.18 (s, 3 H). _

A solution of L-lysine (41 mg, 0.281 mmol) in distilled water (0.5 mL) was added to a solution of the above acid (158 mg, 0.286 mmol) in dry tetrahydrofuran (5 mL). The resulting solution was stirred for 60 min, evaporated *in vacuo* and the residue was evaporated with absolute ethanol (3 x 10 mL). The residue was triturated with anhydrous ether (3 x 10 mL) yielding L-lysinate of the title acid.
Yield: 173 mg (87 %).
M.p. 150-159 °C (amorphous).
¹H NMR spectrum (300 MHz, DMSO-d₆, δ_{H}): 8.99 (m, 1 H); 8.74 (m, 1 H); 7.51-6.56 (m, ∼11 H); 6.26 (t, J=8.0 Hz, 1 H); 4.28 (s, 2 H); 3.36 (d, ∼2 H), 3.16 (m, 1 H); 2.69 (m, ~2 H); 2.64 (m, ~6 H); 2.04 (s, 3 H); 1.74-1.24 (m, 6 H).

### PHARMACOLOGICAL METHODS

### In vitro PPARalpha, PPARgamma and PPARdelta activation activity

The PPAR transient transactivation assays are based on transient transfection into human HEK293 cells of two plasmids encoding a chimeric test protein and a reporter protein respectively. The chimeric test protein is a fusion of the DNA binding domain (DBD) from the yeast GAL4 transcription factor to the ligand binding domain (LBD) of the human PPAR proteins. The PPAR-LBD moiety harbored in addition to the ligand binding pocket also the native activation domain (activating function 2 = AF2) allowing the fusion protein to function as a PPAR ligand dependent transcription factor. The GAL4 DBD will direct the chimeric protein to bind only to Gal4 enhancers (of which none existed in HEK293 cells). The reporter plasmid contained a Gal4 enhancer driving the expression of the firefly luciferase protein. After transfection, HEK293 cells expressed the GAL4-DBD-PPAR-LBD fusion protein. The fusion protein will in turn bind to the Gal4 enhancer controlling the luciferase expression, and do nothing in the absence of ligand. Upon addition to the cells of a PPAR ligand luciferase protein will be produced in amounts corresponding to the activation of the PPAR protein. The amount of luciferase protein is measured by light emission after addition of the appropriate substrate.

### CELL CULTURE AND TRANSFECTION

HEK293 cells were grown in DMEM + 10% FCS. Cells were seeded in 96-well plates the day before transfection to give a confluency of 50-80 % at transfection. A total of 0,8 µg DNA containing 0,64 µg pM1 α/γLBD, 0,1 µg pCMVβGal, 0,08 µg pGL2(Gal4)ₛ and 0,02 µg pADVANTAGE was transfected per well using FuGene transfection reagent according to the manufacturers instructions (Roche). Cells were allowed to express protein for 48 h followed by addition of compound.
Plasmids: Human PPAR α, γ and δ was obtained by PCR amplification using cDNA synthesized by reverse transcription of mRNA from human liver, adipose tissue and plancenta respectively. Amplified cDNAs were cloned into pCR2.1 and sequenced. The ligand binding domain (LBD) of each PPAR isoform was generated by PCR (PPARα: aa 167 - C-terminus; PPARγ. aa 165 - C-terminus; PPARδ: aa 128 - C-terminus) and fused to the DNA binding domain (DBD) of the yeast transcription factor GAL4 by subcloning fragments in frame into the vector pM1 (Sadowski et al. (1992), Gene 118, 137) generating the plasmids pM1 αLBD, pM1γLBD and pM1δ. Ensuing fusions were verified by sequencing. The reporter was constructed by inserting an oligonucleotide encoding five repeats of the GAL4 recognition sequence (5 x CGGAGTACTGTCCTCCG(AG)) (Webster et al. (1988), Nucleic Acids Res. 16, 8192) into the vector pGL2 promotor (Promega) generating the plasmid pGL2(GAL4)₅. pCMVβGal was purchased from Clontech and pADVANTAGE was purchased from Promega.

### IN VITRO TRANSACTIVATION ASSAY

Compounds: All compounds were dissolved in DMSO and diluted 1:1000 upon addition to the cells. Compounds were tested in quadruple in concentrations ranging from 0.001 to 300 µM. Cells were treated with compound for 24 h followed by luciferase assay. Each compound was tested in at least two separate experiments.

Luciferase assay: Medium including test compound was aspirated and 100 µl PBS incl. 1 mM Mg++ and Ca++ was added to each well. The luciferase assay was performed using the LucLite kit according to the manufacturers instructions (Packard Instruments). Light emission was quantified by counting on a Packard LumiCounter. To measure β-galactosidase activity 25 µl supernatant from each transfection lysate was transferred to a new microplate. β-galactosidase assays were performed in the microwell plates using a kit from Promega and read in a Labsystems Ascent Multiscan reader. The β-galactosidase data were used to normalize (transfection efficiency, cell growth etc.) the luciferase data.

### STATISTICAL METHODS

The activity of a compound is calculated as fold induction compared to an untreated sample. For each compound the efficacy (maximal activity) is given as a relative activity compared to Wy14,643 for PPARα, Rosiglitazone for PPARγ and Carbacyclin for PPARδ. The EC50 is the concentration giving 50% of maximal observed activity. EC50 values were calculated via non-linear regression using GraphPad PRISM 3.02 (GraphPad Software, San Diego, Ca). The results were expressed as means ± SD.

## Claims

1. A compound of the general formula (I): 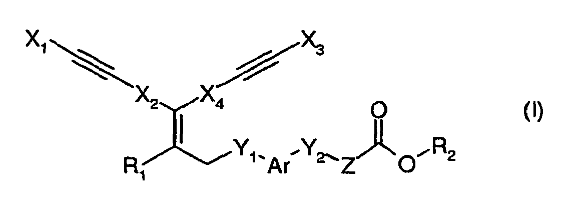 wherein X₁ is aryl or heteroaryl each of which is optionally substituted with one or more substituents selected from
• halogen, hydroxy, cyano, amino or carboxy; or
• C₁₋₆-alkyl, C₃₋₆-cycloalkyl, C₂₋₆-alkenyl, C₂₋₆-alkynyl, aryl, aralkyl, heteroaryl, heteroaralkyl, C₁₋₆-alkoxy, C₃₋₆-cycloalkoxy, aryloxy, aralkoxy, heteroaralkoxy, C₁₋₆-alkylthio, arylthio, C₃₋₆-cycloalkylthio, C₁₋₆-alkylcarbonyl, arylcarbonyl, C₁₋₆-alkylsulfonyl, arylsulfonyl, C₁₋₆-alkylamido, arylamido, C₁₋₆-alkylaminocarbonyl, C₁₋₆-alkylamino, C₁₋₆-dialkylamino or C₃₋₆-cycloalkylamino each of which is optionally substituted with one or more halogens; or
X₁ is C₁₋₆-alkyl, C₃₋₆-cycloalkyl, C₂₋₆-alkenyl, carbamoyl or C₃₋₆-cycloalkyl-C₁₋₆-alkyl each of which is optionally substituted with one or more substituents selected from
• halogen, hydroxy, cyano, amino or carboxy; or
• C₁₋₆-alkyl, C₃₋₆-cycloalkyl, C₂₋₆-alkenyl, C₂₋₆-alkynyl, C₃₋₆-cycloalkyl-C₁₋₆-alkyl, aryl, aralkyl, heteroaryl, heteroaralkyl, heterocyclyl, C₁₋₆-alkoxy, C₃₋₆-cycloalkoxy, C₃₋₆-cycloalkyl-C₁₋₆-alkoxy, aryloxy, heteroaryloxy, aralkoxy, heteroaralkoxy, C₁₋₆-alkylthio, C₃₋₆-cycloalkylthio, C₃₋₆-cycloalkyl-C₁₋₆-alkylthio,arylthio, heteroarylthio, aryl-C₁₋₆-alkylthio, heteroaryl-C₁₋₆-alkylthio, C₁₋₆-alkylcarbonyl, C₃₋₆-cycloalkylcarbonyl, C₃₋₆-cycloalkyl- C₁₋₆-alkyl-carbonyl, arylcarbonyl, heteroarylcarbonyl, C₁₋₆-alkylsulfonyl, C₃₋₆-cycloalkylsulfonyl, C₃₋₆-cycloalkyl-C₁₋₆-alkylsulfonyl, arylsulfonyl, heteroarylsulfonyl, C₁₋₆-alkylsulfamoyl, di-(C₁₋₆-alkyl)sulfamoyl, C₁₋₆-alkoxycarbonyl, C₃₋₆-cycloalkoxycarbonyl, C₃₋₆-cycloalkyl-C₁₋₆-alkoxycarbonyl, amino-C₁₋₆-alkyl, C₁₋₆-alkylamino-C₁₋₆-alkyl, di-(C₁₋₆-alkyl)amino-C₁₋₆-alkyl, C₁₋₆-alkylamido, C₃₋₆-cycloalkylamido, C₃₋₆-cycloalkyl-C₁₋₆-alkylamido, arylamido, C₁₋₆-alkylaminocarbonyl, C₃₋₆-cycloalkylaminocarbonyl, C₃₋₆-cycloalkyl-C₁₋₆-alkylaminocarbonyl, di-(C₁₋₆-alkyl)aminocarbonyl, di-(C₃₋₆-cycloalkyl-C₁₋₆-alkyl)aminocarbonyl, C₁₋₆-alkylamino, C₃₋₆-cycloalkylamino, C₃₋₆-cycloalkyl-C₁₋₆-alkylamino, di-(C₁₋₆-alkyl)amino, di-(C₃₋₆-cycloalkyl)amino or di-(C₃₋₆-cycloalkyl-C₁₋₆-alkyl)amino each of which is optionally substituted with one or more of halogen, cyano, hydroxy, acetyl or oxo; and
X₂ is arylene or heteroarylene each of which is optionally substituted with one or more substituents selected from
• halogen, hydroxy, cyano, amino or carboxy; or
• C₁₋₆-alkyl, C₃₋₆-cycloalkyl, C₂₋₆-alkenyl, C₂₋₆-alkynyl, C₁₋₆-alkoxy, C₃₋₆-cycloalkoxy, C₁₋₆-alkylthio, C₃₋₆-cycloalkylthio, C₁₋₆-alkylamino, C₁₋₆-dialkylamino or C₃₋₆-cycloalkylamino each of which is optionally substituted with one or more halogens; and
X₃ is aryl or heteroaryl each of which is optionally substituted with one or more substituents selected from
• halogen, hydroxy, cyano, amino or carboxy; or
• C₁₋₆-alkyl, C₃₋₆-cycloalkyl, C₂₋₆-alkenyl, C₂₋₆-alkynyl, aryl, aralkyl, heteroaryl, heteroaralkyl, C₁₋₆-alkoxy, C₃₋₆-cycloalkoxy, aryloxy, aralkoxy, heteroaralkoxy, C₁₋₆-alkylthio, arylthio, C₃₋₆-cycloalkylthio, C₁₋₆-alkylcarbonyl, arylcarbonyl, C₁₋₆-alkylsulfonyl, arylsulfonyl, C₁₋₆-alkylamido, arylamido, C₁₋₆-alkylaminocarbonyl, C₁₋₆-alkylamino, C₁₋₆-dialkylamino or C₃₋₆-cycloalkylamino each of which is optionally substituted with one or more halogens; or
X₃ is C₁₋₆-alkyl, C₃₋₆-cycloalkyl, C₂₋₆-alkenyl, carbamoyl or C₃₋₆-cycloalkyl-C₁₋₆-alkyl each of which is optionally substituted with one or more substituents selected from
• halogen, hydroxy, cyano, amino or carboxy; or
• C₁-₆-alkyl, C₃₋₆-cycloalkyl, C₂₋₆-alkenyl, C₂₋₆-alkynyl, C₃₋₆-cycloalkyl-C₁₋₆-alkyl, aryl, aralkyl, heteroaryl, heteroaralkyl, heterocyclyl, C₁₋₆-alkoxy, C₃₋₆-cycloalkoxy, C₃₋₆-cycloalkyl-C₁₋₆-alkoxy, aryloxy, heteroaryloxy, aralkoxy, heteroaralkoxy, C₁₋₆-alkylthio, C₃₋₆-cycloalkylthio, C₃₋₆-cycloalkyl-C₁₋₆-alkylthio,arylthio, heteroarylthio, aryl-C₁₋₆-alkylthio, heteroaryl-C₁₋₆-alkylthio, C₁₋₆-alkylcarbonyl, C₃₋₆-cycloalkylcarbonyl, C₃₋₆-cycloalkyl- C₁₋₆-alkyl-carbonyl, arylcarbonyl, heteroarylcarbonyl, C₁₋₆-alkylsulfonyl, C₃₋₆-cycloalkylsulfonyl, C₃₋₆-cycloalkyl-C₁₋₆-alkylsulfonyl, arylsulfonyl, heteroarylsulfonyl, C₁₋₆-alkylsulfamoyl, di-(C₁₋₆-alkyl)sulfamoyl, C₁₋₆-alkoxycarbonyl, C₃₋₆-cycloalkoxycarbonyl, C₃₋₆-cycloalkyl-C₁₋₆-alkoxycarbonyl, amino-C₁₋₆-alkyl, C₁₋₆-alkylamino-C₁₋₆-alkyl, di-(C₁₋₆-alkyl)amino-C₁₋₆-alkyl, C₁₋₆-alkylamido, C₃₋₆-cycloalkylamido, C₃₋₆-cycloalkyl-C₁₋₆-alkylamido, arylamido, C₁₋₆-alkylaminocarbonyl, C₃₋₆-cycloalkylaminocarbonyl, C₃₋₆-cycloalkyl-C₁₋₆-alkylaminocarbonyl, di-(C₁₋₆-alkyl)aminocarbonyl, di-(C₃₋₆-cycloalkyl-C₁₋₆-alkyl)aminocarbonyl, C₁₋₆-alkylamino, C₃₋₆-cycloalkylamino, C₃₋₆-cycloalkyl-C₁₋₆-alkylamino, di-(C₁₋₆-alkyl)amino, di-(C₃₋₆-cycloalkyl)amino or di-(C₃₋₆-cycloalkyl-C₁₋₆-alkyl)amino each of which is optionally substituted with one or more of halogen, cyano, hydroxyo, acetyl or oxo; and
X₄ is arylene or heteroarylene each of which is optionally substituted with one or more substituents selected from
• halogen, hydroxy, cyano, amino or carboxy; or
• C₁₋₆-alkyl, C₃₋₆-cycloalkyl, C₂₋₆-alkenyl, C₂₋₆-alkynyl, C₁₋₆-alkoxy, C₃₋₆-cycloalkoxy, C₁₋₆-alkylthio, C₃₋₆-cycloalkylthio, C₁₋₆-alkylamino, C₁₋₆-dialkylamino or C₃₋₆-cycloalkylamino each of which is optionally substituted with one or more halogens; and
Ar is arylene which is optionally substituted with one or more substituents selected from
• halogen, hydroxy or cyano; or
• C₁₋₆-alkyl, C₃₋₆-cycloalkyl, C₂₋₆-alkenyl, C₂₋₆-alkynyl, aryl, heteroaryl, aralkyl, heteroaralkyl, C₁₋₆-alkoxy, C₃₋₆-cycloalkoxy, aryloxy, aralkoxy, heteroaralkoxy, C₁₋₆-alkylthio, arylthio or C₃₋₆-cycloalkylthio each of which is optionally substituted with one or more halogens; or
• two of the substituents when placed in adjacent positions together with the atoms to
which they are attached may form a five to eight member ring; and
Y₁ is O or S; and
Y₂ is O or S; and
Z is -(CH₂)ₙ- wherein n is 1, 2 or 3; and
R₁ is hydrogen, halogen or a substituent selected from
• C₁₋₆-alkyl, C₃₋₆-cycloalkyl, C₂₋₆-alkenyl, C₂₋₆-alkynyl, aralkyl, heteroaralkyl, C₁₋₆-alkoxy, C₃₋₆-cycloalkoxy, aryloxy, aralkoxy, heteroaralkoxy, C₁₋₆-alkylthio, arylthio or C₃₋₆-cycloalkylthio each of which is optionally substituted with one or more halogens; and R₂ is hydrogen, C₁₋₆-alkyl, C₃₋₆-cycloalkyl, C₂₋₆-alkenyl, C₂₋₆-alkynyl, C₄₋₆-alkenynyl or aryl; or
a pharmaceutically acceptable salt thereof, or a pharmaceutically acceptable solvate thereof, or any tautomeric forms, stereoisomers, mixture of stereoisomers including a racemic mixture, or polymorphs.

2. A compound according to claim 1, wherein X₁ is aryl or heteroaryl each of which is optionally substituted with one or more substituents selected from
• halogen, hydroxy, cyano, amino or carboxy; or
• C₁₋₆-alkyl, C₃₋₆-cycloalkyl, C₂₋₆-alkenyl, C₂₋₆-alkynyl, aryl, aralkyl, heteroaryl, heteroaralkyl, C₁₋₆-alkoxy, C₃₋₆-cycloalkoxy, aryloxy, aralkoxy, heteroaralkoxy, C₁₋₆-alkylthio, arylthio, C₃₋₆-cycloalkylthio, C₁₋₆-alkylcarbonyl, arylcarbonyl, C₁₋₆-alkylsulfonyl, arylsulfonyl, C₁₋₆-alkylamido, arylamido, C₁₋₆-alkylaminocarbonyl, C₁₋₆-alkylamino, C₁₋₆-dialkylamino or C₃₋₆-cycloalkylamino each of which is optionally substituted with one or more halogens; and
X₂ is arylene or heteroarylene each of which is optionally substituted with one or more substituents selected from
• halogen, hydroxy, cyano, amino or carboxy; or
• C₁₋₆-alkyl, C₃₋₆-cycloalkyl, C₂₋₆-alkenyl, C₂₋₆-alkynyl, C₁₋₆-alkoxy, C₃₋₆-cycloalkoxy, C₁₋₆-alkylthio, C₃₋₆-cycloalkylthio, C₁₋₆-alkylamino, C₁₋₆-dialkylamino or C₃₋₆-cycloalkylamino each of which is optionally substituted with one or more halogens; and
X₃ is aryl or heteroaryl each of which is optionally substituted with one or more substituents selected from
• halogen, hydroxy, cyano, amino or carboxy; or
• C₁₋₆-alkyl, C₃₋₆cycloalkyl, C₂₋₆-alkenyl, C₂₋₆-alkynyl, aryl, aralkyl, heteroaryl, heteroaralkyl, C₁₋₆-alkoxy, C₃₋₆-cycloalkoxy, aryloxy, aralkoxy, heteroaralkoxy, C₁₋₆-alkylthio, arylthio, C₃₋₆-cycloalkylthio, C₁₋₆-alkylcarbonyl, arylcarbonyl, C₁₋₆-alkylsulfonyl, arylsulfonyl, C₁₋₆-alkylamido, arylamido, C₁₋₆-alkylaminocarbonyl, C₁₋₆-alkylamino, C₁₋₆-dialkylamino or C₃₋₆-cycloalkylamino each of which is optionally substituted with one or more halogens; and
X₄ is arylene or heteroarylene each of which is optionally substituted with one or more substituents selected from
• halogen, hydroxy, cyano, amino or carboxy; or
• C₁₋₆-alkyl, C₃₋₆-cycloalkyl, C₂₋₆-alkenyl, C₂₋₆-alkynyl, C₁₋₆-alkoxy, C₃₋₆-cycloalkoxy, C₁₋₆-alkylthio, C₃₋₆-cycloalkylthio, C₁₋₆-alkylamino, C₁₋₆-dialkylamino or C₃₋₆-cycloalkylamino each of which is optionally substituted with one or more halogens; and
Ar is arylene which is optionally substituted with one or more substituents selected from
• halogen, hydroxy or cyano; or
• C₁₋₆-alkyl, C₃₋₆-cycloalkyl, C₂₋₆-alkenyl, C₂₋₆-alkynyl, aryl, heteroaryl, aralkyl, heteroaralkyl, C₁₋₆-alkoxy, C₃₋₆-cycloalkoxy, aryloxy, aralkoxy, heteroaralkoxy, C₁₋₆-alkylthio, arylthio or C₃₋₆-cycloalkylthio each of which is optionally substituted with one or more halogens; or
• two of the substituents when placed in adjacent positions together with the atoms to
which they are attached may form a five to eight member ring; and
Y₁ is O or S; and
Y₂ is O or S; and
Z is -(CH₂)ₙ- wherein n is 1, 2 or 3; and
R₁ is hydrogen, halogen or a substituent selected from
• C₁₋₆-alkyl, C₃₋₆-cycloalkyl, C₂₋₆-alkenyl, C₂₋₆-alkynyl, aralkyl, heteroaralkyl, C₁₋₆-alkoxy, C₃₋₆-cycloalkoxy, aryloxy, aralkoxy, heteroaralkoxy, C₁₋₆-alkylthio, arylthio or C₃₋₆-cycloalkylthio each of which is optionally substituted with one or more halogens; and
R₂ is hydrogen, C₁₋₆-alkyl, C₃₋₆-cydoalkyl, C₂₋₆-alkenyl, C₂₋₆-alkynyl, C₄₋₆-alkenynyl or aryl; or
a pharmaceutically acceptable salt thereof, or a pharmaceutically acceptable solvate thereof, or any tautomeric forms, stereoisomers, mixture of stereoisomers including a racemic mixture, or polymorphs.

3. A compound according to claim 1 or 2, wherein X₁ is aryl optionally substituted with one or more substituents selected from
• halogen; or
• C₁₋₆-alkyl, aryl, C₁₋₆-alkoxy or C₁₋₆-alkylsulfonyl each of which is optionally substituted with one or more halogens.

4. A compound according to claim 3, wherein X₁ is aryl optionally substituted with one or more substituents selected from
• halogen; or
• C₁₋₆-alkyl optionally substituted with one or more halogens.

5. A compound according to claim 3, wherein X₁ is phenyl optionally substituted with one or more substituents selected from
• halogen; or
• C₁₋₆-alkyl, aryl, C₁₋₆-alkoxy or C₁₋₆-alkylsulfonyl each of which is optionally substituted with one or more halogens.

6. A compound according to claim 4 or 5, wherein X₁ is phenyl optionally substituted with one or more substituents selected from
• halogen; or
• C₁₋₆-alkyl optionally substituted with one or more halogens.

7. A compound according to any one of the preceding claims, wherein X₁ is phenyl.

8. A compound according to claim 1 or 2, wherein X₁ is heteroaryl optionally substituted with one or more substituents selected from
• halogen; or
• C₁₋₆-alkyl, aryl, C₁₋₆-alkoxy or C₁₋₆-alkylsulfonyl each of which is optionally substituted with one or more halogens.

9. A compound according to claim 8, wherein X₁ is heteroaryl optionally substituted with one or more substituents selected from
• halogen; or
• C₁₋₆-alkyl optionally substituted with one or more halogens.

10. A compound according to claim 8 or 9 wherein X₁ is pyrrolyl, pyridyl, furyl or thienyl, each of which is optionally substituted with one or more of halogens or C₁₋₆-alkyl, which is optionally substituted with one or more halogens.

11. A compound according to claim 10, wherein X₁ is furyl or thienyl optionally substituted with one or more halogens.

12. A compound according to claim 10, wherein X₁ is pyrrolyl or pyridyl, each of which is optionally substituted with one or more of C₁₋₆-alkyl.

13. A compound according to claim 1, wherein X₁ is C₁₋₆-alkyl or carbamoyl optionally substituted with one or more substituents selected from
• halogen, hydroxy, cyano, amino or carboxy; or
• C₁₋₆-alkyl, C₃₋₆-cycloalkyl, C₂₋₆-alkenyl, C₂₋₆-alkynyl, C₃₋₆-cycloalkyl-C₁₋₆-alkyl, aryl, aralkyl, heteroaryl, heteroaralkyl, heterocyclyl, C₁₋₆-alkoxy, C₃₋₆-cycloalkoxy, C₃₋₆-cycloalkyl-C₁₋₆-alkoxy, aryloxy, heteroaryloxy, aralkoxy, heteroaralkoxy, C₁₋₆-alkylthio, C₃₋₆-cycloalkylthio, C₃₋₆-cycloalkyl-C₁₋₆-alkylthio,arylthio, heteroarylthio, aryl-C₁₋₆-alkylthio, heteroaryl-C₁₋₆-alkylthio, C₁₋₆-alkylcarbonyl, C₃₋₆-cycloalkylcarbonyl, C₃₋₆-cyloalkyl- C₁₋₆-alkyl-carbonyl, arylcarbonyl, heteroarylcarbonyl, C₁₋₆-alkylsulfonyl, C₃₋₆-cycloalkylsulfonyl, C₃₋₆-cycloalkyl-C₁₋₆-alkylsulfonyl, arylsulfonyl, heteroarylsulfonyl, C₁₋₆-alkylsulfamoyl, di-(C₁₋₆-alkyl)sulfamoyl, C₁₋₆-alkoxycarbonyl, C₃₋₆-cycloalkoxycarbonyl, C₃₋₆-cycloalkyl-C₁₋₆-alkoxycarbonyl, amino-C₁₋₆-alkyl, C₁₋₆-alkylamino-C₁₋₆-alkyl, di-(C₁₋₆-alkyl)amino-C₁₋₆-alkyl, C₁₋₆-alkylamido, C₃₋₆-cycloalkylamido, C₃₋₆-cyoalkyl-C₁₋₆-alkylamido, arylamido, C₁₋₆-alkylaminocarbonyl, C₃₋₆-cycloalkylaminocarbonyl, C₃₋₆-cycloalkyl-C₁₋₆-alkylaminocarbonyl, di-(C₁₋₆-alkyl)aminocarbonyl, di-(C₃₋₆-cycloalkyl-C₁₋₆-alkyl)aminocarbonyl, C₁₋₆-alkylamino, C₃₋₆-cycloalkylamino, C₃₋₆-cycloalkyl-C₁₋₆-alkylamino, di-(C₁₋₆-alkyl)amino, di-(C₃₋₆-cycloalkyl)amino or di-(C₃₋₆-cycloalkyl-C₁₋₆-alkyl)amino each of which is optionally substituted with one or more of halogen, cyano, hydroxy, acetyl or oxo.

14. A compound according to claim 13 wherein X₁ is C₁₋₆-alkyl or carbamoyl optionally substituted with one or more substituents selected from
• halogen or hydroxy; or
• C₁₋₆-alkyl, aryl, heteroaryl, heterocyclyl, C₁₋₆-alkoxy, C₁₋₆-alkyfthio, C₃₋₆-cycloalkylthio, C₃₋₆-cycloalkyl-C₁₋₆-alkylthio,arylthio, heteroarylthio, aryl-C₁₋₆-alkylthio, C₁₋₆-alkylcarbonyl, arylcarbonyl, C₁₋₆-alkylsulfonyl, arylsulfonyl, amino-C₁₋₆-alkyl, C₁₋₆-alkylamino-C₁₋₆-alkyl, di-(C₁₋₆-alkyl)amino-C₁₋₆-alkyl, C₁₋₆-alkylamido, arylamido, C₁₋₆-alkylaminocarbonyl, di-(C₁₋₆-alkyl)-aminocarbonyl, C₁₋₆-alkylamino, di-(C₁₋₆-alkyl)amino, each of which is optionally substituted with one or more of halogen, cyano, hydroxy, acetyl or oxo.

15. A compound according to claim 13 or 14 wherein X₁ is C₁₋₆-alkyl optionally substituted with one or more substituents selected from C₁₋₆-alkyl , heteroaryl, heterocyclyl, C₁₋₆-alkylamino, di-(C₁₋₆-alkyl)amino, each of which is optionally substituted with one or more of acetyl or oxo.

16. A compound according to claim13 or 14 wherein X₃ is carbamoyl optionally substituted with acetyl.

17. A compound according any one of the preceding claims, wherein X₂ is arylene optionally substituted with one or more substituents selected from
• halogen or
• C₁₋₆-alkyl optionally substituted with one or more halogens.

18. A compound according to claim 17, wherein X₂ is phenylene optionally substituted with one or more substituents selected from
• halogen or
• C₁₋₆-alkyl optionally substituted with one or more halogens.

19. A compound according to claim 17 or 18, wherein X₂ is phenylene.

20. A compound according to any one of the claims 1-16, wherein X₂ is heteroarylene optionally substituted with one or more substituents selected from
• halogen or
• C₁₋₆-alkyl optionally substituted with one or more halogens.

21. A compound according to any one of the preceding claims, wherein X₃ is aryl optionally ' substituted with one or more substituents selected from
• halogen; or
• C₁₋₆-alkyl, aryl, C₁₋₆-alkoxy or C₁₋₆-alkylsulfonyl each of which is optionally substituted with one or more halogens.

22. A compound according to claim 21, wherein X₃ is aryl optionally substituted with one or more substituents selected from
• halogen; or
• C₁₋₆-alkyl optionally substituted with one or more halogens.

23. A compound according to claim 21, wherein X₃ is phenyl optionally substituted with one or more substituents selected from
• halogen; or
• C₁₋₆-alkyl, aryl, C₁₋₆-alkoxy or C₁₋₆-alkylsulfonyl each of which is optionally substituted with one or more halogens.

24. A compound according to claim 22 or 23, wherein X₃ is phenyl optionally substituted with one or more substituents selected from
• halogen; or
• C₁₋₆-alkyl optionally substituted with one or more halogens.

25. A compound according to any one of the claims 21-24, wherein Xₐ is phenyl.

26. A compound according to any one of the claims 1-20, wherein X₃ is heteroaryl optionally substituted with one or more substituents selected from
• halogen; or
• C₁₋₆-alkyl, aryl, C₁₋₆-alkoxy or C₁₋₆-alkylsulfonyl each of which is optionally substituted with one or more halogens.

27. A compound according to claim 26, wherein X₃ is heteroaryl optionally substituted with one or more substituents selected from
• halogen; or
• C₁₋₆-alkyl optionally substituted with one or more halogens.

28. A compound according to claim 27 wherein X₃ is pyrrolyl, pyridyl, furyl or thienyl, each of which is optional substituted with one or more of halogens or C₁₋₆-alkyl, which is optionally substituted with one or more halogens.

29. A compound according to claim 28, wherein X₃ is furyl or thienyl optionally substituted with one or more halogens.

30. A compound according to claim 28, wherein X₃ is pyrrolyl or pyridyl, each of which is optionally substituted with one or more of C₁₋₆-alkyl.

31. A compound according to claim 1, wherein X₃ is C₁₋₆-alkyl or carbamoyl optionally substituted with one or more substituents selected from
• halogen, hydroxy, cyano, amino or carboxy; or
• C₁₋₆-alkyl, C₃₋₆-cycloalkyl, C₂₋₆-alkenyl, C₂₋₆-alkynyl, C₃₋₆-cycloalkyl-C₁₋₆-alkyl, aryl, aralkyl, heteroaryl, heteroaralkyl, heterocyclyl, C₁₋₆-alkoxy, C₃₋₆-cycloalkoxy, C₃₋₆-cycloalkyl-C₁₋₆-alkoxy, aryloxy, heteroaryloxy, aralkoxy, heteroaralkoxy, C₁₋₆-alkylthio, C₃₋₆-cycloalkylthio, C₃₋₆-cycloalkyl-C₁₋₆-alkylthio,arylthio, heteroarylthio, aryl-C₁₋₆-alkylthio, heteroaryl-C₁₋₆-alkylthio, C₁₋₆-alkylearbonyl, C₃₋₆-cycloalkylcarbonyl, C₃₋₆-cycloalkyl- C₁₋₆-alkyl-carbonyl, arylcarbonyl, heteroarylcarbonyl, C₁₋₆-alkylsulfonyl, C₃₋₆-cycloalkylsulfonyl, C₃₋₆-cycloalkyl-C₁₋₆-alkylsulfonyl, arylsulfonyl, heteroarylsulfonyl, C₁₋₆-alkylsulfamoyl, di-(C₁₋₆-alkyl)sulfamoyl, C₁₋₆-alkoxycarbonyl, C₃₋₆-cycloalkoxycarbonyl, C₃₋₆-cycloalkyl-C₁₋₆-alkoxycarbonyl, amino-C₁₋₆-alkyl, C₁₋₆-alkylamino-C₁₋₆-alkyl, di-(C₁₋₆-alkyl)amino-C₁₋₆-alkyl, C₁₋₆-alkylamido, C₃₋₆-cycloalkylamido, C₃₋₆-cycloalkyl-C₁₋₆-alkylamido, arylamido, C₁₋₆-alkylaminocarbonyl, C₃₋₆-cycloalkylaminocarbonyl, C₃₋₆-cycloalkyl-C₁₋₆-alkylaminocarbonyl, di-(C₁₋₆-alkyl)aminocarbonyl, di-(C₃₋₆-cycloalkyl-C₁₋₆-alkyl)aminocarbonyl, C₁₋₆-alkylamino, C₃₋₆-cycloalkylamino, C₃₋₆-cycloalkyl-C₁₋₆-alkylamino, di-(C₁₋₆-alkyl)amino, di-(C₃₋₆-cycloalkyl)amino or di-(C₃₋₆-cycloalkyl-C₁₋₆-alkyl)amino each of which is optionally substituted with one or more of halogen, cyano, hydroxy, acetyl or oxo.

32. A compound according to claim 31 wherein X₃ is C₁₋₆-alkyl or carbamoyl optionally substituted with one or more substituents selected from
• halogen or hydroxy; or
• C₁₋₆-alkyl, aryl, heteroaryl, heterocyclyl, C₁₋₆-alkoxy, C₁₋₆-alkylthio, C₃₋₆-cycloalkylthio, C₃₋₆-cycloalkyl-C₁₋₆-alkylthio,arylthio, heteroarylthio, aryl-C₁₋₆-alkylthio, C₁₋₆-alkylcarbonyl, arylcarbonyl, C₁₋₆-alkylsulfonyl, arylsulfonyl, amino-C₁₋₆-alkyl, C₁₋₆-alkylamino-C₁₋₆-alkyl, di-(C₁₋₆-alkyl)amino-C₁₋₆-alkyl, C₁₋₆-alkylamido, arylamido, C₁₋₆-alkylaminocarbonyl, di-(C₁₋₆-alkyl)-aminocarbonyl, C₁₋₆-alkylamino, di-(C₁₋₆-alkyl)amino, each of which is optionally substituted with one or more of halogen, cyano, hydroxy, acetyl or oxo.

33. A compound according to claim 31 or 32 wherein X₃ is C₁₋₆-alkyl optionally substituted with one or more substituents selected from C₁₋₆-alkyl, heteroaryl, heterocyclyl, C₁₋₅-alkylamino, di-(C₁₋₆-alkyl)amino, each of which is optionally substituted with one or more of acetyl or oxo.

34. A compound according to claim 31 or 32 wherein X₃ is carbamoyl optionally substituted with acetyl.

35. A compound according to any one of the preceding claims, wherein X₄ is arylene optionally substituted with one or more substituents selected from
• halogen or
• C₁₋₆-alkyl optionally substituted with one or more halogens.

36. A compound according to claim 35, wherein X₄ is phenylene optionally substituted with one or more substituents selected from
• halogen or
• C₁₋₆-alkyl optionally substituted with one or more halogens.

37. A compound according to claim 35 or36, wherein X₄ is phenylene.

38. A compound according to any one of the claims 1-34, wherein X₄ is heteroarylene optionally substituted with one or more substituents selected from
• halogen or
• C₁₋₆-alkyl optionally substituted with one or more halogens.

39. A compound according to any one of the preceding claims, wherein Ar is phenylene which is optionally substituted with one or more substituents selected from
• halogen, hydroxy or cyano; or
• C₁₋₆-alkyl, C₃₋₆-cycloalkyl, C₂₋₆-alkenyl, C₂₋₆-alkynyl, aryl, heteroaryl, aralkyl, heteroaralkyl, C₁₋₆-alkoxy, C₃₋₆-cycloalkoxy, aryloxy, aralkoxy, heteroaralkoxy, C₁₋₆-alkylthio, arylthio or C₃₋₆-cycloalkylthio each of which is optionally substituted with one or more halogens; or
• two of the substituents when placed in adjacent positions together with the atoms to which they are attached may form a five to eight member ring.

40. A compound according to claim 39, wherein Ar is phenylene which is optionally substituted with one or more substituents selected from
• halogen; or
• C₁₋₆-alkyl, C₁₋₆-alkoxy, aryloxy or aralkoxy each of which is optionally substituted with one or more halogens; or
• two of the substituents when placed in adjacent positions together with the atoms to which they are attached form a five membered carbon cycle.

41. A compound according to claim 39 or 40, wherein Ar is phenylene which is optionally substituted with halogen.

42. A compound according to any one of the claims 39-41, wherein Ar is phenylene which is optionally substituted with methyl.

43. A compound according to any one of the preceding claims, wherein Y₁ is S.

44. A compound according to any one of the preceding claims, wherein Y₂ is O.

45. A compound according to any one of the preceding claims, wherein n is 1.

46. A compound according to any one of the preceding claims, wherein R₁ is hydrogen or a substituent selected from
• C₁₋₆-alkyl, aralkyl, C₁₋₆-alkoxy, aryloxy, aralkoxy each of which is optionally substituted with one or more halogens.

47. A compound according to claim 46, wherein R₁ is hydrogen or a substituent selected from
• C₁₋₆-alkyl, C₁₋₆-alkoxy each of which is optionally substituted with one or more halogens.

48. A compound according to claim 46 or 47, wherein R₁ is hydrogen.

49. A compound according to any one of the preceding claims, wherein R₂ is hydrogen or C₁₋₆-alkyl.

50. A compound according to claim 49 wherein R₂ is hydrogen.

51. A compound according to any one of the preceding claims, which is:
{4-[3,3-Bis-(4-phenylethynyl-phenyl)-allylsulfanyl]-2-methyl-phenoxy}-acetic acid;
{4-[3,3-Bis-(4-phenylethynyl-phenyl)-allylsulfanyl]-2-bromo-phenoxy}-acetic acid; or
a salt thereof with a pharmaceutically acceptable acid or base, or any optical isomer or mixture of optical isomers, including a racemic mixture, or any tautomeric forms.

52. A compound according to any one of the preceding claims 1-50, which is:
[4-[3,3-Bis[4-[(thiofen-2-yl)ethylnyl]phenyl]allylsulfanyl]-2-methylphenoxy]acetic acid;
{4-[3,3-Bis-(4-thiophen-3-ylethynylphenyl)allylsulfanyl]-2-methylphenoxy}acetic acid;
[4-[3,3-Bis[4-[(pyridine-2-yl)ethylnyl]phenyl]allylsulfanyl]-2-methylphenoxy]acetic acid;
{4-[3,3-Bis-(4-pyridin-2-ylethynylphenyl)allyloxy]-2-methylphenoxy}acetic acid;
{4-[3,3-Bis-(4-furan-2-ylethynylphenyl)allylsulfanyl]-2-methylphenoxy}acetic acid;
(4-{3,3-Bis-[4-(1-methyl-1H-pyrrol-2-ylethynyl)phenyl]allylsulfanyl}-2-methylphenoxy)acetic acid;
[4-[3,3-Bis[4-[3-(morpholine-4-yl)propyn-1-yl]phenyl]allylsulfanyl]-2-methylphenoxy]acetic acid;
[4-[3,3-Bis[4-[3-(N,N-dimethylamino)propyn-1-yl]phenyl]allylsulfanyl]-2-methylphenoxy]acetic acid;
[4-[3,3-Bis[4-[3-(morpholine-4-yl)propynyl]phenyl]allyloxy]-2-methylphenoxy]acetic acid;
[4-(3,3-Bis-{4-[3-(4-acetyl-piperazin-1-yl)-prop-1-ynyl]-phenyl}allylsulfanyl)-2-methyl-phenoxy]acetic acid;
[4-[3,3-Bis[4-(3-pyrrolidin-1-yl-prop-1-ynyl)phenyl]allylsulfanyl]-2-methylphenoxy]acetic acid;
(4-{3,3-Bis-[4-(3-pyrrolidin-1-yl-prop-1-ynyl)phenyl]allyloxy}-2-methylphenoxy)acetic acid;
[4-[3,3-Bis[5-[3-(morpholine-4-yl)propynyl]thiophene-2-yl]allylsulfanyl]-2-methylphenoxy]-acetic acid;
[4-[3,3-Bis[5-[3-(N-acetyl-N-methylamino)propynyl]thiophene-2-yl]allylsulfanyl]-2-methylphenoxy]acetic acid;
[4-[3,3-Bis[4-[3,3,3-trimethylpropynyl]phenyl]allylsulfanyl]-2-methylphenoxy]acetic acid;
[4-[3,3-Bis[4-[3-(imidazol-1-yl)propynyl]phenyl]allylsulfanyl]-2-methylphenoxy]acetic acid;
[4-[3,3-Bis[4-[3-(2-oxopyrrolidin-1-yl)propynyl]phenyl]allylsulfanyl]-2-methylphenoxy]acetic acid;
[4-[3,3-Bis(4-methylcarbamoylethynylphenyl)allylsulfanyl]-2-methylphenoxy]acetic acid; or
a salt thereof with a pharmaceutically acceptable acid or base, or any optical isomer or mixture of optical isomers, including a racemic mixture, or any tautomeric forms.

53. A compound according to any one of the preceding claims, which is a PPARδ agonist.

54. A compound according to claim 53, which is a selective PPARδ agonist.

55. A combination of a compound as defined in any one of claims 1 to 54 and one or more additional pharmacologically active substances.

56. The combination according to claim 55 wherein the one or more additional pharmacologically active substances are selected from antiobesity agents, antidiabetics, antihypertensive agents, agents for the treatment and/or prevention of complications resulting from or associated with diabetes and agents for the treatment and/or prevention of complications and disorders resulting from or associated with obesity.

57. A pharmaceutical composition comprising, as an active ingredient, at least one compound according to any one of claims 1-54, or a combination according to claim 55 or 56, together with one or more pharmaceutically acceptable carriers or excipients.

58. A pharmaceutical composition according to claim 57 in unit dosage form, comprising from about 0.05 mg to about 1000 mg, preferably from about 0.1 to about 500 mg of and especially preferred from about 0.5 mg to about 200 mg per day of compound according to any one of claims 1-54.

59. A pharmaceutical composition according to claim 57 or claim 58 for oral, nasal, transdermal, pulmonal or parenteral administration.

60. A compound according to any one of claims 1-54, a combination according to claim z or 56, or a composition according to any one of claims 57-59, for use as a medicament.

61. A compound, combination or composition according to claim 60 for the treatment and/or prevention of conditions mediated by nuclear receptors, in particular the Peroxisome Proliferator-Activated Receptors (PPAR).

62. A compound, combination or composition according to claim 60 for the treatment and/or prevention of Type 1 diabetes, Type 2 diabetes, dyslipidemia, syndrome X (including the metabolic syndrome, i.e. impaired glucose tolerance, insulin resistance, hypertriglyceridaemia and/or obesity), cardiovascular diseases (including atherosclerosis) and hypercholesteremia.

63. A compound, combination or composition according to claim 60 for the treatment and/or prevention of type I diabetes, type II diabetes, impaired glucose tolerance, insulin resistance or obesity.

64. A compound, combination or composition according to claim 60 wherein the effective amount of the compound according to any one of the claims 1-54 is in the range of from about 0.05 mg to about 1000 mg, preferably from about 0.1 to about 500 mg of and especially preferred from about 0.5 mg to about 200 mg per day.

65. Use of a compound as defined in any one of claims 1 to 54, or a combination as defined in claim 55 or claim 56, or a composition as defined in any one of claims 57 to 59, in the manufacture of a medicament for the treatment and/or prevention of a disease or condition as defined in any one of claims 61 to 64.

## Patentansprüche

1. Verbindung der allgemeinen Formel (I): 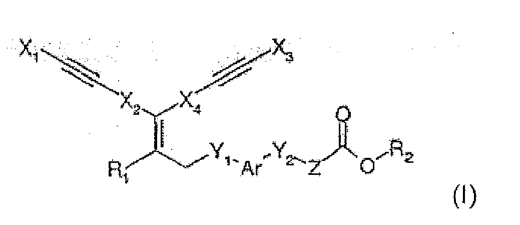 wobei X₁ Aryl oder Heteroaryl ist, von denen jedes optional mit einem oder mehreren Substituenten substituiert ist, die ausgewählt sind aus:
• Halogen, Hydroxy, Cyano, Amino oder Carboxy, oder
• C₁₋₆-Alkyl, C₃₋₆-Cycloalkyl, C₂₋₆-Alkenyl, C₂₋₆-Alkinyl, Aryl, Aralkyl, Heteroaryl, Heteroaralkyl, C₁₋₆-Alkoxy, C₃₋₆-Cycloalkoxy, Aryloxy, Aralkoxy, Heteroaralkoxy, C₁₋₆-Alkylthio, Arylthio, C₃₋₆-Cycloalkylthio, C₁₋₆-Alkylcarbonyl, Arylcarbonyl, C₁₋₆-Alkylsulfonyl, Arylsulfonyl, C₁₋₆-Alkylamido, Arylamido, C₁₋₆-Alkylaminocarbonyl, C₁₋₆-Alkylamino, C₁₋₆-Dialkylamino oder C₃₋₆-Cycloalkylamino, von denen jedes optional mit einem oder mehreren Halogenen substituiert ist, oder
X₁ C₁₋₆-Alkyl, C₃₋₆-Cycloalkyl, C₂₋₆-Alkenyl, Carbamoyl oder C₃₋₆-Cycloalkyl-C₁₋₆-alkyl ist, von denen jedes optional mit einem oder mehreren Substituenten substituiert ist, die ausgewählt sind aus:
• Halogen, Hydroxy, Cyano, Amino oder Carboxy, oder
• C₁₋₆-Alkyl, C₃₋₆-Cycloalkyl, C₂₋₆-Alkenyl, C₂₋₆-Alkinyl, C₃₋₆-Cycloalkyl-C₁₋₆-alkyl, Aryl, Aralkyl, Heteroaryl, Heteroaralkyl, Heterocyclyl, C₁₋₆-Alkoxy, C₃₋₆-Cycloalkoxy, C₃₋₆-Cycloalkyl-C₁₋₆-alkoxy, Aryloxy, Heteroaryloxy, Aralkoxy, Heteroaralkoxy, C₁₋₆-Alkylthio, C₃₋₆-Cycloalkylthio,
C₃₋₆-Cycloalkyl-C₁₋₆-alkylthio, Arylthio, Heteroarylthio, Aryl-C₁₋₆-alkylthio, Heteroaryl-C₁₋₆-alkylthio, C₁₋₆-Alkylcarbonyl, C₃₋₆-Cycloalkylcarbonyl, C₃₋₆-Cycloalkyl-C₁₋₆-alkylcarbonyl, Arylcarbonyl, Heteroarylcarbonyl, C₁₋₆-Alkylsulfonyl, C₃₋₆-Cycloalkylsulfonyl, C₃₋₆-Cycloalkyl-C₁₋₆-alkylsulfonyl, Arylsulfonyl, Heteroarylsulfonyl, C₁₋₆-Alkylsulfamoyl, Di-(C₁₋₆-alkyl)sulfamoyl, C₁₋₆-Alkoxycarbonyl, C₃₋₆-Cycloalkoxycarbonyl, C₃₋₆-Cycloalkyl-C₁₋₆-alkoxycarbonyl, Amino-C₁₋₆-alkyl, C₁₋₆-Alkylamino-C₁₋₆-alkyl, Di-(C₁₋₆-alkyl)amino-C₁₋₆-alkyl, C₁₋₆-Alkylamido, C₃₋₆-Cycloalkylamido, C₃₋₆-Cycloalkyl-C₁₋₆alkylamido, Arylamido, C₁₋₆-Alkylaminocarbonyl, C₃₋₆-Cycloalkylaminocarbonyl, C₃₋₆-Cycloalkyl-C₁₋₆-alkylaminocarbonyl, Di-(C₁₋₆-alkyl)aminocarbonyl, Di-(C₃₋₆-cycloalkyl-C₁₋₆-alkyl)aminocarbonyl, C₁₋₆-Alkylamino, C₃₋₆-Cycloalkylamino, C₃₋₆-Cycloalkyl-C₁₋₆-alkylamino, Di-(C₁₋₆-alkyl)amino, Di-(C₃₋₆-cycloalkyl)amino oder Di-(C₃₋₆-cycloalkyl-C₁₋₆-alkyl)amino, von denen jedes optional mit einem oder mehreren aus Halogen, Cyano, Hydroxy, Acetyl oder Oxo substituiert ist, und
X₂ Arylen oder Heteroarylen ist, von denen jedes optional mit einem oder mehreren Substituenten substituiert ist, die ausgewählt sind aus:
• Halogen, Hydroxy, Cyano, Amino oder Carboxy, oder
• C₁₋₆-Alkyl, C₃₋₆-Cycloalkyl, C₂₋₆-Alkenyl, C₂₋₆-Alkinyl, C₁₋₆-Alkoxy, C₃₋₆-Cycloalkoxy, C₁₋₆-Alkylthio, C₃₋₆-Cycloalkylthio, C₁₋₆-Alkylamino, C₁₋₆-Dialkylamino oder C₃₋₆-Cycloalkylamino, von denen jedes optional mit einem oder mehreren Halogenen substituiert ist, und
X₃ Aryl oder Heteroaryl ist, von denen jedes optional mit einem oder mehreren Substituenten substituiert ist, die ausgewählt sind aus:
• Halogen, Hydroxy, Cyano, Amino oder Carboxy, oder
• C₁₋₆-Alkyl, C₃₋₆-Cycloalkyl, C₂₋₆-Alkenyl, C₂₋₆-Alkinyl, Aryl, Aralkyl, Heteroaryl, Heteroaralkyl, C₁₋₆-Alkoxy, C₃₋₅-Cycloalkoxy, Aryloxy, Aralkoxy, Heteroaralkoxy, C₁₋₆-Alkylthio, Arylthio, C₃₋₆-Cycoalkylthio, C₁₋₆-Alkylcarbonyl, Arylcarbonyl, C₁₋₆-Alkylsulfonyl, Arylsulfonyl, C₁₋₆-Alkylamido, Arylamido, C₁₋₆-Alkylaminocarbonyl, C₁₋₆-Alkylamino, C₁₋₆-Dialkylamino oder C₃₋₆-Cycloalkylamino, von denen jedes optional mit einem oder mehreren Halogenen substituiert ist, oder
X₃ C₁₋₆-Alkyl, C₃₋₆-Cycloalkyl, C₂₋₆-Alkenyl, Carbamoyl oder C₃₋₆-Cycloalkyl-C₁₋₆-alkyl ist, von denen jedes optional mit einem oder mehreren Substituenten substituiert ist, die ausgewählt sind aus:
• Halogen, Hydroxy, Cyano, Amino oder Carboxy, oder
• C₁₋₆-Alkyl, C₃₋₆-Cycloalkyl C₂₋₆-Alkenyl, C₂₋₆-Alkinyl; C₃₋₆-Cycloalkyl-C₁₋₆-alkyl, Aryl, Aralkyl, Heteroaryl, Heteroaralkyl, Heterocyclyl, C₁₋₆-Alkoxy, C₃₋₆-Cycloalkoxy, C₃₋₆-Cycloalkyl-C₁₋₆-alkoxy, Aryloxy, Heteroaryloxy, Aralkoxy, Heteroaralkoxy, C₁₋₆-Alkylthio, C₃₋₆-Cycloalkylthio,
C₃₋₆-Cycloalkyl-C₁₋₆-alkylthio, Arylthio, Heteroarylthio, Aryl-C₁₋₆-alkylthio, Heteroaryl-C₁₋₆-alkylthio, C₁₋₆-Alkylcarbonyl, C₃₋₆-Cycloalkylcarbonyl, C₃₋₆-Cycloalkyl-C₁₋₆-alkylcarbonyl, Arylcarbonyl, Heteroarylcarbonyl, C₁₋₆-Alkylsulfonyl, C₃₋₆-Cycloalkylsulfonyl, C₃₋₆-Cycloalkyl-C₁₋₆-alkylsulfonyl, Arylsulfonyl, Heteroarylsulfonyl, C₁₋₆-Alkylsulfamoyl, Di-(C₁₋₆-alkyl)sulfamoyl, C₁₋₆-Alkoxycarbonyl, C₃₋₆-Cycloalkoxycarbonyl, C₃₋₆-Cycloalkyl-C₁₋₆-alkoxycarbonyl, Amino-C₁₋₆-alkyl, C₁₋₆-Alkylamino-C₁₋₆-alkyl, Di-(C₁₋₆-alkyl)amino-C₁₋₆-alkyl, C₁₋₆-Alkylamido, C₃₋₆-Cycloalkylamido, C₃₋₆-Cycloalkyl-C₁₋₆-alkylamido, Arylamido, C₁₋₆-Alkylaminocarbonyl, C₃₋₆-Cycloalkylaminocarbonyl, C₃₋₆-Cycloalkyl-C₁₋₆-alkylaminocarbonyl, Di-(C₁₋₆-alkyl)aminocarbonyl, D1-(C₃₋₆-cycloalkyl-C₁₋₆-alkyl)aminocarbonyl, C₁₋₆-Alkylamino, C₃₋₆-Cycloalkylamino, C₃₋₆-Cycloalkyl-C₁₋₆-alkylamino, Di-(C₁₋₆-alkyl)amino, Di-(C₃₋₆-cycloalkyl)amino oder Di-(C₃₋₆-cycloalkyl-C₁₋₆-alkyl)amino, von denen jedes optional mit einem oder mehreren aus Halogen, Cyano, Hydroxy, Acetyl oder Oxo substituiert ist, und
X₄ Arylen oder Heteroarylen ist, von denen jedes optional mit einem oder mehreren Substituenten substituiert ist, die ausgewählt sind aus:
• Halogen, Hydroxy, Cyano, Amino oder Carboxy, oder
• C₁₋₆-Alkyl, C₃₋₆-Cycloalkyl, C₂₋₆-Alkenyl, C₂₋₆-Alkinyl, C₁₋₆-Alkoxy, C₃₋₆-Cycloalkoxy, C₁₋₆-Alkylthio, C₃₋₆-Cycloalkylthio, C₁₋₆-Alkylamino, C₁₋₆-Dialkylamino oder C₃₋₆-Cycloalkylamino, von denen jedes optional mit einem oder mehreren Halogenen substituiert ist, und
Ar Arylen ist, das optional mit einem oder mehreren Substituenten substituiert ist, die ausgewählt sind aus:
• Halogen, Hydroxy oder Cyano, oder
• C₁₋₆-Alkyl, C₃₋₆-Cycloalkyl, C₂₋₆-Alkenyl, C₂₋₆-Alkinyl, Aryl, Heteroaryl, Aralkyl, Heteroaralkyl, C₁₋₆-Alkoxy, C₃₋₆-Cycloalkoxy, Aryloxy, Aralkoxy, Heteroaralkoxy, C₁₋₆-Alkylthio, Arylthio oder C₃₋₆-Cycloalkylthio, von denen jedes optional mit einem oder mehreren Halogenen substituiert ist, oder
• zwei der Substituenten, wenn sie in benachbarten Positionen gelegen sind, zusammen mit den Atomen, mit denen sie verknüpft sind, einen fünf- bis achtgliedrigen Ring bilden können und
Y₁ O oder S ist und
Y₂ O oder S ist und
Z -(CH₂)ₙ- ist, wobei n gleich 1, 2 oder 3 ist, und
R₁ Wasserstoff, Halogen oder ein Substituent ist, der ausgewählt ist aus:
• C₁₋₆-Alkyl, C₃₋₆-Cycloalkyl, C₂₋₆-Alkenyl, C₂₋₆-Alkinyl, Aralkyl, Heteroaralkyl, C₁₋₆-Alkoxy, C₃₋₆-Cycloalkoxy, Aryloxy, Aralkoxy, Heteroaralkoxy, C₁₋₆-Alkylthio, Arylthio oder C₃₋₆-Cycloalkylthio, von denen jedes optional mit einem oder mehreren Halogenen substituiert ist, und
R₂ Wasserstoff, C₁₋₆-Alkyl, C₃₋₆-Cycloalkyl, C₂₋₆-Alkenyl, C₂₋₆-Alkinyl, C₄₋₆-Alkeninyl oder Aryl ist, oder
ein pharmazeutisch akzeptables Salz davon oder ein pharmazeutisch akzeptables Solvat davon oder beliebige tautomere Formen, Stereoisomere, Mischungen von Stereoisomeren, darunter racemische Mischungen, oder Polymorphe.

2. Verbindung nach Anspruch 1, wobei X₁ Aryl oder Heteroaryl ist, von denen jedes optional mit einem oder mehreren Substituenten substituiert ist, die ausgewählt sind aus:
• Halogen, Hydroxy, Cyano, Amino oder Carboxy, oder
• C₁₋₆-Alkyl, C₃₋₆-Cycloalkyl, C₂₋₆-Alkenyl, C₂₋₆-Alkinyl, Aryl, Aralkyl, Heteroaryl, Heteroaralkyl, C₁₋₆-Alkoxy, C₃₋₆-Cycloalkoxy, Aryloxy, Aralkoxy, Heteroaralkoxy, C₁₋₆-Alkylthio, Arylthio, C₃₋₆-Cycloalkylthio, C₁₋₆-Alkylcarbonyl, Arylcarbonyl, C₁₋₆-Alkylsulfonyl, Arylsulfonyl, C₁₋₆-Alkylamido, Arylamido, C₁₋₆-Alkylaminocarbonyl, C₁₋₆-Alkylamino, C₁₋₆-Dialkylamino oder C₃₋₆-Cycloalkylamino, von denen jedes optional mit einem oder mehreren Halogenen substituiert ist, und
X₂ Arylen oder Heteroarylen ist, von denen jedes optional mit einem oder mehreren Substituenten substituiert ist, die ausgewählt sind aus:
• Halogen, Hydroxy, Cyano, Amino oder Carboxy, oder
• C₁₋₆-Alkyl, C₃₋₆-Cycloalkyl, C₂₋₆-Alkenyl, C₂₋₆-Alkinyl, C₁₋₆-Alkoxy, C₃₋₆-Cycloalkoxy, C₁₋₆-Alkylthio, C₃₋₆-Cycloalkylthio, C₁₋₆-Alkylamino, C₁₋₆-Dialkylamino oder C₃₋₆-Cycloalkylamino, von denen jedes optional mit einem oder mehreren Halogenen substituiert ist, und
X₃ Aryl oder Heteroaryl ist, von denen jedes optional mit einem oder mehreren Substituenten substituiert ist, die ausgewählt sind aus:
• Halogen, Hydroxy, Cyano, Amino oder Carboxy, oder
• C₁₋₆-Alkyl, C₃₋₆-Cycloalkyl, C₂₋₆-Alkenyl, C₂₋₆-Alkinyl, Aryl, Aralkyl, Heteroaryl, Heteroaralkyl, C₁₋₆-Alkoxy, C₃₋₆-Cycloalkoxy, Aryloxy, Aralkoxy, Heteroaralkoxy, C₁₋₆-Alkylthio, Arylthio, C₃₋₆-Cycloalkylthio, C₁₋₆-Alkylcarbonyl, Arylcarbonyl, C₁₋₆-Alkylsulfonyl, Arylsulfonyl, C₁₋₆-Alkylamido, Arylamido, C₁₋₆-Alkylaminocarbonyl, C₁₋₆-Alkylamino, C₁₋₆-Dialkylamino oder C₃₋₆-Cycloalkylamino, von denen jedes optional mit einem oder mehreren Halogenen substituiert ist, und
X₄ Arylen oder Heteroarylen ist, von denen jedes optional mit einem oder mehreren Substituenten substituiert ist, die ausgewählt sind aus:
• Halogen, Hydroxy, Cyano, Amino oder Carboxy, oder
• C₁₋₆-Alkyl, C₃₋₆-Cycloalkyl, C₂₋₆-Alkenyl, C₂₋₆-Alkinyl, C₁₋₆-Alkoxy, C₃₋₆-Cycloalkoxy, C₁₋₆-Alkylthio, C₃₋₆-Cycloalkylthio, C₁₋₆-Alkylamino, C₁₋₆-Dialkylamino oder C₃₋₆-Cycloalkylamino, von denen jedes optional mit einem oder mehreren Halogenen substituiert ist, und
Ar Arylen ist, das optional mit einem oder mehreren Substituenten substituiert ist, die ausgewählt sind aus:
• Halogen, Hydroxy oder Cyano, oder
• C₁₋₆-Alkyl, C₃₋₆-Cycloalkyl, C₂₋₆-Alkenyl, C₂₋₆-Alkinyl, Aryl, Heteroaryl, Aralkyl, Heteroaralkyl, C₁₋₆-Alkoxy, C₃₋₆-Cycloalkoxy, Aryloxy, Aralkoxy, Heteroaralkoxy, C₁₋₆-Alkylthio, Arylthio oder C₃₋₆-Cycloalkylthio, von denen jedes optional mit einem oder mehreren Halogenen substituiert ist, oder
• zwei der Substituenten, wenn sie in benachbarten Positionen gelegen sind, zusammen mit den Atomen, mit denen sie verknüpft sind, einen fünf- bis achtgliedrigen Ring bilden können und
Y₁ O oder S ist und
Y₂ O oder S ist und
Z -(CH₂)ₙ- ist, wobei n gleich 1, 2 oder 3 ist, und
R₁ Wasserstoff, Halogen oder ein Substituent ist, der ausgewählt ist aus:
• C₁₋₆-Alkyl, C₃₋₆-Cycloalkyl, C₂₋₆-Alkenyl, C₂₋₆-Alkinyl, Aralkyl, Heteroaralkyl, C₁₋₆-Alkoxy, C₃₋₆-Cycloalkoxy, Aryloxy, Aralkoxy, Heteroaralkoxy, C₁₋₆-Alkylthio, Arylthio oder C₃₋₆-Cycloalkylthio, von denen jedes optional mit einem oder mehreren Halogenen substituiert ist, und
R₂ Wasserstoff, C₁₋₆-Alkyl, C₃₋₆-Cycloalkyl, C₂₋₆-Alkenyl, C₂₋₆-Alkinyl, C₄₋₆-Alkeninyl oder Aryl ist, oder
ein pharmazeutisch akzeptables Salz davon oder ein pharmazeutisch akzeptables Solvat davon oder beliebige tautomere Formen, Stereoisomere, Mischungen von Stereoisomeren, darunter racemische Mischungen, oder Polymorphe.

3. Verbindung nach Anspruch 1 oder 2, wobei X₁ Aryl ist, das optional mit einem oder mehreren Substituenten substituiert ist, die ausgewählt sind aus:
• Halogen oder
• C₁₋₆-Alkyl, Aryl, C₁₋₆-Alkoxy oder C₁₋₆-Alkylsulfonyl, von denen jedes optional mit einem oder mehreren Halogenen substituiert ist.

4. Verbindung nach Anspruch 3, wobei X₁ Aryl ist, das optional mit einem oder mehreren Substituenten substituiert ist, die ausgewählt sind aus:
• Halogen oder
• C₁₋₆-Alkyl, das optional mit einem oder mehreren Halogenen substituiert ist.

5. Verbindung nach Anspruch 3, wobei X₁ Phenyl ist, das optional mit einem oder mehreren Substituenten substituiert ist, die ausgewählt sind aus:
• Halogen oder
• C₁₋₆-Alkyl, Aryl, C₁₋₆-Alkoxy oder C₁₋₆-Alkylsulfonyl, von denen jedes optional mit einem oder mehreren Halogenen substituiert ist.

6. Verbindung nach Anspruch 4 oder 5, wobei X₁ Phenyl ist, das optional mit einem oder mehreren Substituenten substituiert ist, die ausgewählt sind aus:
• Halogen oder
• C₁₋₆-Alkyl, das optional mit einem oder mehreren Halogenen substituiert ist.

7. Verbindung nach einem der vorhergehenden Ansprüche, wobei X₁ Phenyl ist.

8. Verbindung nach Anspruch 1 oder 2, wobei X₁ Heteroaryl ist, das optional mit einem oder mehreren Substituenten substituiert ist, die ausgewählt sind aus:
• Halogen oder
• C₁₋₆-Alkyl, Aryl, C₁₋₆-Alkoxy oder C₁₋₆-Alkylsulfonyl, von denen jedes optional mit einem oder mehreren Halogenen substituiert ist.

9. Verbindung nach Anspruch 8, wobei X₁ Heteroaryl ist, das optional mit einem oder mehreren Substituenten substituiert ist, die ausgewählt sind aus:
• Halogen oder
• C₁₋₆-Alkyl, das optional mit einem oder mehreren Halogenen substituiert ist.

10. Verbindung nach Anspruch 8 oder 9, wobei X₁ Pyrrolyl, Pyridyl, Furyl oder Thienyl ist, von denen jedes optional mit einem oder mehreren Halogenen oder C₁₋₆-Alkyl substituiert ist, das optional mit einem oder mehreren Halogenen substituiert ist.

11. Verbindung nach Anspruch 10, wobei X₁ Furyl oder Thienyl ist, das optional mit einem oder mehreren Halogenen substituiert ist.

12. Verbindung nach Anspruch 10, wobei X₁ Pyrrolyl oder Pyridyl ist, das optional mit einem oder mehreren C₁₋₆-Alkyl(en) substituiert ist.

13. Verbindung nach Anspruch 1, wobei X₁ C₁₋₆-Alkyl oder Carbamoyl ist, das optional mit einem oder mehreren Substituenten substituiert ist, die ausgewählt sind aus:
• Halogen, Hydroxy, Cyano, Amino oder Carboxy, oder
• C₁₋₆-Alkyl, C₃₋₆-Cycloalkyl, C₂₋₆-Alkenyl, C₂₋₆-Alkinyl, C₃₋₆-Cycloalkyl-C₁₋₆-alkyl, Aryl, Aralkyl, Heteroaryl, Heteroaralkyl, Heterocyclyl, C₁₋₆-Alkoxy, C₃₋₆-Cycloalkoxy, C₃₋₆-Cycloalkyl-C₁₋₆-alkoxy, Aryloxy, Heteroaryloxy, Aralkoxy, Heteroaralkoxy, C₁₋₆-Alkylthio, C₃₋₆-Cycloalkylthio, C₃₋₆-Cycloalkyl-C₁₋₆-alkylthio, Arylthio, Heteroarylthio, Aryl-C₁₋₆-alkylthio, Heteroaryl-C₁₋₆-alkylthio, C₁₋₆-Alkylcarbonyl, C₃₋₆-Cycoalkylcarbonyl, C₃₋₆-Cycloalkyl-C₁₋₆-alkylcarbonyl, Arylcarbonyl, Heteroarylcarbonyl, C₁₋₆-Alkylsulfonyl, C₃₋₆-Cycloalkylsulfonyl, C₃₋₆-Cycloalkyl-C₁₋₆-alkylsulfonyl, Arylsulfonyl, Heteroarylsulfonyl, C₁₋₆-Alkylsulfamoyl, Di-(C₁₋₆-alkyl)sulfamoyl, C₁₋₆-Alkoxycarbonyl, C₃₋₆-Cycloalkoxycarbonyl, C₃₋₆-Cycloalkyl-C₁₋₆-alkoxycarbonyl, Amino-C₁₋₆-alkyl, C₁₋₆-Alkylamino-C₁₋₆-alkyl, Di-(C₁₋₆-alkyl)amino-C₁₋₆-alkyl, C₁₋₆-Alkylamido, C₃₋₆-Cycloalkylamido, C₃₋₆-Cycloalkyl-C₁₋₆-alkylamido, Arylamido, C₁₋₆-Alkylaminocarbonyl, C₃₋₆-Cycloalkylaminocarbonyl, C₃₋₆-Cycloalkyl-C₁₋₆-alkylaminocarbonyl, D₁-(C₁₋₆-alkyl)aminocarbonyl, Di-(C₃₋₆-cycloalkyl-C₁₋₆-alkyl)aminocarbonyl, C₁₋₆-Alkylamino, C₃₋₆-Cycloalkylamino, C₃₋₆-Cycloalkyl-C₁₋₆-alkylamino, Di-(C₁₋₆-alkyl)amino, Di-(C₁₋₆-cycloalkyl)amino oder Di-(C₃₋₆-cycloalkyl-C₁₋₆-alkyl)amino, von denen jedes optional mit einem oder mehreren aus Halogen, Cyano, Hydroxy, Acetyl oder Oxo substituiert ist.

14. Verbindung nach Anspruch 13, wobei X₁ C₁₋₆-Alkyl oder Carbamoyl ist, das optional mit einem oder mehreren Substituenten substituiert ist, die ausgewählt sind aus:
• Halogen oder Hydroxy, oder
• C₁₋₆-Alkyl, Aryl, Heteroaryl, Heterocyclyl, C₁₋₆-Alkoxy, C₁₋₆-Alkylthio, C₃₋₆-Cycloalkylthio, C₃₋₆-Cycloalkyl-C₁₋₆-alkylthio, Arylthio, Heteroarylthio, Aryl-C₁₋₆-alkylthio, C₁₋₆-Alkylcarbonyl, Arylcarbonyl, C₁₋₆-Alkylsulfonyl, Arylsulfonyl, Amino-C₁-₆-alkyl, C₁₋₆-Alkylamino-C₁₋₆-alkyl, Di-(C₁₋₆-alkyl)amino-C₁₋₆-alkyl, C₁₋₆-Alkylamido, Arylamido, C₁₋₆-Alkylaminocarbonyl, Di-(C₁₋₆-alkyl)aminocarbonyl, C₁₋₆-Alkylamino, Di-(C₁₋₆-alkyl)amino, von denen jedes optional mit einem oder mehreren aus Halogen, Cyano, Hydroxy, Acetyl oder Oxo substituiert ist.

15. Verbindung nach Anspruch 13 oder 14, wobei X₁ C₁₋₆-Alkyl ist, das optional mit einem oder mehreren Substituenten substituiert ist, die ausgewählt sind aus:
C₁₋₆-Alkyl, Heteroaryl, Heterocyclyl, C₁₋₆-Alkylamino, Di-(C₁₋₆-alkyl)amino, von denen jedes optional mit Acetyl und/oder Oxo substituiert ist.

16. Verbindung nach Anspruch 13 oder 14, wobei X₃ Carbamoyl ist, das optional mit Acetyl substituiert ist.

17. Verbindung nach einem der vorhergehenden Ansprüche, wobei X₂ Arylen ist, das optional mit einem oder mehreren Substituenten substituiert ist, die ausgewählt sind aus:
• Halogen oder
• C₁₋₆-Alkyl, das optional mit einem oder mehreren Halogenen substituiert ist.

18. Verbindung nach Anspruch 17, wobei X₂ Phenylen ist, das optional mit einem oder mehreren Substituenten substituiert ist, die ausgewählt sind aus:
• Halogen oder
• C₁₋₆-Alkyl, das optional mit einem oder mehreren Halogenen substituiert ist.

19. Verbindung nach Anspruch 17 oder 18, wobei X₂ Phenylen ist.

20. Verbindung nach einem der Ansprüche 1 bis 16, wobei X₂ Heteroarylen ist, das optional mit einem oder mehreren Substituenten substituiert ist, die ausgewählt sind aus:
• Halogen oder
• C₁₋₆-Alkyl, das optional mit einem oder mehreren Halogenen substituiert ist.

21. Verbindung nach einem der vorhergehenden Ansprüche, wobei X₃ Aryl ist, das optional mit einem oder mehreren Substituenten substituiert ist, die ausgewählt sind aus:
• Halogen oder
• C₁₋₆-Alkyl, Aryl, C₁₋₆-Alkoxy oder C₁₋₆-Alkylsulfonyl, von denen jedes optional mit einem oder mehreren Halogenen substituiert ist.

22. Verbindung nach Anspruch 21, wobei X₃ Aryl ist, das optional mit einem oder mehreren Substituenten substituiert ist, die ausgewählt sind aus:
• Halogen oder
• C₁₋₆-Alkyl, das optional mit einem oder mehreren Halogenen substituiert ist.

23. Verbindung nach Anspruch 21, wobei X₃ Phenyl ist, das optional mit einem oder mehreren Substituenten substituiert ist, die ausgewählt sind aus:
• Halogen oder
• C₁₋₆-Alkyl, Aryl, C₁₋₆-Alkoxy oder C₁₋₆-Alkylsulfonyl, von denen jedes optional mit einem oder mehreren Halogenen substituiert ist.

24. Verbindung nach Anspruch 22 oder 23, X₃ Phenyl ist, das optional mit einem oder mehreren Substituenten substituiert ist, die ausgewählt sind aus:
• Halogen oder
• C₁₋₆-Alkyl, das optional mit einem oder mehreren Halogenen substituiert ist.

25. Verbindung nach einem der Ansprüche 21 bis 24, wobei X₃ Phenyl ist.

26. Verbindung nach einem der Ansprüche 1 bis 20, wobei X₃ Heteroaryl ist, das optional mit einem oder mehreren Substituenten substituiert ist, die ausgewählt sind aus:
• Halogen oder
• C₁₋₆-Alkyl, Aryl, C₁₋₆-Alkoxy oder C₁₋₆-Alkylsulfonyl, von denen jedes optional mit einem oder mehreren Halogenen substituiert ist.

27. Verbindung nach Anspruch 26, wobei X₃ Heteroaryl ist, das optional mit einem oder mehreren Substituenten substituiert ist, die ausgewählt sind aus:
• Halogen oder
• C₁₋₆-Alkyl, das optional mit einem oder mehreren Halogenen substituiert ist.

28. Verbindung nach Anspruch 27, wobei X₃ Pyrrolyl, Pyridyl, Furyl oder Thienyl ist, von denen jedes optional mit einem oder mehreren Halogenen oder C₁₋₆-Alkyl substituiert ist, das optional mit einem oder mehreren Halogenen substituiert ist.

29. Verbindung nach Anspruch 28, wobei X₃ Furyl oder Thienyl ist, das optional mit einem oder mehreren Halogenen substituiert ist.

30. Verbindung nach Anspruch 28, wobei X₃ Pyrrolyl oder Pyridyl ist, das optional mit einem oder mehreren C₁₋₆-Alkyl(en) substituiert ist.

31. Verbindung nach Anspruch 1, wobei X₃ C₁₋₆-Alkyl oder Carbamoyl ist, das optional mit einem oder mehreren Substituenten substituiert ist, die ausgewählt sind aus:
• Halogen, Hydroxy, Cyano, Amino oder Carboxy, oder
• C₁₋₆-Alkyl, C₃₋₆-Cycloalkyl, C₂₋₆-Alkenyl, C₂₋₆-Alkinyl, C₃₋₆-Cycloalkyl-C₁₋₆-alkyl, Aryl, Aralkyl, Heteroaryl, Heteroaralkyl, Heterocyclyl, C₁₋₆-Alkoxy, C₃₋₆-Cycloalkoxy, C₃₋₆-Cycloalkyl-C₁₋₆-alkoxy, Aryloxy, Heteroaryloxy, Aralkoxy, Heteroaralkoxy, C₁₋₆-Alkylthio, C₃₋₆-Cycloalkylthio, C₃₋₆-Cycloalkyl-C₁₋₆-alkylthio, Arylthio, Heteroarylthio, Aryl-C₁₋₆-alkylthio, Heteroaryl-C₁₋₆-alkylthio, C₁₋₆-Alkylcarbonyl, C₃₋₆-Cycloalkylcarbonyl, C₃₋₆-Cycloalkyl-C₁₋₆-alkylcarbonyl, Arylcarbonyl, Heteroarylcarbonyl, C₁₋₆-Alkylsulfonyl, C₃₋₆-Cycloalkylsulfonyl, C₃₋₆-Cycloalkyl-C₁₋₆-alkylsulfonyl, Arylsulfonyl, Heteroarylsulfonyl, C₁₋₆-Alkylsulfamoyl, Di-(C₁₋₆-alkyl)sulfamoyl, C₁₋₆-Alkoxycarbonyl, C₃₋₆-Cycloalkoxycarbonyl, C₃₋₆-Cycloalkyl-C₁₋₆-alkoxycarbonyl, Amino-C₁₋₆-alkyl, C₁₋₆-Alkylamino-C₁₋₆-alkyl, Di-(C₁₋₆-alkyl)amino-C₁₋₆-alkyl, C₁₋₆-Alkylamido, C₃₋₆-Cycloalkylamido, C₃₋₆-Cycloalkyl-C₁₋₆-alkylamido, Arylamido, C₁₋₆-Alkylaminocarbonyl, C₃₋₆-Cycloalkylaminocarbonyl, C₃₋₆-Cycloalkyl-C₁₋₆-alkylaminocarbonyl, Di-(C₁₋₆-alkyl)aminocarbonyl, Di-(C₃₋₆-cycloalkyl-C₁₋₆-alkyl)aminocarbonyl, C₁₋₆-Alkylamino, C₃₋₆-Cycloalkylamino, C₃₋₆-Cycloalkyl-C₁₋₆-alkylamino, Di-(C₁₋₆-alkyl)amino, Di-(C₃₋₆-cycloalkyl)amino oder Di-(C₃₋₆-cycloalkyl-C₁₋₆-alkyl)amino, von denen jedes optional mit einem oder mehreren aus Halogen, Cyano, Hydroxy, Acetyl oder Oxo substituiert ist.

32. Verbindung nach Anspruch 31, wobei X₃ C₁₋₆-Alkyl oder Carbamoyl ist, das optional mit einem oder mehreren Substituenten substituiert ist, die ausgewählt sind aus:
• Halogen oder Hydroxy, oder
• C₁₋₆-Alkyl, Aryl, Heteroaryl, Heterocyclyl, C₁₋₆-Alkoxy, C₁₋₆-Alkylthio, C₃₋₆-Cycloalkylthio, C₃₋₆-Cycloalkyl-C₁₋₆-alkylthio, Arylthio, Heteroarylthio, Aryl-C₁₋₆-alkylthio, C₁₋₆-Alkylcarbonyl, Arylcarbonyl, C₁₋₆-Alkylsulfonyl, Arylsulfonyl, Amino-C₁₋₆-alkyl, C₁₋₆-Alkylamino-C₁₋₆-alkyl, Di-(C₁₋₆-alkyl)amino-C₁₋₆-alkyl, C₁₋₆-Alkylamido, Arylamido, C₁₋₆-Alkylaminocarbonyl, Di-(C₁₋₆-alkyl)aminocarbonyl, C₁₋₆-Alkylamino, Di-(C₁₋₆-alkyl)amino, von denen jedes optional mit einem oder mehreren aus Halogen, Cyano, Hydroxy, Acetyl oder Oxo substituiert ist.

33. Verbindung nach Anspruch 31 oder 32, wobei X₃ C₁₋₆-Alkyl ist, das optional mit einem oder mehreren Substituenten substituiert ist, die ausgewählt sind aus:
C₁₋₆-Alkyl, Heteroaryl, Heterocyclyl, C₁₋₆-Alkylamino, Di-(C₁₋₆-alkyl)amino, von denen jedes optional mit Acetyl und/oder Oxo substituiert ist.

34. Verbindung nach Anspruch 31 oder 32, wobei X₃ Carbamoyl ist, das optional mit Acetyl substituiert ist.

35. Verbindung nach einem der vorhergehenden Ansprüche, wobei X₄ Arylen ist, das optional mit einem oder mehreren Substituenten substituiert ist, die ausgewählt sind aus:
• Halogen oder
• C₁₋₆-Alkyl, das optional mit einem oder mehreren Halogenen substituiert ist.

36. Verbindung nach Anspruch 35, wobei X₄ Phenylen ist, das optional mit einem oder mehreren Substituenten substituiert ist, die ausgewählt sind aus:
• Halogen oder
• C₁₋₆-Alkyl, das optional mit einem oder mehreren Halogenen substituiert ist.

37. Verbindung nach Anspruch 35 oder 36, wobei X₄ Phenylen ist.

38. Verbindung nach einem der Ansprüche 1 bis 34, wobei X₄ Heteroarylen ist, das optional mit einem oder mehreren Substituenten substituiert ist, die ausgewählt sind aus:
• Halogen oder
• C₁₋₆-Alkyl, das optional mit einem oder mehreren Halogenen substituiert ist.

39. Verbindung nach einem der vorhergehenden Ansprüche, wobei Ar Phenylen ist, das optional mit einem oder mehreren Substituenten substituiert ist, die ausgewählt sind aus:
• Halogen, Hydroxy oder Cyano, oder
• C₁₋₆-Alkyl, C₃₋₆-Cycloalkyl, C₂₋₆-Alkenyl, C₂₋₆-Alkinyl, Aryl, Heteroaryl, Aralkyl, Heteroaralkyl, C₁₋₆-Alkoxy, C₃₋₆-Cycloalkoxy, Aryloxy, Aralkoxy, Heteroaralkoxy, C₁₋₆-Alkylthio, Arylthio oder C₃₋₆-Cycloalkylthio, von denen jedes optional mit einem oder mehreren Halogenen substituiert ist, oder
• zwei der Substituenten, wenn sie in benachbarten Positionen gelegen sind, zusammen mit den Atomen, mit denen sie verknüpft sind, einen fünf- bis achtgliedrigen Ring bilden können.

40. Verbindung nach Anspruch 39, wobei Ar Phenylen ist, das optional mit einem oder mehreren Substituenten substituiert ist, die augewählt sind aus:
• Halogen oder
• C₁₋₆-Alkyl, C₁₋₆-Alkoxy, Aryloxy oder Aralkoxy, von denen jedes optional mit einem oder mehreren Halogenen substituiert ist, oder
• zwei der Substituenten, wenn sie in benachbarten Positionen gelegen sind, zusammen mit den Atomen, mit denen sie verknüpft sind, einen fünfgliedrigen Kohlenstoffring bilden.

41. Verbindung nach Anspruch 39 oder 40, wobei Ar Phenylen ist, das optional mit Halogen substituert ist.

42. Verbindung nach einem der Ansprüche 39 bis 41, wobei Ar Phenylen ist, das optional mit Methyl substituiert ist.

43. Verbindung nach einem der vorhergehenden Ansprüche, wobei Y₁ S ist.

44. Verbindung nach einem der vorhergehenden Ansprüche, wobei Y₂ O ist.

45. Verbindung nach einem der vorhergehenden Ansprüche, wobei n gleich 1 ist.

46. Verbindung nach einem der vorhergehenden Ansprüche, wobei R₁ Wasserstoff oder ein Substituent ist, der ausgewählt ist aus:
• C₁₋₆-Alkyl, Aralkyl, C₁₋₆-Alkoxy, Aryloxy, Aralkoxy, von denen jedes optional mit einem oder mehreren Halogenen substituiert ist.

47. Verbindung nach Anspruch 46, wobei R₁ Wasserstoff oder ein Substituent ist, der ausgewählt ist aus:
• C₁₋₆-Alkyl, C₁₋₆-Alkoxy, von denen jedes optional mit einem oder mehreren Halogenen substituiert ist.

48. Verbindung nach Anspruch 46 oder 47, wobei R₁ Wasserstoff ist.

49. Verbindung nach einem der vorhergehenden Ansprüche, wobei R₂ Wasserstoff oder C₁₋₆-Alkyl ist.

50. Verbindung nach Anspruch 49, wobei R₂ Wasserstoff ist.

51. Verbindung nach einem der vorhergehenden Ansprüche, die ist:
{4-[3,3-Bis-(4-phenylethinyl-phenyl)-allylsulfanyl]-2-methyl-phenoxy}-essigsäure,
{4-[3,3-Bis-(4-phenylethinyl-phenyl)-allylsulfanyl]-2-brom-phenoxy}-essigsäure oder
ein Salz davon mit einer pharmazeutisch akzeptablen Säure oder Base oder ein beliebiges optisches Isomer oder eine Mischung von optischen Isomeren, darunter racemische Mischungen oder beliebige tautomere Formen.

52. Verbindung nach einem der vorhergehenden Ansprüche 1 bis 50, die ist:
[4-[3,3-Bis-[4-[(thiophen-2-yl)-ethinyl]-phenyl]-allylsulfanyl]-2-methylphenoxy]-essigsäure,
{4-[3,3-Bis-(4-thiophen-3-yl-ethinylphenyl)-allylsulfanyl]-2-methylphenoxy}-essigsäure,
[4-[3,3-Bis-[4-[(pyridin-2-yl)-ethinyl]-phenyl]-allyfsulfanyl]-2-methylphenoxy]-essigsäure,
{4-[3,3-Bis-(4-pyridin-2-yl-ethinylphenyl)-allyloxy]-2-methylphenoxy}-essigsäure,
{4-[3,3-Bis-(4-furan-2-yl-ethinylphenyl)-allylsulfanyl]-2-methylphenoxy}-essigsäure,
(4-{3,3-Bis-[4-(1-methyl-1H-pyrrol-2-yl-ethinyl)-phenyl]-allylsulfanyl}-2-methylphenoxy)-essigsäure;
[4-[3,3-Bis-[4-[3-(morpholin-4-yl)-propin-1-yl]-phenyl]-allylsulfanyl]-2-methylphenoxy]-essigsäure;
[4-[3,3-Bis-[4-[3-(N,N-dimethylamino)-propin-1-yl]-phenyl]-allylsulfanyl}-2-methylphenoxy]-essigsäure;
[4-[3,3-Bis-[4-[3-(morpholin-4-yl)-propinyl]-phenyl]-allyloxy]-2-methylphenoxy]-essigsäure;
[4-(3,3-Bis-{4-[3-(4-acetyl-piperazin-1-yl)-prop-1-inyl]-phenyl}-allylsulfanyl)-2-methylphenoxy]-essigsäure,
[4-[3,3-Bis-[4-(3-pyrrolidin-1-yl-prop-1-inyl)-phenyl]-allylsulfanyl]-2-methylphenoxy]-essigsäure;
(4-{3,3-Bis-[4-(3-pyrrolidin-1-yl-prop-1-inyl)-phenyl]-allyloxy}-2-methylphenoxy)-essigsaure,
[4-[3,3-Bis-[5-[3-(morpholin-4-yl)-propinyl]-thiophen-2-yl]-allylsulfanyl]-2-methylphenoxy]-essigsäure;
[4-[3,3-Bis-[5-[3-(N-acetyl-N-methylamino)-propinyl]-thiophen-2-yl]-allylsulfanyl]-2-methylphenoxy]-essigsäure;
[4-[3,3-Bis-[4-[3,3,3-trimethylpropinyl]-phenyl]-allylsulfanyl]-2-methylphenoxy]-essigsäure;
[4-[3,3-Bis-[4-[3-(imidazol-1-yl)-propinyl]-phenyl]-allylsulfanyl]-2-methylphenoxy]-essigsäure;
[4-[3,3-Bis-[4-[3-(2-oxopyrrolidin-1-yl)-propinyl]-phenyl]-allylsulfanyl]-2-methylphenoxy]-essigsäure;
[4-[3,3-Bis-(4-methylcarbamoylethinylphenyl)-allylsulfanyl]-2-methylphenoxy]-essigsäure oder ein Salz davon mit einer pharmazeutisch akzeptablen Säure oder Base oder ein beliebiges optisches Isomer oder Mischung von optischen Isomeren, darunter racemische Mischungen oder
beliebige tautomere Formen.

53. Verbindung nach einem der vorhergehenden Ansprüche, die ein PPARδ-Agonist ist.

54. Verbindung nach Anspruch 53, die ein selektiver PPARδ-Agonist ist.

55. Kombination einer Verbindung wie in einem der Ansprüche 1 bis 54 definiert und eine oder mehrere zusätzliche pharmakologisch aktive Substanzen.

56. Kombination nach Anspruch 55, wobei die eine oder mehrere zusätzliche pharmakologisch aktive Substanzen ausgewählt sind aus Mitteln gegen Fettleibigkeit, Antidiabetika, Mitteln gegen hohen Blutdruck, Mitteln zur Behandlung und/oder Vorbeugung von Komplikationen, die durch Diabetes bedingt sind oder damit in Zusammenhang stehen, und Mitteln zur Behandlung und/oder Vorbeugung von Komplikationen und Störungen, die durch Fettleibigkeit bedingt sind oder damit in Zusammenhang stehen.

57. Pharmazeutische Zusammensetzung, die als Wirkstoff umfasst:
mindestens eine Verbindung nach einem der Ansprüche 1 bis 54 oder eine Kombination nach Anspruch 55 oder 56 zusammen mit einem oder mehreren pharmazeutisch akzeptablen Trägern oder Exzipienten.

58. Pharmazeutische Zusammensetzung nach Anspruch 57 in Form einer Dosiseinheit, umfassend von ungefähr 0,05 mg bis ungefähr 1000 mg, bevorzugt von ungefähr 0,1 bis ungefähr 500 mg und besonders bevorzugt von ungefähr 0,5 mg bis ungefähr 200 mg pro Tag der Verbindung nach einem der Ansprüche 1 bis 54.

59. Pharmazeutische Zusammensetzung nach Anspruch 57 oder 58 für orale, nasale, transdermale, pulmonale oder parenterale Verabreichung.

60. Verbindung nach einem der Ansprüche 1 bis 54, eine Kombination nach Anspruch 55 oder 56 oder eine Zusammensetzung nach einem der Ansprüche 57 bis 59 zur Verwendung als Medikament.

61. Verbindung, Kombination oder Zusammensetzung nach Anspruch 60 zur Behandlung und/oder Vorbeugung von Zuständen, die durch Nuklearrezeptoren, insbesondere die Peroxisom-Proliferator-aktivierien Rezeptoren (PPAR), mediiert sind.

62. Verbindung, Kombination oder Zusammensetzung nach Anspruch 60 zur Behandlung und/oder Vorbeugung von Typ 1 Diabetes, Typ 2 Diabetes, Dyslipidämie, Syndrom X (einschließlich dem metabolischen Syndrom, d. h. gestörte Glucosetoleranz, Insulinresistenz, Hypertriglyceridämie und/oder Fettleibigkeit), kardiovaskuläre Erkrankungen (einschließlich Atherosklerose) und Hypercholesterämie.

63. Verbindung, Kombination oder Zusammensetzung nach Anspruch 60 zur Behandlung und/oder Vorbeugung von Typ 1 Diabetes, Typ II Diabetes, gestörter Glucosetoleranz, Insulinresistenz oder Fettleibigkeit.

64. Verbindung, Kombination oder Zusammensetzung nach Anspruch 60, wobei die effektive Menge der Verbindung nach einem der Ansprüche 1 bis 54 im Bereich von ungefähr 0,05 mg bis ungefähr 1000 mg, bevorzugt von ungefähr 0,1 bis ungefähr 500 mg und besonders bevorzugt von ungefähr 0,5 mg bis ungefähr 200 mg pro Tag liegt.

65. Verwendung einer Verbindung wie in einem der Ansprüche 1 bis 54 definiert, oder einer Kombination wie in Anspruch 55 oder 56 definiert, oder einer Zusammensetzung wie in einem der Ansprüche 57 bis 59 definiert, bei der Herstellung eines Medikaments zur Behandlung und/oder Vorbeugung einer Erkrankung oder eines Zustands wie in einem der Ansprüche 61 bis 64 definiert.

## Revendications

1. Composé de formule générale (I) : 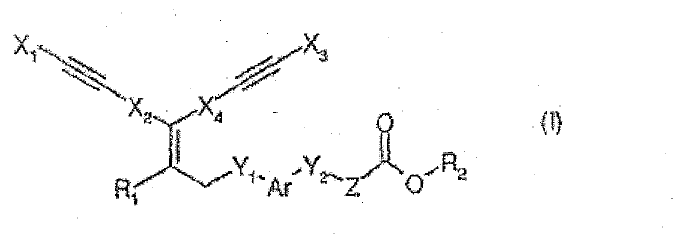 dans laquelle X₁ est un groupe aryle ou hétéroaryle dont chacun est facultativement substitué par un ou plusieurs substituants choisis parmi :
• de l'halogène, un groupe hydroxy, cyano, amino ou carboxy ; ou
• un groupe alkyle en C_{1 à 6}, cycloalkyle en C_{3 à 6}, alcényle en C_{2 à 6}, alcynyle en C_{2 à 6,} aryle, aralkyle, hétéroaryle, hétéroaralkyle, alcoxy en C_{1 à 6}, cycloalcoxy en C_{3 à 6}, aryloxy, aralcoxy, hétéroaralcoxy, alkylthio en C_{1 à 6}, arylthio, cycloalkylthio en C_{3 à 6}, alkylcarbonyle en C_{1 à 6}, arylcarbonyle, alkylsulfonyle en C_{1 à 6}, arylsulfonyle, alkylamino en C_{1 à 6}, arylamido, alkylaminocarbonyle en C_{1 à 6}, alkylamino, en C_{1 à 6}, dialkylamino en C_{1 à 6} ou cycloalkylamino en C_{3 à 6}, dont chacun est facultativement substitué par un ou plusieurs atomes d'halogène ; ou
X₁ est un groupe alkyle en C_{1 à 6}, cycloalkyle en C_{3 à 6}, alcényle en C_{2 à 6}, carbamoyle ou cycloalkyl en C_{3 à 6}-alkyle en C_{1 à 6} dont chacun est facultativement substitué par un ou plusieurs substituant choisis parmi :
• de l'halogène, un groupe hydroxy, cyano, amino ou carboxy ; ou
• un groupe alkyle en C_{1 à 6}, cycloalkyle en C_{3 à 6}, alcényle en C_{2 à 6}, alcynyle en C_{2 à 6}, cycloalkyl en C_{3 à 6}-alkyle en C_{1 à 6}, aryle, aralkyle, hétéroaryle, hétéroaralkyle, hétérocyclyle, alcoxy en C_{1 à 6}, cycloalcoxy en C_{3 à 6}, cycloalkyl en C_{3 à 6}-alcoxy en C_{1 à 6}, aryloxy, hétéroaryloxy, aralcoxy, hétéroaralcoxy, alkylthio en C_{1 à 6}, cycloalkylthio en C_{3 à 6}, cycloalkyl en C_{3 à 6}-alkylthio en C_{1 à 6}, arylthio, hétéroarylthio, aryl-alkylthio en C_{1 à 6}, hétéroaryl-alkylthio en C_{1 à 6}, alkylcarbonyle en C_{1 à 6}, cycloalkylcarbonyle en C_{3 à 6}, cycloalkyl en C_{3 à 6}-alkylcarbonyle en C_{1 à 6}, arylcarbonyle, hétéroarylcarbonyle, alkylsulfonyle en C_{1 à 6}, cycloalkylsulfonyle en C_{3 à 6}, cycloalkyl en C_{3 à 6}-alkylsulfonyle en C_{1 à 6}, arylsulfonyle, hétéroarylsulfonyle, alkylsulfamoyle en C_{1 à 6}, di-(alkyl en C_{1 à 6})sulfamoyle, alcoxycarbonyle en C_{1 à 6}, cycloalcoxycarbonyle en C_{3 à 6}, cycloalkyl en C_{3 à 6}-alcoxycarbonyle en C_{1 à 6}, amino-alkyle en C_{1 à 6}, alkylamino en C_{1 à 6}-alkyle en C_{1 à 6}, di-(alkyl en C_{1 à 6})amino-alkyle en C_{1 à 6}, alkylamino en C_{1 à 6}, cycloalkylamido en C_{3 à 6}, cycloalkyl en C_{3 à 6}-alkylamino en C_{1 à 6}, arylamido, alkylaminocarbonyle en C_{1 à 6}, cycloalkylamino en C_{3 à 6}-carbonyle, cycloalkyl en C_{3 à 6}-alkylaminocarbonyle en C_{1 à 6}, di-(alkyl en C_{1 à 6})-alkylaminocarbonyle, di-(cycloalkyl en C_{3 à 6}-alkyl en C_{1 à 6})aminocarbonyle, alkylamino, en C_{1 à 6}, cycloalkylamino en C_{3 à 6}, cycloalkyl en C_{3 à 6}-alkylamino en C_{1 à 6}, di-(alkyl en C_{1 à 6})amino, di-(cycloalkyl en C_{3 à 6})amino ou di-(cycloalkyl en C_{3 à 6}-alkyl en C_{1 à 6})amino dont chacun est facultativement substitué par un ou plusieurs éléments parmi de l'halogène, un groupe cyano, hydroxy, acétyle ou oxo. ; et
X₂ est un groupe arylène ou hétéroarylène dont chacun est facultativement substitué par un ou plusieurs substituants choisis parmi :
• de l'halogène, un groupe hydroxy, cyan, amino ou carboxy ; ou
• un groupe alkyle en C_{1 à 6}, cycloalkyle en C_{3 à 6}, alcényle en C_{2 à 6}, alcynyle en C_{2 à 6}, alcoxy en C_{1 à 6}, cycloalcoxy en C_{3 à 6}, alkylthio en C_{1 à 6}, cycloalkylthio en C_{3 à 6}, alkylamino, en C_{1 à 6}, dialkylamino en C_{1 à 6} ou cycloalkylamino en C_{3 à 6}, dont chacun est facultativement substitué par un ou plusieurs atomes d'halogène ; et
X₃ est un groupe aryle ou hétéroaryle dont chacun est facultativement substitué par un ou plusieurs substituants choisis parmi :
• de l'halogène, un groupe hydroxy, cyano, amino, ou carboxy ; ou
• un groupe alkyle en C_{1 à 6}, cycloalkyle en C_{3 à 6}, alcényle en C_{2 à 6}, alcynyle en C_{2 à 6}, aryle, aralkyle, hétéroaryle, hétéroaralkyle, alcoxy en C_{1 à 6}, cycloalcoxy en C_{3 à 6}, aryloxy, aralcoxy, hétéroaralcoxy, alkylthio, en C_{1 à 6}, arylthio, cycloalkylthio en C_{3 à 6}, alkylcarbonyle en C_{1 à 6}, arylcarbonyle, alkylsulfonyle en C_{1 à 6}, arylsulfonyle, alkylamido en C_{1 à 6}, arylamido, alkylaminocarbonyle en C_{1 à 6}, alkylamino en C_{1 à 6}, dialkylamino en C_{1 à 6} ou cycloalkylamino en C_{1 à 6}, dont chacun est facultativement substitué par un ou plusieurs atomes d'halogène ; ou
X₃ est un groupe alkyle en C_{1 à 6}, cycloalkyle en C_{3 à 6}, alcényle en C_{2 à 6}, carbamoyl ou cycloalkyl en C_{3 à 6}-alkyle en C_{1 à 6} dont chacun est facultativement substitué par une ou plusieurs substituant choisis parmi :
• de l'halogène, un groupe hydroxy, cyano, amino, ou carboxy ; ou
• un groupe alkyle en C_{1 à 6}, cycloalkyle en C_{3 à 6}, alcényle en C_{2 à 6}, alcynyle en C_{2 à 6}, cycloalkyl en C_{3 à 6}-alkyle en C_{1 à 6}, aryle, aralkyle, hétéroaryle, hétéroaralkyle, hétérocyclyle, alcoxy en C_{1 à 6}, cycloalcoxy en C_{3 à 6}, cycloalkyl en C_{3 à 6}-alcoxy en C_{1 à 6}, aryloxy, hétéroaryloxy, aralcoxy, hétéroaralcoxy, alkylthio en C_{1 à 6}, cycloalkylthio en C_{3 à 6}, cycloalkyl en C_{3 à 6}-alkylthio en C_{1 à 6}, arylthio, hétéroarylthio, aryl-alkylthio en C_{1 à 6}, hétéroaryl-alkylthio en C_{1 à 6}, alkylcarbonyle en C_{1 à 6}, cycloalkylcarbonyle en C_{3 à 6}, cycloalkyl en C_{3 à 6}-alkylcarbonyle en C_{1 à 6}, arylcarbonyle, hétéroarylcarbonyle, alkylsulfonyle en C_{1 à 6}, cycloalkylsulfonyle en C_{3 à 6}, cycloalkyl en C_{3 à 6}-alkylsulfonyle en C_{1 à 6}, arylsulfonyle, hétéroarylsulfonyle, alkylsulfamoyle en C_{1 à 6}, di-(alkyl en C_{1 à 6})sulfamoyle, alcoxycalrbonyle en C_{1 à 6}, cycloalcoxycarbonyle en C_{3 à 6}, cycloalkyl en C_{3 à 6}-alcoxycarbonyle en C_{1 à 6}, amino-alkyle en C_{1 à 6}, alkylamino en C_{1 à 6}-alkyle en C_{1 à 6}, di-(alkyl en C_{1 à 6})amino-alkyle en C_{1 à 6}, alkylamido en C_{1 à 6}, cycloalkylamido en C_{3 à 6}, cycloalkyl en C_{3 à 6}-alkylamido en C_{1 à 6}, arylamido, alkylaminocarbonyle en C_{1 à 6}, cycloalkylamino en C_{3 à 6}-carbonyl, cycloaloyl en C₃ à ₆-alkylaminocarbonyle en C_{1 à 6}, di-(alkyl en C_{1 à 6})-alkylaminocarbonyle, di-(cycloalkyl en C_{3 à 6}-alkyl en C_{1 à 6})aminocarbonyle, alkylamino en C_{1 à 6}, cycloalkylamino en C_{3 à 6}, cycloalkyl en C_{3 à 6}-alkylamino en C_{1 à 6}, di-(alkyl en C_{1 à 6})amino, di-(cycloalkyl en C_{3 à 6})amino ou di-(cycloalkyl en C_{3 à 6}-alkyl en C_{1 à 6})amino dont chacun est facultativement substitué par un ou plusieurs éléments parmi halogène, un groupe cyano, hydroxyo, acétyle ou oxo ; et
X₄ est un groupe arylène ou hétéroarylène dont chacun est facultativement substitué par un ou plusieurs substituants choisis parmi :
• de l'halogène, un groupe hydroxy, cyano, amino ou carboxy ; ou
• un groupe alkyle en C_{1 à 6}, cycloalkyle en C_{3 à 6}, alcényle en C_{2 à 6}, alcynyle en C_{2 à 6}, alcoxy en C_{1 à 6}, cycloalcoxy en C_{3 à 6}, alkylthio en C_{1 à 6}, cycloalkylthio en C_{3 à 6}, alkylamino en C_{1 à 6}, dialkylamino en C_{1 à 6} où cycloalkylamino en C_{3 à 6}, dont chacun est facultativement substitué par un ou plusieurs atomes d'halogène; et
Ar est un groupe arylène qui est facultativement substitué par un ou plusieurs substituants choisis parmi :
• de l'halogène, un groupe hydroxy ou cyano ; ou
• un groupe alkyle en C_{1 à 6}, cycloalkyle en C_{3 à 6}, alcényle en C_{2 à 6}, alcynyle en C_{2 à 6}, aryle, hétéroaryle, aralkyle, hétéroaralkyle, alcoxy en C_{1 à 6}, cycloalcoxy en C_{3 à 6}, aryloxy, aralcoxy, hétéroaralcoxy, alkylthio en C_{1 à 6}, arylthio ou cycloalkylthio en C_{3 à 6}, dont chacun est facultativement substitué par un ou plusieurs atomes d'halogène ; ou
• deux des substituant lorsqu'ils sont placés dans les positions adjacentes conjointement avec les atomes auxquels ils sont attachés peuvent former un cycle à cinq à huit chaînons ; et
Y₁ est O ou S ; et
Y₂ est O ou S ; et
Z est -(CH₂)ₙ- où n vaut 1, 2 ou 3 ; et
R₁ est un atome d'hydrogène, d'halogène ou un substituant choisi parmi :
• un groupe allyle en C_{1 à 6}, cycloalkyle en C_{3 à 6}, alcényle en C_{2 à 6}, alcynyle en C_{2 à 6}, aralkyle, hétéroaralkyle, alcoxy en C_{1 à 6}, cycloalcoxy en C_{3 à 6}, aryloxy, aralcoxy, hétéroaralcoxy, alkylthio en C_{1 à 6}, arylthio ou cycloalkylthio en C_{3 à 6}, dont chacun est facultativement substitué par une ou plusieurs atomes d'halogène ; et
R₂ est de l'hydrogène, un groupe alkyle en C_{1 à 6}, cycloalkyle en C_{3 à 6}, alcényle en C_{2 à 6}, alcynyle en C_{2 à 6}, alcénynyle en C_{4 à 6} ou aryle ; ou
un de sels pharmaceutiquement acceptable, ou un de ses solvates pharmaceutiquement acceptable, ou toute forme tautomère, stéréroisomère, mélange de stéréoisomères y compris un mélange racémique, ou polymorphe.

2. Composé selon la revendication 1, dans lequel X₁ est un groupe aryle ou héréroaryle dont chacun est facultativement substitué par un ou plusieurs substituants choisis parmi :
• de l'halogène, un groupe hydroxy, cyano, amino ou carboxy ; ou
• un groupe alkyle en C_{1 à 6,} cycloalkyle en C_{3 à 6}, alcényle en C_{2 à 6}, alcynyle en C_{2 à 6}, aryle, aralkyle, hétéroaryle, hétéroaralkyle, alcoxy en C_{1 à 6,} cycloalcoxy en C_{3 à 6}, aryloxy, aralcoxy, hétéroaralcoxy, alkylthio en C_{1 à 6}, arylthio, cycloalkylthio en C_{3 à 6}, alkylcarbonyle en C_{1 à 6}, arylcarbonyle, alkylsulfonyle en C_{1 à 6}, arylsulfonyle, alkylamido en C_{1 à 6}, arylamido, alkylaminocarbonyle en C_{1 à 6}, alkylamino en C_{1 à 6}, dialkylamino en C_{1 à 6} ou cycloalkylamino en C_{3 à 6}, dont chacun est facultativement substitué par un ou plusieurs atomes d'halogène ; ou
X₂ est un groupe arylène ou hétéroarylène dont chacun est facultativement substitué par un ou plusieurs substituant choisis parmi :
• de l'halogène, un groupe hydroxy, cyano, amino ou carboxy ; ou
• un groupe alkyle en C_{1 à 6}, cycloalkyle en C_{3 à 6}, alcényle en C_{2 à 6}, alcynyle sen C_{2 à 6}, alcoxy en C_{1 à 6}, cycloalcoxy en C_{3 à 6}, alkylthio en C_{1 à 6}, cycloalkylthio en C_{3 à 6}, alkylamino, en C_{1 à 6}, dialkylamino en C_{1 à 6} ou cycloalkylamino en C_{3 à 6}, dont chacun est facultativement substitué par un ou plusieurs atomes d'halogène ; et
X₃ est un groupe aryle ou hétéroaryle dont chacun est facultativement substitué par un ou plusieurs substituants choisis parmi :
• de l'halogène, un groupe hydroxy, cyano, amino ou carboxy ; ou
• un groupe alkyle en C_{1 à 6}, cycloalkyle en C_{3 à 6}, alcényle en C_{2 à 6}, alcynyle en C_{2 à 6}, aryle, aralkyle, hétéroaryle, hétéroaralkyle, alcoxy en C_{1 à 6}, cycloalcoxy en C_{3 à 6}, aryloxy, aralcoxy, hétéroaralcoxy, alkylthio en C_{1 à 6}, arylthio, cycloalkylthio en C_{3 à 6}, alkylcarbonyle en C_{1 à 6}, arylcarbonyle, alkylsulfonyle en C_{1 à 6}, arylsulfonyle, alkylamido en C_{1 à 6}, arylamido, alkylaminocarbonyle en C_{1 à 6}, alkylamino en C_{1 à 6}, dialkylamino en C_{1 à 6} ou cycloalkylamino en C_{3 à 6}, dont chacun est facultativement substitué par un ou plusieurs halogènes ; et
X₄ est un groupe arylène ou hétéroarylène dont chacun est facultativement substitué par un ou plusieurs substituants choisis parmi:
• de l'halogène, un groupe hydroxy, cyano, amino ou carboxy; ou
• un groupe alkyle en C_{1 à 6}, cycloalkyle en C_{3 à 6}, alcényle en C_{2 à 6}, alcynyle en C_{2 à 6}, alcoxy en C_{1 à 6}, cycloalcoxy en C_{3 à 6}, alkylthio en C_{1 à 6}, cycloalkylthio en C_{3 à 6}, alkylamino en C_{1 à 6}, dialkylamino en C_{1 à 6} ou cycloalkylamino en C_{3 à 6}, dont chacun est facultativement substitué par un ou plusieurs halogènes ; et
Ar est un groupe arylène qui est facultativement substitué par une ou plusieurs substituants choisis parmi :
• a de l'halogène, un groupe hydroxy ou cyano ; ou
• un groupe alkyle en C_{1 à 6}, cycloalkyle en C_{3 à 6}, alcényle en C_{2 à 6}, alcynyle en C_{2 à 6}, aryle, hétéroaryle, aralkyle, hétéroaralkyle, alcoxy en C_{1 à 6}, cycloalcoxy en C_{3 à 6}, aryloxy, aralcoxy, hétéroaralcoxy, alkylthio en C_{1 à 6}, arylthio ou cycloalkylthio en C_{3 à 6}, dont chacun est facultativement substitué par un ou plusieurs halogènes ; ou
• deux des substituants lorsqu'ils sont placés dans les positions adjacentes conjointement avec les atomes auxquels ils sont attachés peuvent former un cycle à cinq à huit chaînons ; et
Y₁ est O ou S ; et
Y₂ est O ou S ; et
Z est -(CH₂)ₙ- où n vaut 1, 2 ou 3 ; et
R₁ est de l'hydrogène, de l'halogène ou un substituant choisi parmi :
• un groupe alkyle en C_{1 à 6}, cycloalkyle en C_{3 à 6}, alcényle en C_{2 à 6}, alcynyle en C_{2 à 6}, aralkyle, hétéroaralkyle, alcoxy en C_{1 à 6}, cycloalcoxy en C_{3 à 6}, aryloxy, aralcoxy, hétéroaralcoxy, alkylthio en C_{1 à 6}, arylthio ou cycloalkylthio en C_{3 à 6}, dont chacun est facultativement substitué par un ou plusieurs atomes d'halogène ; et
R₂ est de l'hydrogène, un groupe alkyle en C_{1 à 6}, cycloalkyle en C_{3 à 6}, alcényle en C_{2 à 6}, alcynyle en C_{2 à 6}, alcénynyle en C_{4 à 6} ou aryle ; ou
un de ses sels pharmaceutiquement acceptables, ou une de ses solvates pharmaceutiquement acceptables, ou toute forme tautomère, stéréroisomère, mélange de stéréoisomères y compris mélange racémique, ou polymorphe.

3. Composé selon la revendication 1 ou 2, dans lequel X₁ est une groupe aryle facultativement substitué par un ou plusieurs substituants choisis parmi :
• de l'halogène ; ou
• un groupe alkyle en C_{1 à 6}, aryle, alcoxy en C_{1 à 6} ou alkylsulfonyle en C_{1 à 6}, dont chacun est facultativement substitué par un ou plusieurs atomes d'halogène.

4. Composé selon la revendication 3, dans lequel X₁ est un groupe aryle facultativement substitué par un ou plusieurs substituants choisis parmi :
• de l'halogène ; ou
• un groupe alkyle en C_{1 à 6} facultativement substitué par un ou plusieurs atomes d'halogène.

5. Composé selon la revendication 3, dans lequel X₁ est un groupe phényle facultativement substitué par un ou plusieurs substituants choisis parmi :
• de l'halogène ; ou
• un groupe alkyle en C_{1 à 6}, aryle, alcoxy- en C_{1 à 6} ou alkylsulfonyle en C_{1 à 6}, dont chacun est facultativement substitué par un ou plusieurs halogènes.

6. Composé selon la revendication 4 ou 5, dans lequel X₁ est un groupe phényle facultativement substitué par un ou plusieurs substituant choisis parmi :
• de l'halogène ; ou
• un groupe alkyle en C_{1 à 6} facultativement substitué par un plusieurs halogènes.

7. Composé selon l'une quelconque des revendications précédentes, dans lequel X₁ est un groupe phényle.

8. Composé selon la revendication 1 ou 2, dans lequel X₁ est un groupe hétéroaryle facultativement substitué par une ou plusieurs substituants choisis parmi
• de l'halogène ; ou
• un groupe alkyle en C_{1 à 6}, aryle, alcoxy en C_{1 à 6} ou alkylsulfonyle en C_{1 à 6}, dont chacun est facultativement substitué par un ou plusieurs halogènes.

9. Composé selon la revendication 8, dans lequel X₁ est un groupe hétéroaryle facultativement substitué par un ou plusieurs substituants choisis parmi :
• de l'halogène ; ou
• un groupe allyle en C_{1 à 6} facultativement substitué par une plusieurs halogène.

10. Composé selon la revendication 8 ou 9, dans lequel X₁ est un groupe pyrrolyle, pyridyle, furyle ou thiényle, dont chacun est facultativement substitué par un ou plusieurs halogènes ou un groupe alkyle en C_{1 à 6}, qui est facultativement substitué par un ou plusieurs halogènes.

11. Composé selon la revendication 10, dans lequel X₁ est un groupe furyle ou thiényle facultativement substitué par un ou plusieurs atomes d'halogène.

12. Composé selon la revendication 10, dans lequel X₁ est un groupe pyrrolyle ou pyridyle dont chacun est facultativement substitué par un ou plusieurs groupes alkyle en C_{1 à 6}.

13. Composé selon la revendication 1, dans lequel X₁ est un groupe alkyle en C_{1 à 6} ou carbamoyle facultativement substitué par un ou plusieurs substituant choisis parmi :
• de l'halogène, un groupe hydroxy, cyano, amino ou carboxy ; ou
• un groupe alkyle en C_{1à 6}, cycloalkyle en C_{3 à 6}, alcényle en C_{2 à 6}, alcynyle en C_{2 à 6}, cycloalkyl en C_{3 à 6}-alkyle en C_{1 à 6}, aryle, aralkyle, hétéroaryle, hétéroaralkyle, hétérocyclyle, alcoxy en C_{1 à 6}, cycloalcoxy en C_{3 à 6}, cycloalkyl en C_{3 à 6}-alcoxy en C_{1 à 6}, aryloxy, hétéroaryloxy, aralcoxy, hétéroaralcoxy, alkylthio, en C_{1 à 6}, cycloalkylthio en C_{3 à 6}, cycloalkyl en C_{3 à 6}-alkythio en C_{1 à 6}, arylthio, hétéroarylthio, aryl-alkylthio en C_{1 à 6}, hétéroaryl-alkylthio en C_{1 à 6}, alkylcarbonyle en C_{1 à 6}, cycloalkylcarbonyle en C_{3 à 6}, cycloalkyl en C_{3 à 6}-alkylcarbonyle en C_{1 à 6}, arylcarbonyle, hétéroarylcarbonyle, alkylsulfonyle en C_{1 à 6}, cycloalkylsulfonyle en C_{3 à 6}, cycloalkyl en C_{3 à 6}-alkylsulfonyle en C_{1 à 6}, arylsulfonyle, hétéroarylsulfonyle, alkylsulfamoyle en C_{1 à 6}, di-(alkyl en C_{1 à 6})sulfamoyle, alcoxycarbonyle en C_{1 à 6}, cycloalcoxycarbonyle en C_{3 à 6}, cycloalkyl en C_{3 à 6}-alcoxycarbonyle en C_{1 à 6}, amino-alkyle en C_{1 à 6}, alkylamino, en C_{1 à 6}-alkyle en C_{1 à 6}, di-(alkyl en C_{1 à 6})aminoallyle en C_{1 à 6}, alkylamido en C_{1 à 6}, cycloalkylamido en C_{3 à 6}, cycloalkyl en C_{3 à 6}-alkylamido en C_{1 à 6}, arylamido, alkylaminocarbonyle en C_{1 à 6}, cycloalkylamino en C_{3 à 6}-carbonyle, cycloalkyl en C_{3 à 6}-alkylaminocarbonyle en C_{1 à 6}, di-(alkyl en C_{1 à 6})-alkylaminocarbonyle, di-(cycloalkyl en C_{3 à 6}-alkyl en C_{1 à 6})aminocarbonyle, alkylamino en C_{1 à 6}, cycloalkylamino en C_{3 à 6}, cycloalkyl en C_{3 à 6}-alkylamino en C_{1 à 6}, di-(alkyl en C_{1 à 6})amino, di-(cycloalkyl en C_{3 à 6})amino ou di-(cycloalkyl en C_{3 à 6}-alkyl en C_{1 à 6})amino dont chacun est facultativement substitué par un ou plusieurs éléments parmi une halogène, un groupe cyano, hydroxy, acétyle ou oxo.

14. Composé selon la revendication 13, dans lequel X₁ est un groupe alkyle en C_{1 à 6} ou carbamoyle facultativement substitué par un ou plusieurs substituant choisi parmi :
• de l'halogène ou un groupe hydroxy ; ou
• une groupe alkyle en C_{1 à 6}, aryle, hétéroaryle, hétérocyclyle, alcoxy en C₁₋₆, alkylthio en C_{1 à 6}, cycloalkylthio en C_{3 à 6}, cycloalkyl en C_{3 à 6}-alkylthio en C_{1 à 6}, arylthio, hétéroarylthio, aryl-alkylthio en C_{1 à 6}, alkylcarbonyle en C_{1 à 6}, arylcarbonyle, alkylsulfonyle en C_{1 à 6}, arylsulfonyle, amino-alkyle en C_{1 à 6}, alkylamino en C_{1 à 6} alkyle en C_{1 à 6}, di-(alkyl en C_{1 à 6})amino-alkyle en C_{1 à 6}, alkylamido en C_{1 à 6}, arylamido, alkylaminocarbonyle en C_{1 à 6}, di-(alkyl en C_{1 à 6})-aminocarbonyle, alkylamino en C_{1 à 6}, di-(alkyl en C_{1 à 6})amino, dont chacun est facultativement substitué par un ou plusieurs éléments parmi de l'halogène, un groupe cyano, hydroxy, acétyle ou oxo.

15. Composé selon la revendication 13 ou 14, dans lequel X₁ et une groupe allyle en C_{1 à 6} facultativement substitué par un ou plusieurs substituants choisis parmi un groupe alkyle en C_{1 à 6}, hétéroaryle, hétérocyclyle, alkyl en C_{1 à 6}-amino, di-(alkyl en C_{1 à 6})amino, dont chacun est facultativement substitué par un ou plusieurs éléments parmi un groupe acétyle ou oxo.

16. Composé selon la revendication 13 ou 14, dans lequel X₃ et un groupe carbamoyle facultativement substitué par un groupe acétyle.

17. Composé selon l'une quelconque des revendications précédentes, dans lequel X₂ est un groupe arylène facultativement substitué par un ou plusieurs substituants choisis parmi:
• de l'halogène ; ou
• un groupe alkyle en C_{1 à 6} facultativement substitué par un ou plusieurs atomes d'halogène.

18. Composé selon l'une quelconque des revendications précédentes, dans lequel X₂ est un groupe phénylène facultativement substitué par un ou plusieurs substituants choisis parmi:
• de l'halogène; ou
• un groupe alkyle en C_{1 à 6} facultativement substitué par une ou plusieurs atomes d'halogène.

19. Composé selon la revendication 17 ou 18, dans lequel X₂ est un groupe phénylène.

20. Composé selon l'une quelconque des revendications à 16, dans lequel X₂ est un groupe hétéroarylène facultativement substitué par un ou plusieurs substituants choisis parmi :
• de halogène ; ou
• un groupe alkyle en C₁ à ₆ facultativement substitué par un ou plusieurs atomes d'halogène.

21. Composé selon l'une quelconque des revendications précédentes, dans lequel X₃ est un groupe aryle facultativement substitué par un ou plusieurs substituents choisis parmi :
• de l'halogène ; ou
• un groupe alkyle en C_{1 à 6,} aryle, alcoxy en C_{1 à 6} ou alkylsulfonyle en C_{1 à 6}, dont chacun est facultativement substitué par un ou plusieurs atomes d'halogène.

22. Composé selon la revendication 21, dans lequel X₃ est un groupe aryle facultativement substitué par un ou plusieurs substituants choisis parmi ;
• de l'halogène ; ou
• un groupe alkyle en C_{1 à 6} facultativement substitué par un ou plusieurs halogènes.

23. Composé selon la revendication 21, dans lequel X₃ est un groupe phényle facultativement substitué par un ou plusieurs substituants choisis parmi:
• de l'halogène; ou
• un groupe alkyle en C_{1 à 6}, aryle, alcoxy en C_{1 à 6} ou alkylsulfonyle en C_{1 à 6}, dont chacun est facultativement substitué par un ou plusieurs halogènes.

24. Composé selon la revendication 22 ou 23, dans lequel X₃ est un groupe phényle facultativement substitué par un ou plusieurs substituants choisis parmi :
• de l'halogène ; ou
• un groupe alkyle en C_{1 à 6} facultativement substitué par une ou plusieurs halogènes.

25. Composé selon l'une quelconque des revendications 21 à 24, dans lequel X₃ est un groupe phényle.

26. Composé selon l'une quelconque des revendications 1 à 20, dans lequel X₃ est un groupe hétéroaryle facultativement substitué par un ou plusieurs substituant choisis parmi :
• de l'halogène ; ou
• un groupe alkyle en C_{1 à 6}, aryle, alcoxy en C_{1 à 6} ou alkylsulfonyle en C_{1 à 6}, dont chacun est facultativement substitué par un ou plusieurs halogènes.

27. Composé selon la revendications 26, dans lequel X₃ est un groupe hétéroaryle facultativement substitué par un ou plusieurs substituants choisis parme :
• de l'halogène ; ou
• un groupe alkyle en C_{1 à 6} facultativement substitué par un ou plusieurs halogènes.

28. Composé selon la revendication 27, dans lequel X₃ est un groupe pyrrolyle, pyridyle, furyle ou thiényle dont chacun est facultativement substitué par un ou plusieurs éléments parmi de l'halogène ou un
groupe alkyle en C_{1 à 6}, qui est facultativement substitué par un ou plusieurs halogènes.

29. Composé selon la revendication 28, dans lequel X₃ est un groupe furyle ou thiényle facultativement substitué par ou plusieurs halogènes.

30. Composé selon la revendication 28, dans lequel X₃ est un groupe pyrrolyle ou pyridyle, dont chacun est substitué par un ou plusieurs groupes alkyle en C_{1 à 6}.

31. Composé selon la revendication 1, dans lequel X₃ est un groupe alkyle en C_{1 à 6} ou carbamoyle facultativement substitué par un ou plusieurs substituants choisis parmi :
• de l'halogène, un groupe hydroxy, cyano, amino ou carbon ; ou
• un groupe alkyle en C_{1 à 6}, cycloalkyle en C_{3 à 6}, alcényle en C_{2 à 6}, alcynyle en C_{2 à 6}, cycloalkyl en C_{3 à 6}-alkyle en C_{1 à 6}, aryle, aralkyle, hétéroaryle, hétéroaralkyle, hétérocyclyle, alcoxy en C_{1 à 6}, cycloalcoxy en C_{3 à 6}, cycloalkyl en C_{3 à 6}-alcoxy en C_{1 à 6}, aryloxy, hétéroaryloxy, aralcoxy, hétéroaralcoxy, alkylthio, en C_{1 à 6}, cycloalkylthio en C_{3 à 6}, cycloalkyl en C_{3 à 6}-alkythio en C_{1 à 6}, arylthio, hétéroarylthio, aryl-alkylthio en C_{1 à 6}, hétéroaryl-alkylthio en C_{1 à 6}, alkylcarbonyle en C_{1 à 6}, cycloalkylcarbonyle en C_{3 à 6}, cycloalkyl en C_{3 à 6}-alkylcarbonyle en C_{1 à 6}, arylcarbonyle, hétéroarylcarbonyle, alkylsulfonyle en C_{1 à 6}, cycloalkylsulfonyle en C_{3 à 6}, cycloalkyl en C_{3 à 6}-alkylsulfonyle en C_{1 à 6}, arylsulfonyle, hétéroarylsulfonyle, alkylsulfamoyle en C_{1 à 6}, di-(alkyl en C_{1 à 6})sulfamoyle, alcoxycarbonyle en C_{1 à 6}, cycloalcoxycarbonyle en C_{3 à 6}, cycloalkyl en C_{3 à 6}-alcoxycarbonyle en C_{1 à 6}, amino-alkyle en C_{1 à 6}, alkylamino, en C_{1 à 6}-alkyle en C_{1 à 6}, di-(alkyl en C_{1 à 6})amino-alkyle en C_{1 à 6}, alkylamido en C_{1 à 6}, cycloalkylamido en C_{3 à 6}, cycloalkyl en C_{3 à 6}-alkylamino en C_{1 à 6}, arylamido, alkylaminocarbonyle en C_{1 à 6}, cycloalkylamino en C_{3 à 6}-carbonyle, cycloalkyl en C_{3 à 6}-alkylaminocarbonyle en C_{1 à 6}, di-(alkyl en C_{1 à 6})-aminocarbonyle, di-(cycloalkyl en C_{3 à 6}-alkyl en C_{1 à 6})aminocarbonyle, alkylamino en C_{1 à 6}, cycloalkylamino en C3 à 6, cycloalkyl en C_{3 à 6}-alkylamino en C_{1 à 6}, di-(alkyl en C_{1 à 6}) amino, di-(cycloalkyl en C_{3 à 6})amino ou di-(cycloalkyl en C_{3 à 6}-alkyl en C_{1 à 6})amino dont chacun est facultativement substitué par un ou plusieurs éléments parmi de l'halogène, un groupe cyano, hydroxy, acétyle ou oxo.

32. Composé selon la revendication 31, dans lequel X₃ est un groupe alkyle en C_{1 à 6} ou carbamoyle facultativement substitué par un ou plusieurs substituant choisis parmi :
• de l'halogène ou un groupe hydroxy ; ou
• un groupe alkyle en C_{1 à 6}, aryle, hétéroaryle, hétérocyclyle, alcoxy en C_{1 à 6}, alkylthio en C_{1 à 6}, cycloalkylthio en C_{3 à 6}, cycloalkyl en C_{3 à 6}-alkythio en C_{1 à 6}, arylthio, hétéroarylthio, aryl-alkylthio en C_{1 à 6}, alkylcarbonyle en C_{1 à 6}, arylcarbonyle, alkylsulfonyle en C_{1 à 6}, arylsulfanyle, amino-alkyle en C_{1 à 6}, alkylamino en C_{1 à 6}-alkyle en C_{1 à 6}, di-(alkyl en C_{1 à 6})amino-alkyle en C_{1 à 6}, alkylamino en C_{1 à 6}, arylamido, alkylaminocarbonyle en C_{1 à 6}, di-(alkyl en C_{1 à 6})-aminocarbonyle, alkylamino en C_{1 à 6}, di-(alkyl en C_{1 à 6})amino, dont chacun est facultativement substitué par un ou plusieurs éléments parmi de l'halogène, un groupe cyano, hydroxy, acétyle ou oxo.

33. Composé selon la revendication 31 ou 32, dans lequel X₃ est une groupe alkyle en C_{1 à 6} facultativement substitué par un ou plusieurs substituants choisis parmi un groupe alkyle en C_{1 à 6}, hétéroaryle, hétérocyclyle, alkylamino en C_{1 à 6}, di-(alkyl en C_{1 à 6})amino, dont chacun est facultativement substitué par un ou plusieurs éléments parmi un groupe acétyle ou oxo.

34. Composé selon la revendication 31 ou 32, dans lequel X₃ est un groupe carbamoyle facultativement substitué par un groupe acétyle.

35. Composé selon l'une quelconque des revendications précédentes, dans lequel X₄ est un groupe arylène facultativement substitué par un ou plusieurs substituants choisis parmi :
• de l'halogène ; ou
• un groupe alkyle en C_{1 à 6} facultativement substitué par un ou plusieurs halogènes.

36. Composé selon la revendication 35, dans lequel X₄ est un groupe phénylène facultativement substitué par un ou plusieurs substituants choisis parmi :
• de l'halogène ; ou
• un groupe alkyle en C_{1 à 6} facultativement substitué par un ou plusieurs halogènes.

37. Composé selon la revendication 35 ou 36, dans lequel X₄ est un groupe phénylène.

38. Composé selon l'une quelconque des revendications 1 à 34, dans lequel X₄ est un groupe hétéroarylène facultativement substitué par un ou plusieurs substituants choisis parmi ;
• de l'halogène ; ou
• un groupe alkyle en C_{1 à 6} facultativement substitué par un ou plusieurs halogènes.

39. Composé selon l'une quelconque des revendications précédentes, dans lequel Ar est un groupe phénylène facultativement substitué par un ou plusieurs substituants choisis parmi :
• de l'halogène, un groupe hydroxy, ou cyano ; ou
• un groupe alkyle en C_{1 à 6}, cycloalkyle en C_{3 à 6}, alcényle en C_{2 à 6}, alcynyle en C_{2 à 6}, aryle, hétéroaryle, aralkyle, hétéroaralkyle, alcoxy en C_{1 à 6}, cycloalcoxy en C_{3 à 6}, aryloxy, aralcoxy, hétéroaralcoxy, alkylthio, en C_{1 à 6}, arylthio ou cycloalkylthio en C_{3 à 6}, dont chacun est facultativement substitué par un ou plusieurs halogènes ; ou
• deux des substituants lorsqu'ils sont placés dans les positions adjacentes conjointement avec les atomes auxquels ils sont attachés peuvent former un cycle à cinq à huit chaînons.

40. Composé selon la revendication 39, dans lequel Ar est une groupe phénylène qui est facultativement substitué par un ou plusieurs substituants choisis parmi :
• de l'halogène ; ou
• un groupe alkyle en C_{1 à 6}, alcoxy en C_{1 à 6}, aryloxy ou aralcoxy, dont chacun est facultativement substitué par un ou plusieurs halogènes ; ou
• deux des substituants lorsqu'ils sont placés dans les positions adjacentes conjointement avec les atomes auxquels ils sont attachés forment un cycle carbone à cinq chaînons.

41. Composé selon la revendication 39 ou 40, dans lequel Ar est un groupe phénylène qui est facultativement substitué par de l'halogène.

42. Composé selon l'une quelconque des revendications 39 à 41, dans lequel Ar est un groupe phénylène qui est facultativement substitué par un groupe méthyle.

43. Composé selon l'une quelconque des revendications précédentes, dans lequel Y₁ est S.

44. Composé selon l'une quelconque des revendications précédentes, dans lequel Y est O.

45. Composé selon l'une quelconque des revendications précédentes, dans lequel n vaut 1.

46. Composé selon l'une quelconque des revendications précédentes, dans lequel R₁ est de l'hydrogène ou un substituant choisi parmi :
• un groupe alkyle en C_{1 à 6}, aralkyle, alcoxy en C_{1 à 6}, aryloxy ou aralcoxy, dont chacun est facultativement substitué par un ou plusieurs halogènes.

47. Composé selon la revendication 46, dans lequel R₁ est de l'hydrogène ou un substituant choisi parmi :
• un groupe alkyle en C_{1 à 6}, alcoxy en C_{1 à 6}, dont chacun est facultativement substitué par un ou plusieurs halogènes.

48. Composé selon la revendication 46 ou 47, dans lequel R₁ est de l'hydrogène.

49. Composé selon l'une quelconque des revendications précédentes, dans lequel R₂ est de l'hydrogène ou un groupe alkyle en C_{1 à 6}.

50. Composé selon la revendication 49, dans lequel R₂ est de l'hydrogène.

51. Composé selon l'une quelconque des revendications précédentes qui est :
l'acide {4-[3,3-bis-(4-phényléthynyl-phényl)-allylsulfanyl]-2-méthylphénoxy}-acétique ;
l'acide {4-[3,3-bis-(4-phényléthynyl-phényl)-allylsulfanyl]-2-bromophénoxy}-acétique ; ou
un de leurs sels avec un acide ou une base pharmaceutiquement acceptable, ou tout isomère optique ou mélange d'isomères optiques, y compris un mélange racémique, ou toute forme tautomère.

52. Composé selon l'une quelconque des revendications 1 à 50 qui est :
l'acide [4-[3,3-bis[4-[(thiofèn-2-yl)éthylnyl]phényl]allylsulfanyl]-2-méthylphénoxy]acétique ;
l'acide {4-[3,3-bis[4-(thiofèn-3-yléthynylphényl)allylsulfanyl]-2-méthylphénoxy}acétique ;
l'acide [4-[3,3-bis[4-[(pyridine-2-yl)éthynyl]phényl]allylsulfanyl]-2-méthylphénoxyjacétique ;
l'acide {4-[3,3-bis-(4-(pyridin-2-yléthynylphényl)allyloxy]-2-méthylphénoxy}acétique ;
l'acide {4-[3,3-bis-(4-furan-2-yléthynylphényl)allylsulfanyl]-2-méthylphénoxy}acétique;
l'acide (4-{3,3-bis-[4-(1-méthyl-1H-pyrrol-2-yléthynyl)phényl]allylsulfanyl}-2-méthylphénoxy)acétique ;
l'acide [4-[3,3-bis-[4-[3-(morpholine-4-yl)propyn-1-yl]phényl]allylsulfanyl]-2-méthylphénoxy]acétique
l'acide [4-[3,3-bis-[4-[3-(N,N-diméthylamino)propyn-1-yl]phényl]allylsulfanyl]-2-méthylphénoxy]acétique ;
l'acide [4-[3,3-bis-[4-[3-(morpholine-4-yl)propynyl]phényl]allyloxy]-2-méthylphénoxy]acétique ;
l'acide [4-(3,3-bis-{4-[3-(4-acétyl-pipérazin-1-yl)prop-1-ynyl]phényl}allylsulfanyl)-2-méthyl-phénoxy]acétique ;
l'acide [4-[3,3-bis-[4-(3-pyrrolidin-1-yl-prop-1-ynyl)phényl]allylsulfanyl]-2-méthylphénoxy]acétique ;
l'acide (4-{3,3-bis-[4-(3-pyrrolidin-1-yl-prop-1-ynyl)phényl]allyloxy}-2-méthylphénoxy) acétique
l'acide [4-[3,3-bis-[5-[3-(morpholine-4-yl)propynyl]thiophène-2-yl]allylsulfanyl]-2-méthylphénoxy]-acétique;
l'acide [4-[3,3-bis-[5-[3-(N-acétyl-N-méthylamino)propynyl]thiophène-2-yl]allylsulfanyl]-2-méthyl-phénoxy]acétique;
l'acide [4-[3,3-bis[4-[3,3,3-triméthylpropynyl]phényl]allylsulfanyl]-2-méthylphénoxy]acétique ;
l'acide [4-[3,3-bis[4-[3-(imidazol-1-yl)propynyl]phényl]allylsulfanyl]-2-méthylphénoxy]acétique ;
l'acide [4-[3,3-bis[4-[3-(2-oxopyrrolidin-1-yl)propynyl)phényl]allylsulfanyl]-2-méthylphénoxy]acétique ;
l'acide [4-[3,3-bis(4-méthylcarbamoyléthynylphényl)allylsulfanyl]-2-méthylphénoxyjacétique ; ou
un de leurs sels avec un acide ou une base pharmaceutiquement acceptable, ou tout isomère optique ou mélange d'isomères optiques, y compris un mélange racémique, ou toute forme tautomère.

53. Composé selon l'une quelconque des revendications précédentes, qui est un agoniste de PPARδ.

54. Composé selon la revendication 53, qui est un agoniste de PPARδ sélectif.

55. Combinaison d'un composé tel que défini dans l'une quelconque des revendications 1 à 54, et d'une ou plusieurs substances pharmacologiquement actives additionnelles.

56. Combinaison selon la revendication 55, dans laquelle la ou les substances pharmacologiquement actives additionnelles sont choisies parmi les agents anti-obésité, les antidiabétiques, les agents antihypertenseurs, les agents pour le traitement et/ou la prévention des complications résultant de ou associées au diabète et des agents pour le traitement et/ou la prévention des complications et troubles résultant de ou associés à l'obésité.

57. Composition pharmaceutique comprenant, comme ingrédient actif, au moines un composé selon l'une quelconque des revendications 1 à 54, ou une combinaison selon la revendication 55 ou 56, conjointement avec un ou plusieurs supports ou excipients pharmaceutiquement acceptables.

58. Composition pharmaceutique selon la revendication 57, sous forme posologique unitaire, comprenant d'environ 0,05mg à environ 1 000 mg, de préférence d'environ 0,1 à environ 500 mg et de manière spécialement préférée d'environ 0,5 mg à environ 200 mg par jour d'un composé selon l'une quelconque des revendications 1 à 54.

59. Composition pharmaceutique selon la revendication 57 ou la revendication 58, pour une administration orale, nasale, transdermique, pulmonaire ou parentérale.

60. Composé selon l'une quelconque des revendications 1 à 54, combinaison selon la revendication 55 ou 56, ou composition selon l'une quelconque des revendications 57 à 59, à utiliser comme médicament.

61. Composé, combinaison ou composition selon la revendication 60, pour le traitement et/ ou la prévention d'affections véhiculées par des récepteurs nucléaires, en particulier les récepteurs activés par proliférateur de peroxysome (PPAR).

62. Composé, combinaison ou composition selon la revendication 60, pour le traitement et/ou la prévention du diabète du type 1, du diabète du type 2, de la dyslipidémie, du syndrome X (y compris le syndrome métabolique, c'est-à-dire un affaiblissement de la tolérance au glucose, de la résistante à l'insuline, l'hypertriglycéridémie et/ou l'obésité), les maladies cardiovasculaires (y compris l'athérosclérose) et l'hypercholestérolémie.

63. Composé, combinaison ou composition selon la revendication 60, pour le traitement et/ou la prévention du diabète du type 1, du diabète du type 2, d'une tolérance au glucose affaiblie, d'une résistance à l'insuline affaiblie ou de l'obésité.

64. Composé, combinaison ou composition selon la revendication 60, dans laquelle la quantité efficace du composé selon l'une quelconque des revendications 1 à 54 est dans la gamme d'environ 0,05 mg à environ 1 000 mg, de préférence d'environ 0,1 à environ 500 mg et de manière spécialement préférée d'environ 0,5 mg à environ 200 mg par jour.

65. Utilisation d'un composé tel que défini dans l'une quelconque des revendications 1 à 54, ou d'une combinaison telle que définie dans la revendication 55 ou la revendication 56, ou d'une composition telle que définie dans l'une quelconque des revendications 57 à 59, dans la fabrication d'un médicament destiné au traitement et/ou à la prévention d'une maladie ou affection telle que définie dans l'une quelconque des revendications 61 à 64.
